# EUROPEAN PATENT APPLICATION

(11) **EP 3 907 226 A2**
(43) Date of publication of application: **10.11.2021**
(21) Application number: 20735878.9
(22) Date of filing: 03.01.2020
(51) Int. Cl.: C07D 487/04, A61K 31/5517, A61K 45/06, A61P 35/00

(54) **PYRROLOBENZODIAZEPINE DIMER COMPOUND WITH IMPROVED SAFETY AND USE THEREOF**

(30) Priority: 03.01.2019 KR 20190000514; 02.01.2020 KR 20200000270
(71) Applicant: LegoChem Biosciences, Inc., Daejeon 34302 (KR)
(72) Inventor: SONG, Ho Young, Daejeon 34302 (KR); BAEK, Juyuel, Daejeon 34302 (KR); KIM, Sung Min, Daejeon 34302 (KR); KIM, Hyoung Rae, Daejeon 34302 (KR); LEE, Hyeun Joung, Daejeon 34302 (KR); LEE, Ju Young, Daejeon 34302 (KR); LEE, Kun Jung, Daejeon 34302 (KR); PARK, Yun-Hee, Daejeon 34302 (KR); PARK, Chang Sik, Daejeon 34302 (KR); OH, Hwan Hee, Daejeon 34302 (KR); OH, Jihye, Daejeon 34302 (KR); CHAE, Jeiwook, Daejeon 34302 (KR); KIM, Yong Zu, Daejeon 34302 (KR); CHAE, Sang Eun, Daejeon 34302 (KR); RYU, Hyunmin, Daejeon 34302 (KR); HAN, Nara, Daejeon 43402 (KR); CHOI, Min Ji, Daejeon 43402 (KR)
(74) Representative: Vossius & Partner Patentanwälte Rechtsanwälte mbB
(86) International application number: PCT/KR2020/000091
(87) International publication number: WO 2020/141923

(57) **Abstract**

The present invention relates to a novel pyrrolobenzodiazepine dimer compound or a pharmaceutically acceptable salt thereof, a ligand-drug conjugate compound thereof, or a composition containing the same and therapeutic use of the same as an anticancer agent. The pyrrolobenzodiazepine dimer compound according to the present invention exhibits anticancer activity equivalent to or superior to that of existing anticancer agents when being applied to a ligand-drug conjugate as a drug and administered as well as exhibits low activity and greatly diminished toxicity in the free toxin form and thus has a significantly improved therapeutic index. Hence, the pyrrolobenzodiazepine dimer compound is highly industrially applicable in that targeting of proliferative diseases such as cancer is possible, specific treatment of the proliferative diseases is possible, the drug efficacy can be maximized, and the expression of side effects can be minimized.

## Description

### [Technical Field]

The present invention relates to a novel pyrrolobenzodiazepine dimer compound or a pharmaceutically acceptable salt thereof, a ligand-drug conjugate compound thereof, or a composition containing the same and therapeutic use of the same as an anticancer agent. The pyrrolobenzodiazepine dimer compound according to the present invention exhibits anticancer activity equivalent to or superior to that of existing anticancer agents when being applied to a ligand-drug conjugate as a drug and administered as well as exhibits low activity and greatly diminished toxicity in the free toxin form and thus has a significantly improved therapeutic index. Hence, the pyrrolobenzodiazepine dimer compound is highly industrially applicable in that targeting of proliferative diseases such as cancer is possible, specific treatment of the proliferative diseases is possible, the drug efficacy can be maximized, and the expression of side effects can be minimized.

### [Background Art]

Pyrrolobenzodiazepines (PBD) are known as natural substances that are produced by various actinomycetes and exhibit antibiotic or antitumor activity. Pyrrolobenzodiazepines are sequence-selective DNA alkylating anticancer agents that covalently bind to cellular DNA. Pyrrolobenzodiazepines are DNA-crosslinking agents, known to exhibit significantly stronger anticancer activity than systemic chemotherapeutic agents, and can prevent the division of cancer cells without destroying the DNA helix.

Pyrrolobenzodiazepines have the following general structure:

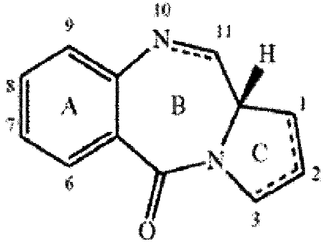

The pyrrolobenzodiazepines differ from one another in the number, type, and position of substituents on the aromatic ring A and the pyrrole ring C, and the degree of saturation of the ring C. In the ring B, there is an imine (N=C), carbinolamine (NH-CH(OH)), or carbinolamine methyl ether (NH-CH(OMe)) at the N10-C11 position that is the electrophilic center responsible for DNA alkylation.

It is known that antibody-drug conjugates using pyrrolobenzodiazepines as a cytotoxic drug are being developed in AbbVie, ADC therapeutics, ImmunoGen, Takeda, Medimmune, and the like. In addition, Spirogen has been developing a technology for treating acute myeloid leukemia based on pyrrolobenzodiazepines.

In this regard, there are a published patent related to pyrrolobenzodiazepines and their conjugates (Medimmune Limited, Patent Literature 1), a published patent related to an asymmetric pyrrolobenzodiazepine dimer for treatment of proliferative diseases (Medimmune Limited, Patent Literature 2), a registered patent related to pyrrolobenzodiazepines (Medimmune Limited, Patent Literature 3), a registered patent related to pyrrolobenzodiazepines for treatment of proliferative diseases (Medimmune Limited, Patent Literature 4), a published patent related to pyrrolobenzodiazepines (Medimmune Limited, Patent Literature 5), and a registered patent related to pyrrolobenzodiazepines (Spirogen Limited, Patent Literature 6). These merely disclose that antitumor activity is enhanced by modifying the structures of pyrrolobenzodiazepine compounds and the like or that such pyrrolobenzodiazepine compounds having modified structures can be administered in the form of antibody-drug conjugates to enhance the anticancer activity.

Meanwhile, there are a technology related to an antibody-drug conjugate in the form of a carbamate-linked pyrrolobenzodiazepine dimer, paper disclosing that low cytotoxicity and stability are achieved by using a monomeric pyrrolobenzodiazepine compound as a prodrug, and research paper related to the preparation and activity of N10-(4-nitrobenzyl)carbamate-protected pyrrolobenzodiazepine prodrugs (see Non Patent Literature 7, Non Patent Literature 8, and Non Patent Literature 9).

However, the technologies have limitations in that there is a problem that scaling up is not easy because of the low yield of pyrrolobenzodiazepines when synthesized and the technologies are insufficient to sufficiently solve the problem of poor stability in blood after administration. In the case of Seattle Genetics, that recently have had a number of pipelines as a pioneer in the ADC field, all clinical trials of SGN-CD123A (target: CD123), SGN-CD19B (target: CD19), and SGN-CD352A (target: CD352) into which pyrrolobenzodiazepine dimers are introduced have been discontinued since the clinical trial of SGN-CD33A for leukemia patients was discontinued because of increased mortality, and this is believed to be due to the strong toxicity of pyrrolobenzodiazepine dimers. ADCT-502 by ADC Therapeutics has also been discontinued because of safety issues due to toxicity of pyrrolobenzodiazepine dimers. However, the clinical trials of pyrrolobenzodiazepine dimer-ADCs against various targets possessed by ADC therapeutics are in progress.

Hence, there is a need for the development of a preparing method capable of increasing the yield of pyrrolobenzodiazepines and the development of a novel pyrrolobenzodiazepine dimer compound that can exhibit an effect equivalent to or superior to that of existing anticancer agents while exhibiting increased stability in blood after administration and diminished toxicity.

Antibody-drug conjugates (ADCs) are target-directed new technologies in which an antigen-binding antibody is bound to a toxin or drug and then the toxic substance is released inside the cells to lead the cancer cells to death. Antibody-drug conjugates (ADCs) are a technology that exhibits superior efficacy to the antibody therapeutic agent itself and can significantly diminish the risk of side effects as compared to existing anticancer agents since healthy cells are hardly affected, the drugs are precisely delivered to the target cancer cells, and the toxic substance is released only under certain conditions.

The basic structure of such antibody-drug conjugates is composed of an antibody-linker-low molecular weight drug (toxin). Here, the linker serves not only a functional role of simply connecting the antibody and the drug to each other but also stably reaches the target cell during circulation in the body and then allows the drug to enter the cell, easily fall off by the antibody-drug dissociation phenomenon (for example, as a result of enzymatic hydrolysis), and exhibit the effect on the target cancer cell.

The inventors of the present invention have developed a linker containing a self-immolative group, which is more stable in plasma, is stable even when circulating in the body, and allows the drug to be easily released in cancer cells and exhibit an effect and secured a patent for this (Korean Patent No. 1,628,872 Registered and the like).

### [Summary of Invention]

### [Technical Problem]

In the present invention, it is intended to develop a novel pyrrolobenzodiazepine dimer compound that can exhibit an effect equivalent to or superior to that of existing anticancer agents as well as does not exhibit toxicity in a free toxin state and thus exhibits increased stability in blood and decreased systemic side effects.

### [Solution to Problem]

The present invention has solved the above problems by providing a novel pyrrolobenzodiazepine dimer compound, a pharmaceutically acceptable salt thereof, or a solvate thereof.

More specifically, the pyrrolobenzodiazepine dimer compound according to the present invention has a structure represented by the following Chemical Formula I:

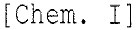

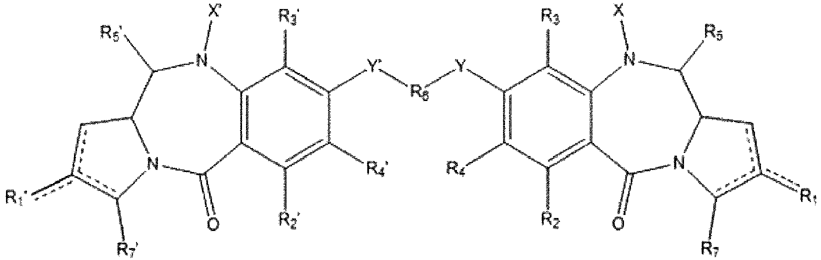

### [Advantageous Effects of Invention]

The pyrrolobenzodiazepine dimer compound according to the present invention exhibits anticancer activity equivalent to or superior to that of existing anticancer agents when being applied to a ligand-drug conjugate as a drug and administered as well as exhibits low activity and greatly diminished toxicity in the free toxin form and thus has a significantly improved therapeutic index. Hence, the pyrrolobenzodiazepine dimer compound has advantages that targeting of proliferative diseases such as cancer is possible, specific treatment of the proliferative diseases is possible, the drug efficacy can be maximized, and the expression of side effects can be minimized.

In the case of ligand-drug conjugates prepared by conventional methods, the content of impurities is high and there is a risk that the exposed imine group may be attacked by a nucleophile to produce a drug having an unwanted structure, but when the pyrrolobenzodiazepine dimer compound according to the present invention is prepared in the form of a ligand-drug conjugate, separation is easy because of high purity and the physical properties are further improved as compared to existing pyrrolobenzodiazepine compounds.

### [Brief Description of Drawings]

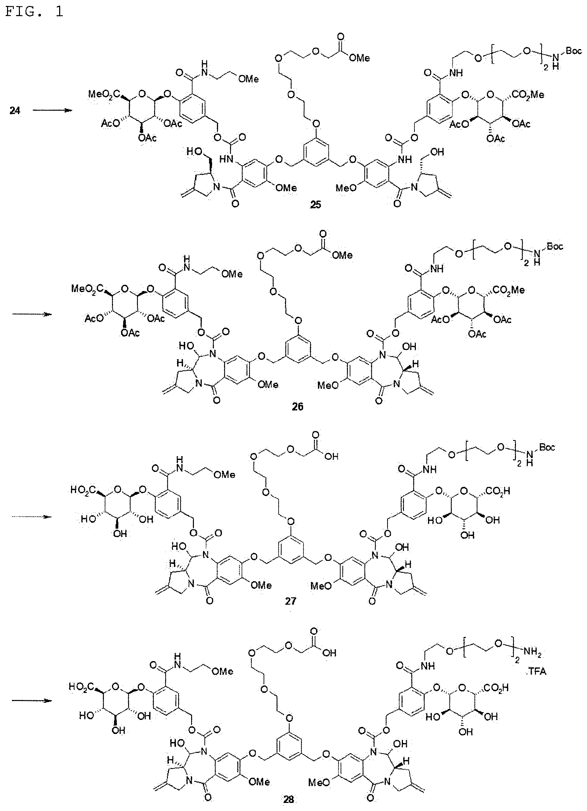

FIG. 1 schematically illustrates the process of synthesizing Compound 28 according to the present invention.
FIG. 2 is a graph illustrating the in vivo experimental results in the JIMT-1 xenograft model.
FIG. 3 is a graph illustrating the results of a single dose toxicity experiment in SD rats.
FIG. 4 is a graph illustrating the results of an experiment on the degree of Bystander effect.

### [Description of Embodiments]

Hereinafter, the present invention will be described in more detail.

The present invention relates to a pyrrolobenzodiazepine dimer compound having a structure represented by the following Chemical Formula I or a pharmaceutically acceptable salt or solvate thereof: 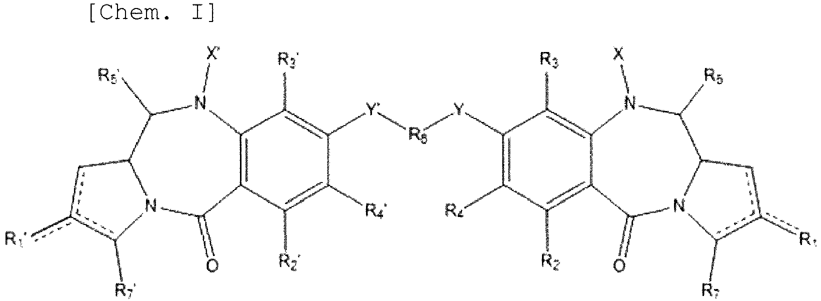 where
a dotted line indicates arbitrary presence of a double bond between C1 and C2 or between C2 and C3,
R₁ is selected from the group consisting of hydrogen, OH, =O, =CH₂, CN, R^{m}, OR^{m}, =CH-R^{m'} =C(R^{m'})₂, O-SO₂-R^{m}, CO₂R^{m}, COR^{m}, halo, and dihalo or may be one of those described in definition of R₆ below,
where R^{m} is selected from the group consisting of substituted or unsubstituted C₁₋₁₂ alkyl, substituted or unsubstituted C₂₋₁₂ alkenyl, substituted or unsubstituted C₂₋₁₂ alkynyl, substituted or unsubstituted C₅₋₂₀ aryl, substituted or unsubstituted C₅₋₂₀ heteroaryl, substituted or unsubstituted C₃₋₆ cycloalkyl, substituted or unsubstituted 3- to 7-membered heterocyclyl, substituted or unsubstituted 3- to 7-membered heterocycloalkyl, and substituted or unsubstituted 5- to 7-membered heteroaryl,
where when C₁₋₁₂ alkyl, C₂₋₁₂ alkenyl, C₂₋₁₂ alkynyl, C₅₋₂₀ aryl, C₅₋₂₀ heteroaryl, C₃₋₆ cycloalkyl, 3- to 7-membered heterocyclyl, 3- to 7-membered heterocycloalkyl, or 5- to 7-membered heteroaryl is substituted, hydrogen atoms of C₁₋₁₂ alkyl, C₁₋₁₂ alkoxy, C₂₋₁₂ alkenyl, C₂₋₁₂ alkynyl, C₅₋₂₀ aryl, C₅₋₂₀ heteroaryl, C₃₋₆ cycloalkyl, 3- to 7-membered heterocyclyl, 3- to 7-membered heterocycloalkyl, or 5- to 7-membered heteroaryl are each independently substituted with any one or more selected from the group consisting of C₁₋₁₂ alkyl, C₂₋₁₂ alkenyl, C₂₋₁₂ alkynyl, C₅₋₂₀ aryl, C₅₋₂₀ heteroaryl, C₃₋₆ cycloalkyl, 3-to 7-membered heterocyclyl, 3- to 7-membered heterocycloalkyl, and 5- to 7-membered heteroaryl, and
R^{m'} is selected from the group consisting of R^{m}, CO₂R^{m}, COR^{m}, CHO, CO₂H, and halo;
R₂, R₃, and R₅ are each independently selected from the group consisting of H, R^{m}, OH, OR^{m}, SH, SR^{m}, NH₂, NHR^{m}, NR^{m}R^{m'}, NO₂, Me₃Sn, and halo,
where R^{m} and R^{m'} are as defined above;
R₄ is selected from the group consisting of hydrogen, R^{m}, OH, OR^{m}, SH, SR^{m}, NH₂, NHR^{m}, NR^{m}R^{m'}, NO₂, Me₃Sn, halo, substituted or unsubstituted C₁₋₆ alkyl, substituted or unsubstituted C₁₋₆ alkoxy, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 7-membered heterocycloalkyl, substituted or unsubstituted C₅₋₁₂ aryl, substituted or unsubstituted 5- to 7-membered heteroaryl, -CN, -NCO, -ORⁿ, -OC(O)Rⁿ, -OC(O)NRⁿR^{n'}, -OS(O)R^{n,} -OS(O)₂Rⁿ, -SRⁿ, -S(O)Rⁿ, - S(O)₂Rⁿ, -S(O)NRⁿR^{n'}, -S(O)₂NRⁿR^{n'}, -OS(O)NRⁿR^{n'}, -OS(O)₂NRⁿR^{n'}, -NRⁿR^{n'}, -NRⁿC(O)R^{o}, -NRⁿC(O)OR^{o}, -NRⁿC(O)NR^{o}R^{o'}, -NRⁿS(O)R^{o}, -NRⁿS(O)₂R^{o}, -NRⁿS(O)NR^{o}R^{o'}, -NRnS(O)₂NR^{o}R^{o'}, -C(O)Rⁿ, - C(O)ORⁿ, and -C(O)NRⁿR^{n'},
where when C₁₋₆ alkyl, C₁₋₆ alkoxy, C₂₋₆ alkenyl, C₂₋₆ alkynyl, C₃₋₆ cycloalkyl, 3- to 7-membered heterocycloalkyl, C₅₋₁₂ aryl, or 5- to 7-membered heteroaryl is substituted, hydrogen atoms of C₁₋₆ alkyl, C₁₋₆ alkoxy, C₂₋₆ alkenyl, C₂₋₆ alkynyl, C₃₋₆ cycloalkyl, 3-to 7-membered heterocycloalkyl, C₅₋₁₂ aryl, or 5- to 7-membered heteroaryl may each be independently substituted with C₁₋₆ alkyl, C₁₋₆ alkoxy, C₂₋₆ alkenyl, C₂₋₆ alkynyl, C₃₋₆ cycloalkyl, 3- to 7-membered heterocycloalkyl, C₅₋₁₀ aryl, 5- to 7-membered heteroaryl, -OR^{p}, -OC(O)R^{p}, -OC(O)NR^{p}_{R}^{p'}, -OS(O)RP, -OS(O)₂R^{p}, -SRP, -S(O)R^{p}, -S(O)₂R^{p}, -S(O)NR^{p}R^{p'}, - S(O)₂NR^{p}R^{p'}, -OS(O)NR^{p}R^{p'}, -OS(O)₂NR^{p}R^{p'}, -NR^{p}R^{p'}, -NR^{p}C(O)R^{q}, -NR^{p}C(O)OR^{x}, -NR^{p}C(O)NR^{x}R^{x'}, -NR^{p}S(O)R^{x}, -NR^{p}S(O)₂R^{x}, - NR^{p}S(O)NR^{x}R^{x'}, -NR^{p}S(O)₂NR^{x}R^{x'}, -C(O)R^{p}, -C(O)OR^{p}, or - C(O)NR^{p}R^{p},
where Rⁿ, R^{o}, R^{p}, R^{x}, R^{n'}, R^{o'}, RP', and R^{x'} are each independently selected from the group consisting of H, C₁₋₇ alkyl, C₂₋₇ alkenyl, C₂₋₇ alkynyl, C₃₋₁₃ cycloalkyl, 3- to 7-membered heterocycloalkyl, C₆₋₁₀ aryl, and 5- to 7-membered heteroaryl, or
R₄ may be one of those described in definition of R₆ below;
any one selected from the group consisting of - C(O)O*, -S(O)O*, -C(O)*, -C(O)NR*, -S(O)₂NR*, -P(O)R'NR*, S(O)NR*, and -PO₂NR* groups is independently attached to each of X and X',
where * is a portion to which a linker is attached, and
R and R' are each independently H, OH, N₃, CN, NO₂, SH, NH₂, ONH₂, NHNH₂, halo, substituted or unsubstituted C₁₋₈ alkyl, substituted or unsubstituted C₃₋₈ cycloalkyl, substituted or unsubstituted C₁₋₈ alkoxy, substituted or unsubstituted C₁₋₃ alkylthio, substituted or unsubstituted C₃-₂₀ heteroaryl, substituted or unsubstituted C₅₋₂₀ aryl, or mono- or di-C₁₋₈ alkylamino;
where C₁₋₈ alkyl, C₃₋₈ cycloalkyl, C₁₋₈ alkoxy, C₁₋₈ alkylthio, C₃-₂₀ heteroaryl, or C₅₋₂₀ aryl is substituted with a substituent selected from the group consisting of H, OH, N₃, CN, NO₂,SH, NH₂, ONH₂, NNH₂, halo, C₁₋₆ alkyl, C₁₋₆ alkoxy, and C₅₋₁₂ aryl when substituted;
Y and Y' are each independently selected from the group consisting of 0, S, and N(H);
R₆ is a polar functional group having a structure represented by the following Chemical Formula II,

[Chem. II] -A-B₁-B₂-C

where
A is C₁₋₆ alkyl, C₂₋₆ alkenyl, C₂₋₆ alkynyl, C₃-₂₀ aryl, C₃₋₈ cycloalkyl, 3- to 7-membered-heterocycloalkyl, or 5-to 7-membered-heteroaryl that is unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl or siloxy,
B₁ is a bond, -0-, -NH-, -S-, -CO₂, -CONR^{m}-, -CONR^{m}R^{m'}, -CH=CH-, -C≡C-, -N-, or -P- or may be a branching unit,
where the branching unit is C₂₋₁₀₀ alkenyl (where carbon atoms of alkenyl may be substituted with one or more heteroatoms selected from the group consisting of N, 0, and S, and alkenyl may be further substituted with one or more C₁₋₂₀ alkyl), a hydrophilic amino acid, -C(O)-, - C(O)NR''''-, -C(O)O-, -(CH₂)ₛ-NHC(O)-(CH₂)ₜ-, -(CH₂)ᵤ-C(O)NH-(CH₂)ᵥ-, -(CH₂)ₛ-NHC(O)-(CH₂)ₜ-C(O)-, -(CH₂)ᵤ-C(O)NH-(CH₂)ᵥ-C(O)-, -S(O)₂NR''''-, -P(O)R'''''NR''''-, - S(O)NR''''-, or -PO₂NR''''- (where R'''' and R''''' are each independently H, C₁₋₈ alkyl, C₃₋₈ cycloalkyl, C₁₋₈ alkoxy, C₁₋₈ alkylthio, mono- or di-C₁₋₈ alkylamino, C₃-₂₀ heteroaryl, or C₅₋₂₀ aryl; and s, t, u, and v are each independently an integer from 0 to 10),
B₂ is a bond or has a structure of -(CH₂)ᵣ(V(CH₂)ₚ)_{q}-, -((CH₂)pV)_{q}-, -V(Y(CH₂)p)_{q}-, -(CH₂)ᵣ(V(CH₂)p)_{q}Y-, - ((CH₂)ₚV)_{q}(CH₂)ᵣ-, -Y((CH₂)pV)_{q}-, or -(CH₂)ᵣ(V (CH₂)ₚ)_{q}YCH₂-, where r is an integer from 0 to 10, p is an integer from 1 to 10, q is an integer from 1 to 20, V and Y are each independently a single bond, -O-, -S-, -NR₂₁-, -C(O)NR₂₂-, -NR₂₃C(O)-, -NR₂₃C(O)R₂₄-, -NR₂₄SO₂-, or -SO₂NR₂₅-, and R₂₁ to R₂₅ are each independently hydrogen, (C₁-₆)alkylene, (C₁₋₆)alkyl, (C₁-₆)alkyl (C₆₋₂₀)aryl, or (C₁-₆)alkyl (C₃₋₂₀)heteroaryl, and
C is selected from the group consisting of -R^{q}OR^{r}, - OR^{r}, -OC(O)OR^{r}, -R^{q}OC(O)OR^{r}, -C(O)OR^{r}, -R^{q}C(O)OR^{r}, -C(O)R^{r}, -R^{q}C(O)R^{r}, -OC(O)R^{r}, -R^{q}OC(O)R^{r}, -(R^{q}O)ₙ-OR^{r}, -(OR^{q})ₙ-OR^{r}, - C(O)-O-C(O)R^{r}, -R^{q}C(O)-O-C(O)R^{r}, -SR^{r}, -R^{q}SR^{r}, -SSR^{r}, - R^{q}SSR^{r}, -S(=O)R^{r}, -R^{q}S(=O)R^{r}, -R^{q}C(=S)R^{r}-, -R^{q}C(=S)SR^{r}, - R^{q}SO₃R^{r}, -SO₃R^{r}, -CN, -R^{q}CN, 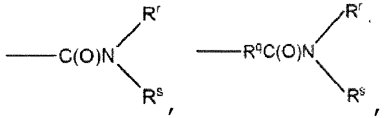 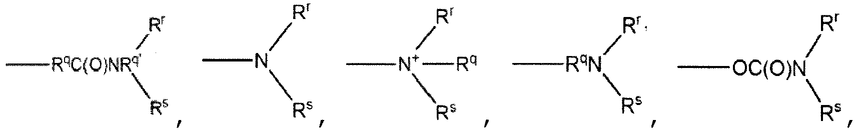 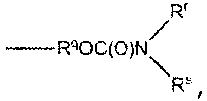 -SO₂R^{q}, -R^{q}SO₂R^{r}, -OSO₂R^{r}, -R^{q}OSO₂R^{r}, -OSO₂OR^{r}, -R^{q}OSO₂OR^{r}, 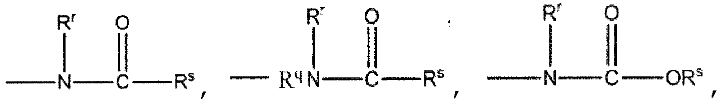 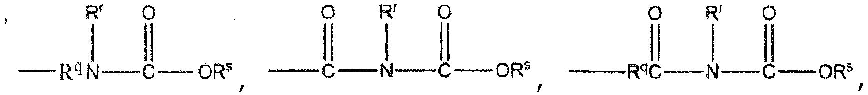 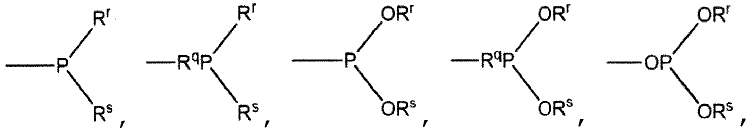 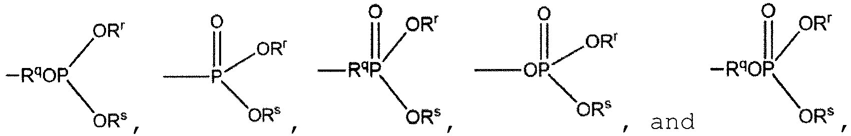
where R^{q} and R^{q'} are the same as or different from each other and are each independently C₁₋₂₀ linear or branched alkylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy;
C₂₋₂₀ linear or branched alkenylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy;
C₂₋₂₀ linear or branched alkynylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy;
C₃₋₁₂ cycloalkylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy;
C₆₋₄₀ arylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy;
C₁₋₂₀ alkoxylylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy; or
C₁₋₂₀ carbonyloxylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy;
R^{r} and R^{s} are the same as or different from each other and are each independently hydrogen; halogen;
C₁₋₂₀ linear or branched alkyl unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy;
C₂₋₂₀ linear or branched alkenyl unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy;
C₂₋₂₀ linear or branched alkynyl unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy;
C₃₋₁₂ cycloalkyl unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy;
C₆₋₄₀ aryl unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy;
C₁₋₂₀ alkoxy unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy; or
C₁₋₂₀ carbonyloxy unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy, and
n is each independently an integer from 1 to 10; and
R₇ is H, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 7-membered heterocycloalkyl, substituted or unsubstituted C₆₋₁₀ aryl, substituted or unsubstituted 5- to 7-membered heteroaryl, -OR^{t}, -OC(O)R^{t}, -OC(O)NR^{t}R^{t'}, -OS(O)R^{t}, - OS(O)₂R^{t}, -SR^{t}, -S(O)R^{t}, -S(O)₂R^{t}, -S(O)NR^{t}R^{t'}, -S(O)₂NR^{r}R^{r'}, -OS(O)NR^{t}R^{t'}, -OS(O)₂NR^{t}R^{t'}, -NR^{t}R^{t'}, -NR^{t}C(O)R^{u}, -NR^{t}C(O)OR^{u}, -NR^{t}C(O)NR^{u}R^{u'}, -NR^{t}S(O)R^{u}, -NR^{t}S(O)₂R^{u}, -NR^{t}S(O)NR^{u}R^{u'}, - NR^{t}S(O)₂NR^{u}R^{u}, -C(O)R^{t}, -C(O)OR^{u}, or -C(O)NR^{t}R^{t'},
where when C₁₋₆ alkyl, C₂₋₆ alkenyl, C₂₋₆ alkynyl, C₃₋₆ cycloalkyl, 3- to 7-membered heterocycloalkyl, C₆₋₁₀ aryl, or 5- to 7-membered heteroaryl is substituted, hydrogen atoms of C₁₋₆ alkyl, C₂₋₆ alkenyl, C₂₋₆ alkynyl, C₃₋₆ cycloalkyl, 3- to 7-membered heterocycloalkyl, C₆₋₁₀ aryl, or 5- to 7-membered heteroaryl are each independently substituted with C₁₋₆ alkyl, C₂₋₆ alkenyl, C₂₋₆ alkynyl, C₃₋₆ cycloalkyl, 3- to 7-membered heterocycloalkyl, C₆₋₁₀ aryl, 5- to 7-membered heteroaryl, -OR^{v}, -OC(O)R^{v}, -OC(O)NR^{v}R^{v'}, -OS(O)R^{v}, -OS(O)₂R^{v}, -SR^{v}, -S(O)R^{v}, -S(O)₂R^{v}, -S(O)NR^{v}R^{v'}, - S(O)₂NR^{v}R^{v'}, -OS(O)NR^{v}R^{v'}, -OS(O)₂NR^{v}R^{v'}, -NR^{v}R^{v'}, -NR^{v}C(O)R^{w}, -NR^{v}C(O)OR^{w}, -NR^{v}, C(O)NR^{w}R^{w'}, -NR^{v}S(O)R^{w}, -NR^{v}S(O)₂R^{w}, - NR^{v}S(O)NR^{w}R^{w'}, -NR^{v}S(O)₂NR^{w}R^{w'}, -C(O)R^{v}, - C(O)OR^{v}, or - C(O)NR^{v}R^{v'},
where R^{t}, R^{t'}, R^{u}, R^{u'}, R^{v}, R^{v'}, R^{w}, and R^{w'} are each independently selected from the group consisting of H, C₁₋₇ alkyl, C₂₋₇ alkenyl, C₂₋₇ alkynyl, C₃₋₁₃ cycloalkyl, 3- to 7-membered heterocycloalkyl, C₅₋₁₀ aryl, and 5- to 7-membered heteroaryl.

In an aspect of the present invention, R₁ in Chemical Formula I may be selected from the group consisting of H, OH, =0, =CH₂, CN, and C₁₋₆ alkyl.

In an aspect of the present invention, R₂, R₃, and R₅ in Chemical Formula I may each be independently selected from the group consisting of H, OH, and OR^{m}, where R^{m} is as defined above.

In an aspect of the present invention, R₄ in Chemical Formula I may be selected from the group consisting of H, R^{m}, OH, and OR^{m}, where R^{m} is C₁₋₆ alkyl.

In an aspect of the present invention, Y and Y' in Chemical Formula I may be 0.

In an aspect of the present invention, R₆ in Chemical Formula I may have a structure represented by the following Chemical Formula II:

[Chem. II] -A-B₁-B₂-C

where
A may be C₁₋₆ alkyl or C₃-₂₀ aryl;
B₁ may be selected from the group consisting of -O-, -NH-, -C≡C-, and -CONR^{m}R^{m'}, wherein R^{m} and R^{m'} are each independently hydrogen or C₁₋₆ alkyl;
B₂ may be -((CH₂)ₚV)_{q}- or -V(Y(CH₂)ₚ)_{q}-,
where V may be 0 or -NR₂₃C(O)R₂₄- (where R₂₃ and R₂₄ may each be independently hydrogen or C₁₋₆ alkylene),
p may be 2, and
q may be an integer from 1 to 10; and 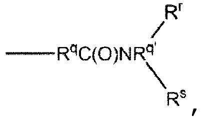
where R^{q} and R^{q'} may each be independently C₁₋₆ alkylene, C₂₋₆ alkenylene, or C₂₋₆ alkynylene, and
R^{r} and R^{s} may each be independently selected from the group consisting of hydrogen, carboxyl, C₁₋₆ alkyl substituted with carboxyl, and C₁₋₆ alkyl.

In an aspect of the present invention, R₇ in Chemical Formula I may be hydrogen, C₁₋₆ alkyl, or OR^{t} (where R^{t} may be H or C₁₋₇ alkyl) .

In an aspect of the present invention, X and X' in Chemical Formula I may each be independently selected from the group consisting of -C(O)O*, -C(O)*, and - C(O)NR*, where each R may be independently H, OH, N₃, CN, NO₂,SH, NH₂, ONH₂, NNH₂, halo, C₁₋₃ alkyl, or C₁₋₃ alkoxy.

In an aspect of the present invention, the pyrrolobenzodiazepine dimer compound may be one selected from the group consisting of 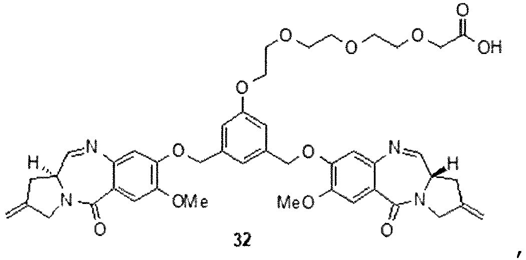 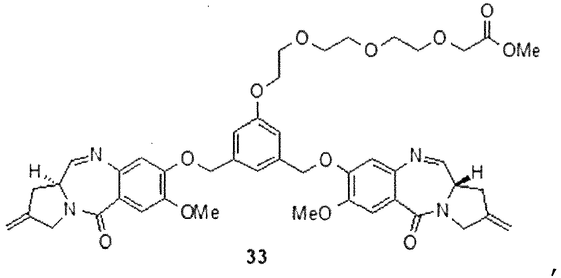 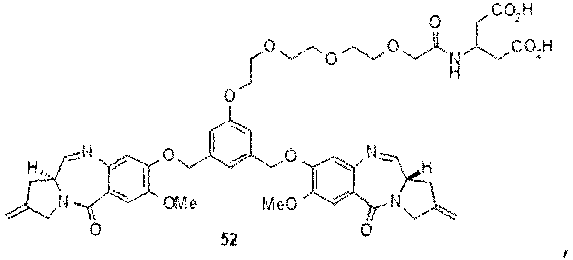 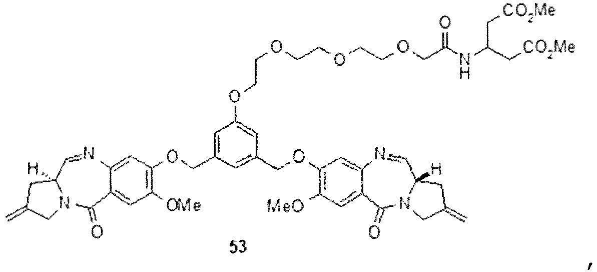 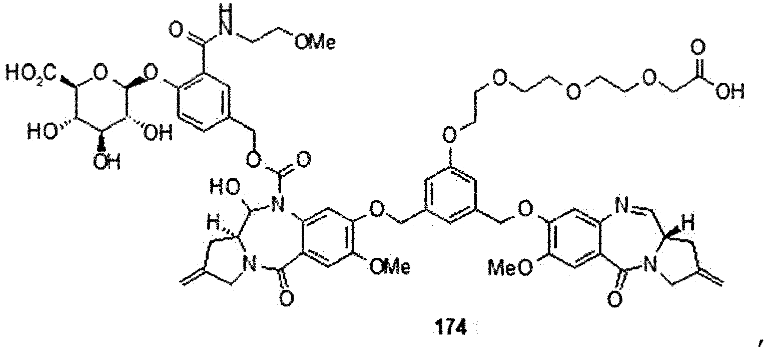 and

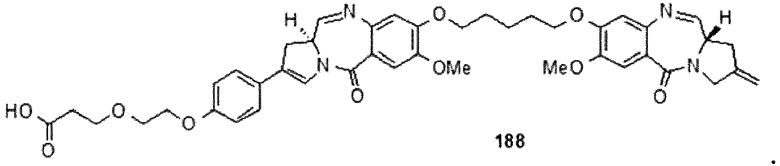

In another aspect of the present invention, there is provided a conjugate having a structure represented by the following Chemical Formula III or a pharmaceutically acceptable salt or solvate thereof:

[Chem. III] **Ligand** - **(L** - **D)n**

where
**Ligand** is a ligand,
L is a linker,
D is the pyrrolobenzodiazepine dimer compound described above,
where the linker is bound to D through N10 position, N10' position, or N10 and N10' positions of D, and
n is an integer from 1 to 20.

In an aspect of the present invention, the linker in Chemical Formula III may be bound to the pyrrolobenzodiazepine dimer compound according to an aspect of the present invention described above through X and X' of the compound.

In an aspect of the present invention, n in Chemical Formula III may be an integer from 1 to 10.

In still another aspect of the present invention, there is provided a pyrrolobenzodiazepine dimer-linker compound having a structure represented by the following Chemical Formula IV or a pharmaceutically acceptable salt or solvate thereof: 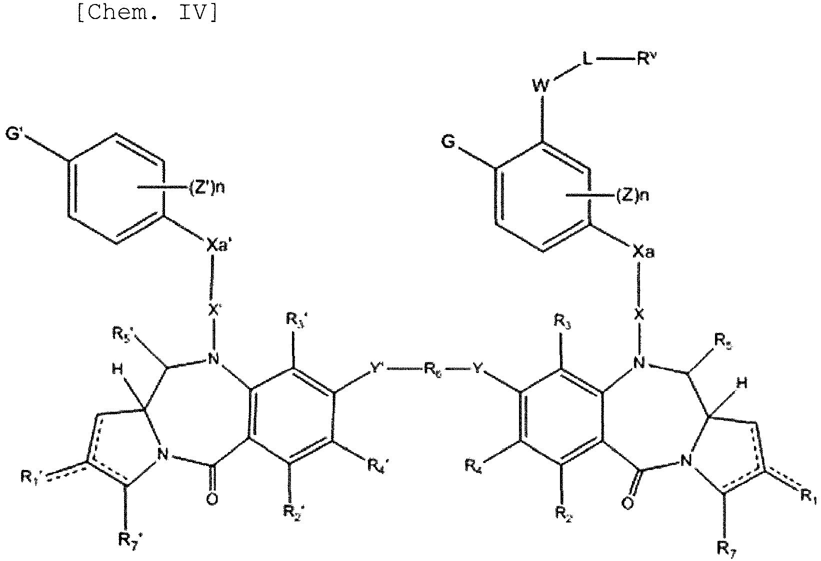 where
a dotted line, R₁, R₂, R₃, R₄, R₅, R₆, R₇, X, Y, R₁' , R₂', R₃', R₄', R₅' , R₇', X', and Y' are each as defined for those of the compound represented by Chemical Formula I;
Xa and Xa' are each independently a bond or substituted or unsubstituted C₁₋₆ alkylene, where C₁₋₆ alkylene is substituted with hydrogen, C₁₋₈ alkyl, or C₃₋₈ cycloalkyl when substituted;
G and G' is a glucuronide group, a galactoside group, or a derivative thereof;
Z is selected from the group consisting of H, C₁₋₈ alkyl, halo, NO₂,CN, 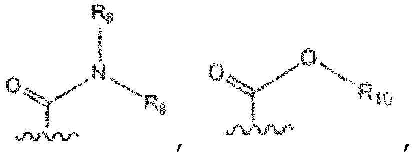 and - (CH₂)ₘ-OCH₃;
n is an integer from 1 to 3, and each Z may be the same as or different from each other when n is an integer 2 or more;
W is -C(O)-, -C(O)NR''-, -C(O)O-, -S(O)₂NR''-, - P(O)R'''NR''-, -S(O)NR''-, or -PO₂NR''-, where R'' and R''' are each independently H, C₁₋₈ alkyl, C₃₋₈ cycloalkyl, C₁₋₃ alkoxy, C₁₋₈ alkylthio, mono- or di-C₁₋₈ alkylamino, C₃₋₂₀ heteroaryl, or C₆₋₂₀ aryl;
L is one or more units selected from the group consisting of a branching unit, a connection unit, and a binding unit, or a combination of these units,
where the connection unit connects W with a binding unit, W with a branching unit, a branching unit with another branching unit, or a branching unit with a binding unit, the branching unit connects a connection unit with W or a connection unit with another connection unit,
the branching unit is C₂₋₁₀₀ alkenyl (where carbon atoms of alkenyl may be substituted with one or more heteroatoms selected from the group consisting of N, O, and S, and alkenyl may be further substituted with one or more C₁₋₂₀ alkyl), a hydrophilic amino acid, -C(O)-, - C(O)NR''''-, -C(O)O-, -(CH₂)ₛ-NHC(O)-(CH₂)t-, -(CH₂)ᵤ-C(O)NH-(CH₂)ᵥ-, -(CH₂)ₛ-NHC(O)-(CH₂)ₜ-C(O)-, -(CH₂)ᵤ-C(O)NH-(CH₂)ᵥ-C(O)-, -S(O)₂NR''''-, -P(O)R'''''NR''''-, - S(O)NR''''-, or -PO₂NR''''- (where R'''' and R''''' are each independently H, C₁₋₈ alkyl, C₃₋₈ cycloalkyl, C₁₋₈ alkoxy, C₁₋₈ alkylthio, mono- or di-C₁₋₈ alkylamino, C₃-₂₀ heteroaryl, or C₅₋₂₀ aryl; and s, t, u, and v are each independently an integer from 0 to 10),
the connection unit is -(CH₂)ᵣ(V(CH₂)ₚ)_{q}-, where r is an integer from 0 to 10, p is an integer from 0 to 12, q is an integer from 1 to 20, V is a single bond, -0-, or - S-, and
the binding unit is 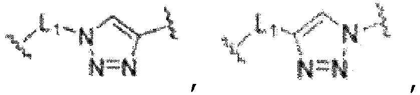 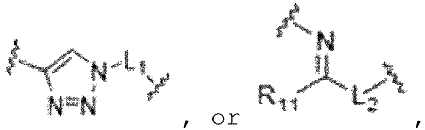 where L₁ is a single bond or C2-30 alkenyl, R₁₁ is H or C₁₋₁₀ alkyl, and L₂ is C₂₋₃₀ alkenyl; and
R^{v} is -NH₂, N₃, substituted or unsubstituted C₁₋₁₂ alkyl, C₁₋₁₂ alkynyl, C₁₋₃ alkoxy, substituted or unsubstituted C₃-₂₀ heteroaryl, C₃-₂₀ heterocyclyl, or substituted or unsubstituted C₅₋₂₀ aryl,
where when C₁₋₁₂ alkyl, C₃-₂₀ heteroaryl, C₃₋₂₀ heterocyclyl, or C₅₋₂₀ aryl is substituted, one or more hydrogen atoms present in C₃-₂₀ heteroaryl, C₃-₂₀ heterocyclyl, or C₅₋₂₀ aryl are each independently substituted with OH, =O, halo, C₁₋₆ alkyl, C₁₋₆ alkoxy, C₂₋₆ alkenyl oxy, carboxy, C₁₋₆ alkoxycarbonyl, C₁₋₆ alkylcarbonyl, formyl, C₃₋₈ aryl, C₅₋₁₂ aryloxy, C₅₋₁₂ arylcarbonyl, or C₃₋₆ heteroaryl.

In an aspect of the present invention, Xa and Xa' in Chemical Formula IV may each be independently a bond or C₁₋₃ alkyl.

In an aspect of the present invention, Z in Chemical Formula IV may be selected from the group consisting of H, 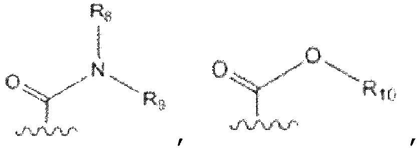 and - (CH₂)ₘ-OCH₃, where R₈, R₉, and R₁₀ may each be independently selected from the group consisting of H, C₁₋₃ alkyl, and C₁₋₃ alkoxy and m may be 1 to 6.

In an aspect of the present invention, W in Chemical Formula IV may be -C(O)-, -C(O)NR'''-, or -C(O)O-, where R''' may be H or C₁₋₈ alkyl.

In an aspect of the present invention, L in Chemical Formula IV may be one or more units selected from the group consisting of a branching unit, a connection unit, and a binding unit, or a combination of these units,
where the connection unit may connect W with a binding unit, W with a branching unit, a branching unit with another branching unit, or a branching unit with a binding unit, the branching unit may connect a connection unit with W or a connection unit with another connection unit,
the branching unit may be C₂₋₁₀₀ alkenyl (where carbon atoms of alkenyl may be substituted with one or more heteroatoms selected from the group consisting of N, 0, and S, and alkenyl may be further substituted with one or more C₁₋₆ alkyl), a hydrophilic amino acid, -C(O)-, - C(O)NR''''-, -C(O)O-, -(CH₂)ₛ-NHC(O)-(CH₂)ₜ-, -(CH₂)ᵤ-C(O)NH-(CH₂)ᵥ-, -(CH₂)ₛ-NHC(O)-(CH₂)ₜ-C(O)-, or -(CH₂)ᵤ-C(O)NH-(CH₂)ᵥ-C(O)- (where R'''' may be H, C₁₋₈ alkyl, C₃₋₈ cycloalkyl, C₁₋₈ alkoxy, C₁₋₈ alkylthio, mono- or di-C₁₋₈ alkylamino, C₃-₂₀ heteroaryl, or C₅₋₂₀ aryl; and s, t, u, and v may each be independently an integer from 0 to 5),
the connection unit may be -(CH₂)ᵣ(V(CH₂)ₚ)_{q}-, where r may be an integer from 0 to 10, p may be an integer from 0 to 12, q may be an integer from 1 to 20, V may be a single bond or -O-,
the binding unit may be 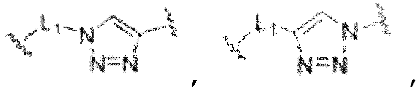 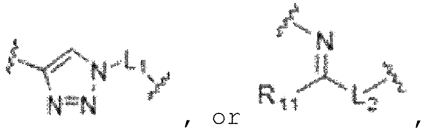 where L₁ may be a single bond or C₂₋₈ alkenyl, R₁₁ may be H or C₁₋₆ alkyl, and L₂ may be C₂₋₈ alkenyl, and
the cennection unit may be -(CH₂)ᵣ(V(CH₂)ₚ)_{q}-, where r may be an integer from 0 to 8, p may be an integer from 1 to 12, q may be an integer from 1 to 10, and V may be a single bond or -0-.

In an aspect of the present invention, the pyrrolobenzodiazepine dimer-linker compound may be one selected from the group consisting of 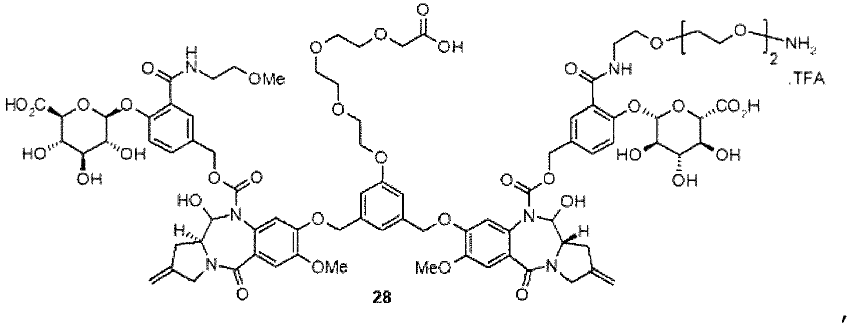 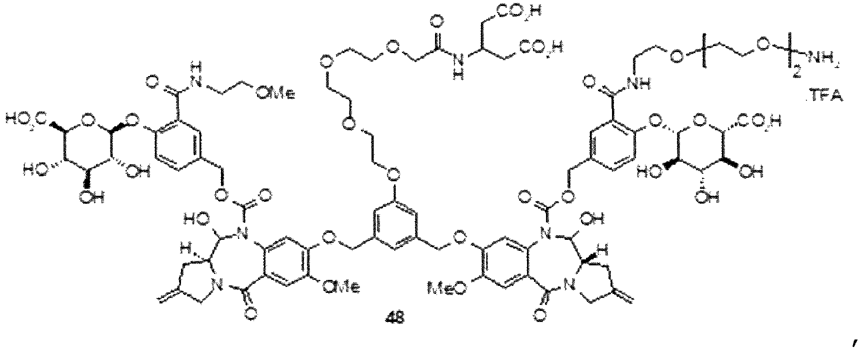 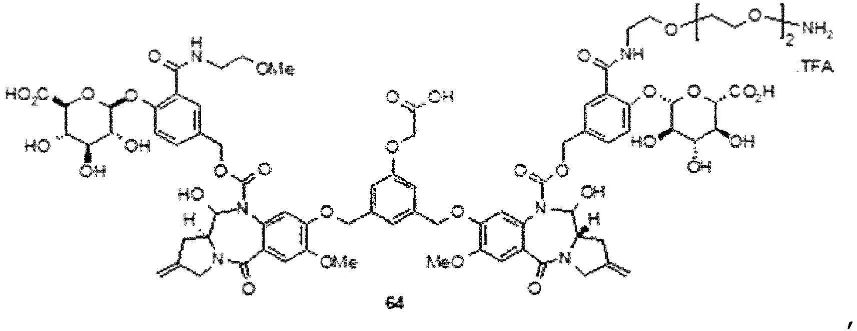 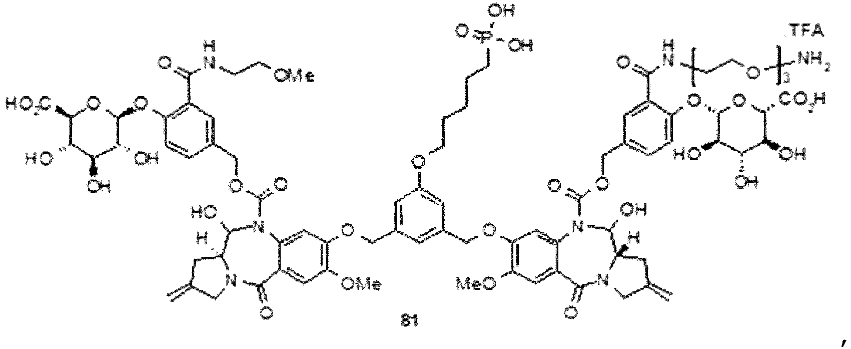 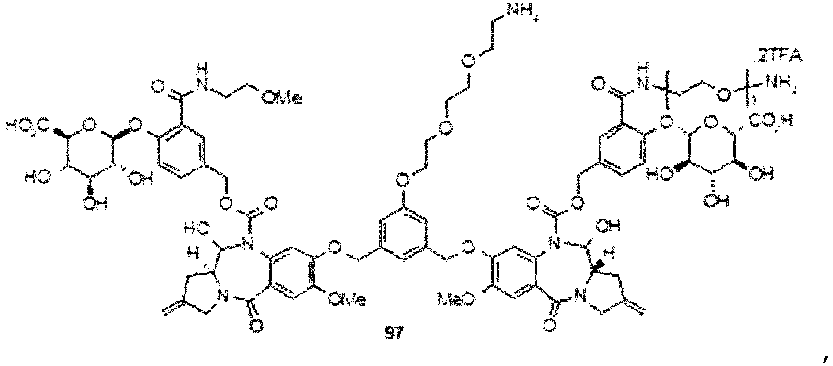 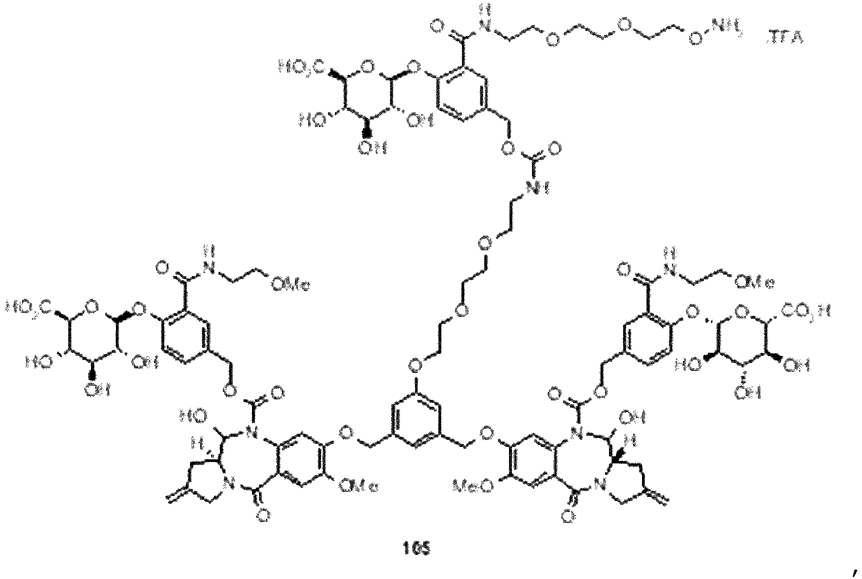 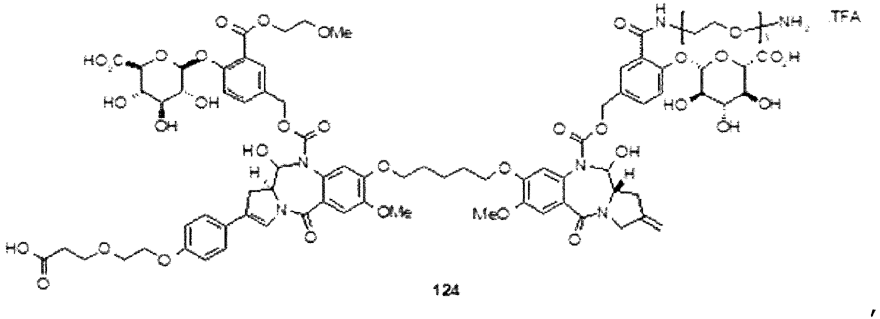 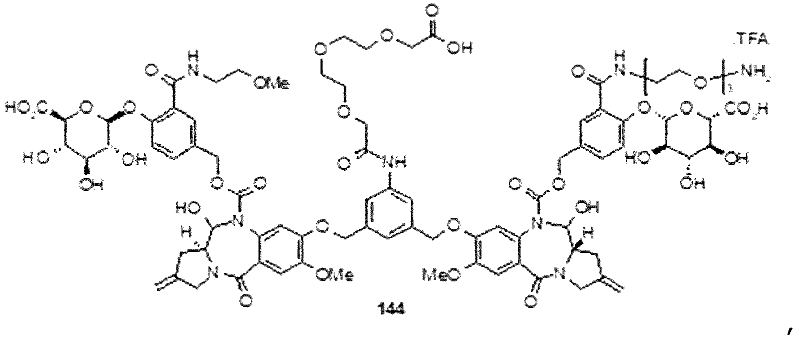 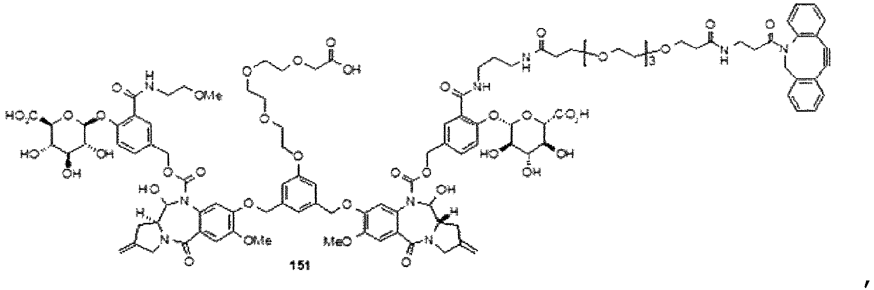 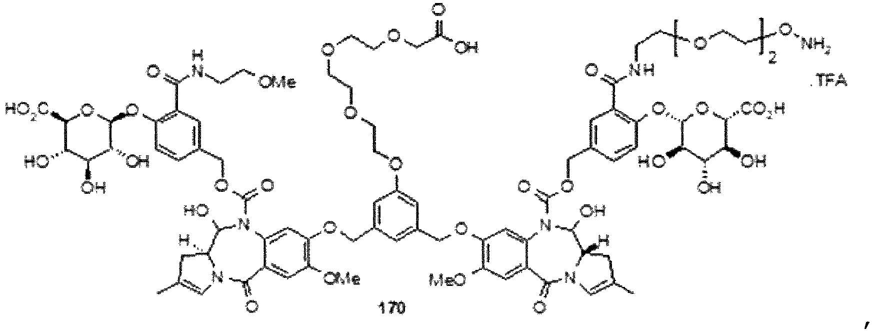 and

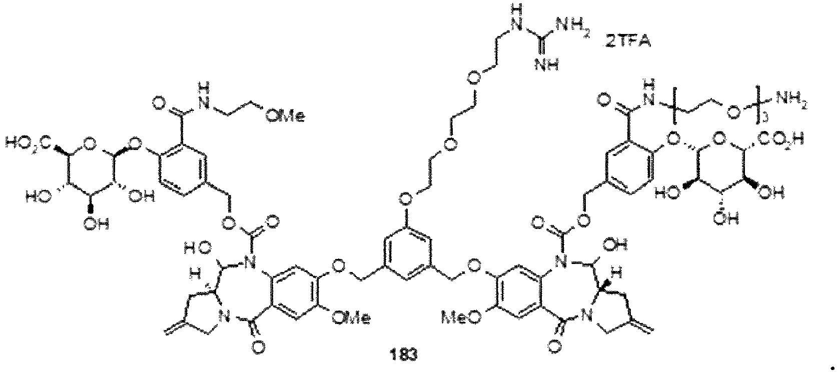

In still another aspect of the present invention, there is provided a pyrrolobenzodiazepine dimer-linker-ligand conjugate having a structure represented by the following Chemical Formula V or a pharmaceutically acceptable salt or solvate thereof: 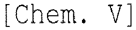 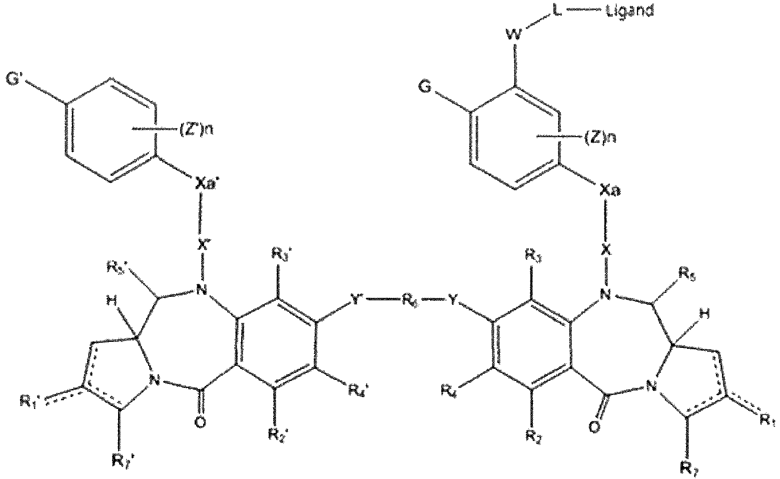 where
a dotted line, R₁, R₂, R₃, R₄, R₅, R₆, R₇, X, Y, R₁', R₂', R₃', R₄' , R₅', R₇', X', and Y' are each as defined for those of the compound represented by Chemical Formula I;
Xa, G, Z, W, L, Xa', G', and Z' are each as defined for those of the compound represented by Chemical Formula IV, and
Ligand is an antigen binding moiety.

In an aspect of the present invention, Ligand in Chemical Formula V may be a protein.

In an aspect of the present invention, the protein may be an oligopeptide, a polypeptide, an antibody, a fragment of an antigenic polypeptide, or a repebody.

In an aspect of the present invention, Ligand in Chemical Formula V may be an antibody, where the antibody may be an anti-HER2 antibody, an anti-DLKl antibody, an anti-RORl antibody, an anti-MUCl antibody, a CD19 antibody, or an anti-CD276 antibody.

In an aspect of the present invention, the protein may have one or more amino acid motifs recognizable by isoprenoid transferase, that is, the C-terminus (fragment or analog or derivative thereof) of the protein may be bound to an amino acid motif recognizable by isoprenoid transferase. A spacer unit composed of an amino acid, an oligopeptide, or a polypeptide may be further included between the protein and the amino acid motif. In an aspect of the present invention, the protein is covalently bound to a linker through an amino acid motif. In an aspect of the present invention, the amino acid motif may be covalently bound to the C-terminus of the protein or covalently bound to at least one spacer unit covalently bound to the C-terminus of the protein. The protein may be directly covalently bound to the amino acid motif or covalently bound to a spacer unit to be linked to the amino acid motif. The amino acid spacer unit is composed of 1 to 20 amino acids, among which a glycine unit is preferable.

In an aspect of the present invention, the C-terminus of the protein is that of a light or heavy chain of an antibody.

In an aspect of the present invention, the protein is a monoclonal antibody.

In an aspect of the present invention, the isoprenoid transferase may be FTase (farnesyl protein transferase) or GGTase (geranylgeranyl transferase).

In an aspect of the present invention, the isoprenoid transferase includes FTase (farnesyl protein transferase) or GGTase (geranylgeranyl transferase), and these each involve the transfer of a farnesyl or geranylgeranyl residue to the C-terminal cysteine(s) of the target protein. GGTase may be classified into GGTase I and GGTase II. FTase and GGTase I can recognize the CAAX motif.

In an aspect of the present invention, the amino acid motif may be CYYX, XXCC, XCXC, or CXX, where C may be cysteine, Y may be an aliphatic amino acid, and X may be an amino acid that determines substrate specificity of isoprenoid transferase.

In an aspect of the present invention, the protein having an amino acid motif is selected from the group consisting of A-HC-(G)_{z}CVIM, A-HC-(G)_{z}CVLL, A-LC-(G)_{z}CVIM, and A-LC-(G)zCVLL, where A represents an antibody, HC represents a heavy chain, LC represents a light chain, G represents a glycine unit, and z is an integer from 0 to 20.

In an aspect of the present invention, isoprenoid transferase can recognize substrates as well as isosubstrates. An isosubstrate refers to a substrate analog with modifications to the substrate. Isoprenoid transferases alkylate specific amino acid motifs (for example, CAAX motifs) at the C-terminus of proteins (see: Benjamin P. Duckworth et al, ChemBioChem 2007, 8, 98; Uyen T. T. Nguyen et al, ChemBioChem 2007, 8, 408; Guillermo R. Labadie et al, J. Org. Chem. 2007, 72(24), 9291; and James W. Wollack et al, ChemBioChem 2009, 10, 2934). Functionalized proteins can be prepared using isoprenoid transferases and isosubstrates through alkylation at the C-terminal cysteine(s).

For example, the cysteine residue of the C-terminal CAAX motif can be reacted with an isosubstrate using an isoprenoid transferase. In certain cases, AAX can then be eliminated by a protease. The obtained cysteine can then be methylated at the carboxy terminus by an enzyme (see Iran M. Bell, J. Med. Chem. 2004, 47(8), 1869).

Proteins of the present invention can be prepared using any molecular or cellular biology techniques well known in the art. For example, transient transfection may be used. A genetic sequence encoding a specific amino acid motif recognizable by isoprenoid transferase can be inserted into a known plasmid vector using standard PCR techniques to express a protein (fragment or analog thereof) having a specific amino acid motif at its C-terminus. A protein having one or more amino acid motifs recognizable by isoprenoid transferases can be thus expressed.

In an aspect of the present invention, when the protein is a monoclonal antibody, one or more light chains of the monoclonal antibody, one or more heavy chains of the monoclonal antibody, or both of these may contain an amino acid moiety having an amino acid motif recognizable by an isoprenoid transferase, and those skilled in the art can readily select a protein (for example, a target cell of a subject) that selectively binds a target of interest.

In an aspect of the present invention, a fragment of an antibody or antigen that specifically binds to a target of interest may be contained.

In an aspect of the present invention, the amino acid motif is CYYX, XXCC, XCXC, or CXX (where C is cysteine, Y is an aliphatic amino acid, and X is an amino acid that determines substrate specificity of isoprenoid transferase), and the amino acid motif is more preferably CYYX.

In a still another aspect of the present invention, there is provided a pharmaceutical composition for prevention or treatment of a proliferative disease, which contains a pyrrolobenzodiazepine dimer-linker-ligand conjugate or a pharmaceutically acceptable salt or solvate thereof.

In a still another aspect of the present invention, there is provided a pharmaceutical composition for prevention or treatment of a proliferative disease, which contains a pyrrolobenzodiazepine dimer-linker-ligand conjugate or a pharmaceutically acceptable salt or solvate thereof; and a pharmaceutically acceptable excipient.

In a still another aspect of the present invention, there is provided a pharmaceutical composition for prevention or treatment of a proliferative disease, which contains a pyrrolobenzodiazepine dimer-linker-ligand conjugate or a pharmaceutically acceptable salt or solvate thereof; one or more therapeutic co-agents; and a pharmaceutically acceptable excipient.

In an aspect of the present invention, the therapeutic co-agent may be, but is not limited to, an agent that exhibits a preventive, ameliorating, therapeutic effect on a proliferative disease, an agent capable of reducing the expression of side effects that occur when a therapeutic agent for a proliferative disease is administered, an agent exhibiting an immune enhancing effect, or the like. This means that any formulation can be combined and applied as long as, when applied in the form of a combined formulation together with pyrrolobenzodiazepines, it exhibits a therapeutically useful effect, further improves the stability of pyrrolobenzodiazepines, reduces side effects that may occur when pyrrolobenzodiazepines are administered, and/or exhibits the effect of maximizing the therapeutic effect through the enhancement of immunity.

In a still another aspect of the present invention, there is provided the use of a pyrrolobenzodiazepine dimer-linker-ligand conjugate or a pharmaceutically acceptable salt or solvate thereof for the treatment of a proliferative disease.

In a still another aspect of the present invention, there is provided a method of treating a proliferative disease, which includes administering a pyrrolobenzodiazepine dimer-linker-ligand conjugate or a pharmaceutically acceptable salt or solvate thereof to a subject. In other words, the present invention provides a method of treating a proliferative disease in a subject having the proliferative disease, which includes administering an effective amount of a pyrrolobenzodiazepine dimer-linker-ligand conjugate or a pharmaceutically acceptable salt or solvate thereof to the subject for the treatment of the proliferative disease.

In an aspect of the present invention, there is provided a method of treating cancer, which includes administering the pharmaceutical composition to a patient.

In an aspect of the present invention, the proliferative disease may be selected from the group consisting of neoplasm, tumor, cancer, leukemia, psoriasis, bone disease, fibroproliferative disorder, and atherosclerosis, but is not limited thereto. In an aspect of the present invention, the proliferative disease refers to a cell proliferation-related disease in which undesirably excessive or abnormal cells are unwantedly uncontrolled, such as neoplastic or hyperplastic growth, whether in vitro or in vivo. Examples of neoplasms and tumors include histiocytoma, glioma, astrocytoma, and osteoma.

In an aspect of the present invention, the cancer may be selected from the group consisting of lung cancer, small cell lung cancer, gastrointestinal cancer, colorectal cancer, bowel cancer, breast cancer, ovarian cancer, prostate cancer, testicular cancer, liver cancer, kidney cancer, bladder cancer, pancreatic cancer, brain cancer, sarcoma, osteosarcoma, Kaposi's sarcoma, and melanoma, but is not limited thereto, and the present invention can be applied to any carcinoma on which pyrrolobenzodiazepines can exhibit therapeutic effects.

### [Definition]

The following definitions apply in the present specification:
In the present specification, a "conjugate" refers to a cell binding agent that is covalently bound to one or more molecules of a cytotoxic compound. Here, the "cell binding agent" is a molecule having affinity for a biological target, and may be, for example, a ligand, a protein, or an antibody, specifically a monoclonal antibody, a protein or antibody fragment, a peptide, an oligonucleotide, or an oligosaccharide. The binding agent functions to lead the biologically active compound to the biological target. In an aspect of the present invention, the conjugate may be designed to target tumor cells through cell surface antigens. The antigen may be a cell surface antigen that is overexpressed or expressed in an abnormal cell type. Specifically, the target antigen may be expressed only on proliferating cells (for example, tumor cells). The target antigen may usually be selected based on different expression between proliferative and normal tissues. In the present invention, the ligand is bound to the linker.

In the present specification, an "antibody" is an immunoglobulin molecule capable of specifically binding to a target, for example, carbohydrates, polynucleotides, lipids, or polypeptides through at least one antigen recognition site located in the variable region of the immunoglobulin molecule. As used herein, the term "antibody" encompasses an intact polyclonal or monoclonal antibody as well as an arbitrary antigen-binding site (for example, "antigen-binding fragment") of an intact antibody having the ability to specifically bind to a predetermined antigen or a single chain thereof, fusion proteins including an antibody, and arbitrary other modified arrangements of an immunoglobulin molecule including an antigen recognition site, for example, but not limited to, Fab; Fab'; F(ab')2Fd fragment; Fv fragment; single domain antibody (dAb) fragment; isolated complementarity determining region (CDR); single chain (scFv) and single domain antibodies (for example shark and camelid antibodies), maxibodies, minibodies, intrabodies, diabodies, triabodies, tetrabodies, v-NARs, and bis-scFvs (see, for example, literature [Hollinger and Hudson, 2005, Nature Biotechnology 23(9): 1126-1136]).

The antibody includes antibodies of an arbitrary class, for example, IgG, IgA, or IgM (or subclasses thereof), and the antibody is not required to be an arbitrary particular class. Immunoglobulins may be assigned to different classes depending on the amino acid sequence of the constant region of the heavy chain of an antibody. There are five main classes of immunoglobulins: IgA, IgD, IgE, IgG, and IgM, and some of these may be further classified into subclasses (isotypes), for example, IgG1, IgG2, IgG3, IgG4, IgA1, and IgA2. The heavy chain (HC) constant domains corresponding to the different classes of immunoglobulins are called alpha, delta, epsilon, gamma, and mu, respectively. The subunit structures and three-dimensional coordinations of different classes of immunoglobulins are well known. The antibody of the present invention may be prepared using techniques well known in the art, for example, recombinant techniques, phage display techniques, synthetic techniques, or combinations of these techniques, or other techniques readily known in the art.

In the present specification, an "isolated antibody" refers to an antibody that is substantially free of other antibodies with different antigenic specificities, and may be substantially free of other cellular material and/or chemicals.

In the present specification, a "biological target" refers to an antigen located on the surface of a tumor, cancer cell, or extracellular matrix.

In the present specification, a "linker" refers to a compound that covalently binds a cytotoxic compound to a ligand. In an aspect of the present invention, the linker disclosed in PCT/US2016/063564 and PCT/US2016/063595 may be used as the linker.

In the present specification, a "subject" is intended to include human and non-human animals, particularly mammals. Examples of the subject include a human subject, and the subject is a concept including, for example, a human patient having a disorder described herein, more specifically cancer, or a normal subject. "Non-human animals" include all vertebrates, for example, non-mammals (for example, chickens, amphibians, and reptiles) and mammals, for example, non-human primates, livestock and/or agriculturally useful animals (for example, sheep, dogs, cats, cattle, and pigs) and rodents (for example, mice, rats, hamsters, and guinea pigs). In certain embodiments, the subject is a human patient.

In the present specification, "treatment" or "to treat" refers to both therapeutic treatment and prophylactic or preventive measures. Subjects in need of treatment include subjects already with a disease, and subjects prone to have a disease or subjects in which a disease is to be prevented. Hence, when used in reference to a disease or subject in need of treatment, the term includes arrest or slowing of disease progression, prevention of symptoms, reduction of disease and/or symptom severity, or reduction of disease duration as compared to an untreated subject, but is not limited thereto.

In the present specification, "administration" or "administering" refers to providing, bringing into contact with, and/or delivering a compound or compounds by an arbitrary suitable route to achieve a desired effect. Administration may include, but is not limited to, oral, sublingual, parenteral (for example, intravenous, subcutaneous, intradermal, intramuscular, intraarticular, intraarterial, intrasynovial, intrasternal, intrathecal, intralesional, or intracranial injection), transdermal, topical, buccal, rectal, vaginal, nasal, and ophthalmic administration and administration by inhalation and via implant.

In the present specification, "unsubstituted or substituted" means a parent group that may be unsubstituted or substituted, "substituted" means a parent group having one or more substituents, and a substituent means a chemical moiety to be covalently bound to a parent group or fused to a parent group.

In the present specification, "halo" refers to fluorine, chlorine, bromine, iodine, and the like.

In the present specification, "alkyl" is a monovalent moiety obtained by eliminating a hydrogen atom from a carbon atom of an aliphatic or alicyclic saturated or unsaturated (unsaturated, fully unsaturated) hydrocarbon compound. Examples of saturated alkyl include methyl, ethyl, propyl, butyl, pentyl, hexyl, heptyl and the like. Examples of saturated straight chain alkyl include methyl, ethyl, n-propyl, n-butyl, n-pentyl (amyl), n-hexyl, n-heptyl and the like. Examples of saturated branched chain alkyl include isopropyl, isobutyl, sec-butyl, tert-butyl, isopentyl, neopentyl and the like.

In the present specification, "alkoxy" means -OR [where R is an alkyl group], and examples thereof include methoxy, ethoxy, n-propoxy, isopropoxy, n-butoxy, sec-butoxy, isobutoxy, tert-butoxy and the like.

In the present specification, "aryl" means a monovalent moiety obtained by eliminating a hydrogen atom from an aromatic ring atom of an aromatic compound having a ring atom.

In the present specification, "alkenyl" is alkyl having one or more carbon-carbon double bonds, and examples of unsaturated alkenyl groups include ethenyl (vinyl, -CH=CH₂), 1-propenyl (-CH=CH-CH₃), 2-propenyl, isopropenyl, butenyl, pentenyl, hexenyl and the like.

In the present specification, "alkynyl" is an alkyl group having one or more carbon-carbon triple bonds, and examples of the unsaturated alkynyl group include ethynyl and 2-propynyl.

In the present specification, "carboxy" refers to - C(=O)OH.

In the present specification, "formyl" refers to - C(=O)H.

In the present specification, "aryl" relates to a monovalent moiety obtained by eliminating a hydrogen atom from an aromatic ring atom of an aromatic compound. For example, "C₅₋₇ aryl" is a moiety having 5 to 7 ring atoms and means a monovalent moiety obtained by eliminating a hydrogen atom from an aromatic ring atom of an aromatic compound, and "C₅₋₁₀ aryl" is a moiety having 5 to 10 ring atoms and means a monovalent moiety obtained by eliminating a hydrogen atom from an aromatic ring atom of an aromatic compound. Here, the prefixes (C₅₋₇, C₅₋₁₀, and the like) refer to the number of ring atoms or a range of the number of ring atoms, whether carbon atoms or heteroatoms. For example, "C₅₋₆ aryl" relates to an aryl group having 5 or 6 ring atoms. Here, the ring atoms may all be carbon atoms as in a "carboaryl group". Examples of carboaryl groups include, but are not limited to, those derived from benzene, naphthalene, azulene, anthracene, phenanthrene, naphthacene, and pyrene. Examples of aryl groups containing fused rings of which at least one is an aromatic ring include, but are not limited to, groups derived from indane, indene, isoindene, tetralin, acenaphthene, fluorene, phenalene, acephenanthrene, and aceanthrene. Alternatively, the ring atom may contain one or more heteroatoms as in a "heteroaryl group".

In the present specification, "heteroaryl" is aryl containing one or more heteroatoms, and examples thereof include pyridine, pyrimidine, benzothiophene, furyl, dioxalanyl, pyrrolyl, oxazolyl, pyridyl, pyridazinyl, pyrimidinyl and the like, more specifically, C₉ having two fused rings derived from benzofuran, isobenzofuran, indole, isoindole, indolizine, indoline, isoindoline, purine (adenine or guanine), benzimidazole, indazole, benzoxazole, benzisoxazole, benzodioxole, benzofuran, benzotriazole, benzothiofuran, benzothiazole, and benzothiadiazole, C₁₀ having two fused rings derived from chromene, isochromene, chroman, isochroman, benzodioxane, quinoline, isoquinoline, quinolizine, benzoxazine, benzodiazine, pyridopyrimidine, quinoxaline, quinazoline, cinnoline, phthalazine, naphthyridine, and pteridine, C₁₁ having two fused rings derived from benzodiazepines, C₁₃ having three fused rings derived from carbazole, dibenzofuran, dibenzothiophene, carboline, pyrimidine, and pyridoindole, and C₁₄ having three fused rings derived from acridine, xanthene, thioxanthene, oxanthrene, phenoxathiin, phenazine, phenoxazine, phenothiazine, thianthrene, phenanthridine, phenanthroline, and phenazine.

In the present specification, "cycloalkyl" is an alkyl group that is a cyclyl group and relates to a monovalent moiety obtained by eliminating a hydrogen atom from an alicyclic ring atom of a cyclic hydrocarbon compound. Examples of cycloalkyl groups include, but are not limited to, those derived from:
saturated monocyclic hydrocarbon compounds:
   cyclopropane, cyclobutane, cyclopentane, cyclohexane, cycloheptane, methylcyclopropane, dimethylcyclopropane, methylcyclobutane, dimethylcyclobutane, methylcyclopentane, dimethylcyclopentane, and methylcyclohexane;
unsaturated monocyclic hydrocarbon compounds:
   cyclopropene, cyclobutene, cyclopentene, cyclohexene, methylcyclopropene, dimethylcyclopropene, methylcyclobutene, dimethylcyclobutene, methylcyclopentene, dimethylcyclopentene and methylcyclohexene; and
saturated heterocyclic hydrocarbon compounds:
   norcarane, norpinane, and norbornane.

In the present specification, "heterocyclyl" relates to a monovalent moiety obtained by eliminating a hydrogen atom from a ring atom of a heterocyclic compound.

In the present specification, the prefixes (C₁₋₁₂, C₃₋₈, and the like) refer to the number of ring atoms or a range of the number of ring atoms, whether carbon atoms or heteroatoms. For example, the term "C₃₋₆ heterocyclyl" as used herein relates to a heterocyclyl group having 3 to 6 ring atoms.

Examples of monocyclic heterocyclyl groups include, but are not limited to, those derived from:
N₁: aziridine, azetidine, pyrrolidine, pyrroline, 2H- or 3H-pyrrole, piperidine, dihydropyridine, tetrahydropyridine, and azepine;
N₂: imidazolidine, pyrazolidine, imidazoline, pyrazoline, and piperazine;
O₁: oxirane, oxetane, oxolane, oxol, oxane, dihydropyran, pyran, and oxepin;
O₂: dioxolane, dioxane and dioxepane;
O₃: trioxane;
N₁O₁: tetrahydrooxazole, dihydrooxazole, tetrahydroisoxazole, dihydroisoxazole, morpholine, tetrahydrooxazine, dihydrooxazine, and oxazine;
S₁: thiirane, thietane, thiolane, thiane, and thiepan;
N₁S₁: thiazoline, thiazolidine, and thiomorpholine;
N₂O₁: oxadiazine;
O₁S₁: oxathiole and oxathiane; and
N₁O₁S₁: oxatiazine.

In the present specification, an acid addition salt formed by a pharmaceutically acceptable free acid may be used as the "pharmaceutically acceptable salt", and an organic acid or an inorganic acid may be used as the free acid.

The organic acid includes, but is not limited to, citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, glutamic acid, and aspartic acid. The inorganic acid includes, but is not limited to, hydrochloric acid, hydrobromic acid, sulfuric acid, and phosphoric acid.

For example, when a compound is an anion or has a functional group that may be an anion (for example, -COOH may be -COO⁻), a salt may be formed using an appropriate cation. Examples of appropriate inorganic cations include, but are not limited to, alkali metal ions, for example, Na⁺ and K⁺, alkaline earth metal cations, for example, Ca²⁺ and Mg²⁺, and other cations, for example, Al³⁺. Examples of appropriate organic cations include, but are not limited to, ammonium ions (namely, NH₄⁺) and substituted ammonium ions (for example, NH₃R⁺, NH₂R₂⁺, NHR₃⁺, NR₄⁺).

Examples of some appropriate substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine and tromethamine as well as amino acids, for example, lysine and arginine. An example of a common quaternary ammonium ion is N(CH₃)₄⁺.

When a compound is a cation or has a functional group that may be a cation (for example, -NH₂ may be - NH₃⁺), a salt may be formed using an appropriate anion. Examples of appropriate inorganic anions include, but are not limited to, those derived from the following inorganic acids: hydrochloric acid, hydrobromic acid, hydroiodic acid, sulfuric acid, sulfurous acid, nitric acid, nitrous acid, phosphoric acid, phosphorous acid and the like.

Examples of appropriate organic anions include, but are not limited to, those derived from the following organic acids: 2-acetyloxybenzoic acid, acetic acid, ascorbic acid, aspartic acid, benzoic acid, camphorsulfonic acid, cinnamic acid, citric acid, edetic acid, ethanedisulfonic acid, ethanesulfonic acid, fumaric acid, glucheptonic acid, gluconic acid, glutamic acid, glycolic acid, hydroxymaleic acid, hydroxynaphthalene carboxylic acid, isethionic acid, lactic acid, lactobionic acid, lauric acid, maleic acid, malic acid, methanesulfonic acid, mucic acid, oleic acid, oxalic acid, palmitic acid, pamoic acid, pantothenic acid, phenylacetic acid, phenylsulfonic acid, propionic acid, pyruvic acid, salicylic acid, stearic acid, succinic acid, sulfanilic acid, tartaric acid, toluenesulfonic acid, valeric acid and the like. Examples of appropriate polymeric organic anions include, but are not limited to, those derived from the following polymeric acids: tannic acid, carboxymethyl cellulose, and the like.

In the present specification, a "solvate" refers to a molecular complex between the compound according to the present invention and a solvent molecule. Examples of the solvate include, but are not limited to, the compound according to the present invention bound with water, isopropanol, ethanol, methanol, dimethyl sulfoxide, ethyl acetate, acetic acid, ethanolamine, or any mixture thereof.

It may be convenient or desirable to prepare, purify, and/or handle an equivalent solvate of an active compound. The term "solvate" is used herein in its conventional sense to refer to a complex of a solute (for example, an active compound, a salt of an active compound) and a solvent. When the solvent is water, the solvate may conveniently be referred to as a hydrate, for example, a monohydrate, dihydrate, trihydrate or the like.

The pharmaceutical composition of the present invention may contain a pharmaceutically acceptable carrier. The pharmaceutically acceptable carrier may typically include macromolecules that are slowly metabolized, for example, proteins, polysaccharides, polylactic acid, polyglycolic acid, polymeric amino acids, amino acid copolymers, lipid aggregates, and the like. Such pharmaceutically acceptable carriers may be appropriately selected and used by those skilled in the art.

The composition containing a pharmaceutically acceptable carrier may be in various oral or parenteral formulations. In the case of preparations, the preparations are prepared using diluents or excipients such as fillers, extenders, binders, wetting agents, disintegrants, and surfactants that are usually used.

Solid preparations for oral administration include tablets, pills, powders, granules, capsules and the like. Such solid preparations are prepared by mixing one or more compounds with at least one or more excipients, for example, starch, calcium carbonate, sucrose or lactose, gelatin, and the like. In addition to simple excipients, lubricants such as magnesium stearate and talc may also be used.

Liquid preparations for oral administration include suspensions, oral solutions, emulsions, syrups and the like. In addition to water and liquid paraffin that are commonly used simple diluents, various excipients, for example, wetting agents, sweetening agents, fragrances, and preservatives may be contained.

Preparations for parenteral administration include sterile aqueous solutions, non-aqueous solutions, suspensions, emulsions, freeze-dried preparations, and suppositories. Non-aqueous solvents and propylene glycol, polyethylene glycol, vegetable oils such as olive oil, and injectable esters such as ethyl oleate as suspensions may be used. As the base of suppositories, witepsol, macrogol, tween 61, cacao butter, laurin, glycerogelatin and the like may be used.

The pharmaceutical composition may have any one formulation selected from the group consisting of injections, tablets, pills, powders, granules, capsules, suspensions, oral solutions, emulsions, syrups, sterile aqueous solutions, non-aqueous solutions, suspensions, emulsions, freeze-dried formulations, and suppositories.

For intravenous, dermal or subcutaneous injection and the like, the active ingredient may be in the form of an acceptable aqueous solution for parenteral administration, which is pyrogen-free and has appropriate pH, isotonicity, and stability. Those skilled in the art can prepare appropriate solutions using isotonic vehicles, for example, aqueous sodium chloride solution, Ringer's solution, and lactate Ringer's solution, and preservatives, stabilizers, buffers, antioxidants or other additives may be contained if necessary. Solid forms suitable for injection may also be prepared as emulsions or in the form of polypeptides encapsulated in liposomes.

As used herein, the phrase "effective amount" or "therapeutically effective amount" refers to the amount necessary (with respect to the dosage and duration and means of administration) to achieve the desired therapeutic result. The effective amount is at least the minimum amount of an active agent necessary to confer a therapeutic benefit to a subject, and is less than a toxic amount. For example, the dosage may range from about 100 ng/kg to about 100 mg/kg, more typically from about 1 µg/kg to about 10 mg/kg per patient. When the active compound is a salt, an ester, an amide, a prodrug or the like, the dosage is calculated based on the parent compound, and the actual weight used thus increases proportionally. The pyrrolobenzodiazepine compound according to the present invention may be formulated to contain the active ingredient at 0.1 mg to 3000 mg, 1 mg to 2000 mg, or 10 mg to 1000 mg per dosage form, but is not limited thereto. The active ingredient may be administered to obtain a peak plasma concentration of the active compound of about 0.05 µM to 100 µM, 1 µM to 50 µM, or 5 µM to 30 µM. For example, the active ingredient may be arbitrarily administered by intravenous injection of a 0.1 w/v% to 5 w/v% solution of the active ingredient in physiological saline.

The concentration of active compound in a pharmaceutical composition may be determined by the absorption, inactivation, and excretion rates of the drug and other factors known to those skilled in the art. The dosage may vary depending on the severity of the condition/disease. The dosage and dosing regimen for a specific patient may be adjusted according to the professional judgment of the administration supervisor, taking into account the severity of the patient's condition/disease, necessity, age, and responsiveness to drugs, and the concentration ranges presented herein are exemplary only and are not intended to limit the embodiments of the claimed compositions thereto. The active ingredient may be administered one time or may be administered several times in smaller dosages.

The pyrrolobenzodiazepine dimer compound, pyrrolobenzodiazepine dimer compound-linker compound, or pyrrolobenzodiazepine dimer compound-linker-ligand conjugate compound according to the present invention can be used to treat proliferative diseases, in particular cancer diseases. The term "proliferative disease" refers to unwanted or uncontrolled cellular proliferation of undesirable excessive or abnormal cells, such as neoplastic or hyperplastic growth, in vitro or in vivo. The proliferative disease includes, for example, neoplasms, tumors, cancer, leukemia, psoriasis, bone diseases, fibroproliferative disorders, and atherosclerosis, may include benign, premalignant, or malignant cell proliferation, but is not limited thereto. The cancer may be, but is not limited to, lung cancer, small cell lung cancer, gastrointestinal cancer, colorectal cancer, bowel cancer, breast cancer, ovarian cancer, prostate cancer, testicular cancer, liver cancer, kidney cancer, bladder cancer, pancreatic cancer, brain cancer, sarcoma, osteosarcoma, Kaposi's sarcoma, or melanoma.

Unless defined otherwise herein, scientific and technical terms used in connection with the present invention have the meanings commonly understood by a person of ordinary skill in the art.

The pyrrolobenzodiazepine dimer-linker compounds and pyrrolobenzodiazepine dimer-linker-ligand conjugates according to the present invention can be prepared by the techniques provided herein using the knowledge of those skilled in the art.

For example, linkers are described in PCT/US2016/063564 and PCT/US2016/063595 of which the entire contents are incorporated herein by reference, are cited herein although not described herein, or can be prepared according to known references by those skilled in the art.

In an aspect of the present invention, the pyrrolobenzodiazepine derivatives and precursors thereof, pyrrolobenzodiazepine dimer-linker compounds, and pyrrolobenzodiazepine-linker-ligand conjugates according to the present invention can be synthesized according to the following procedures.

Synthetic pathway of pyrrolobenzodiazepine precursor-linker and pyrrolobenzodiazepine precursor-linker-ligand conjugate

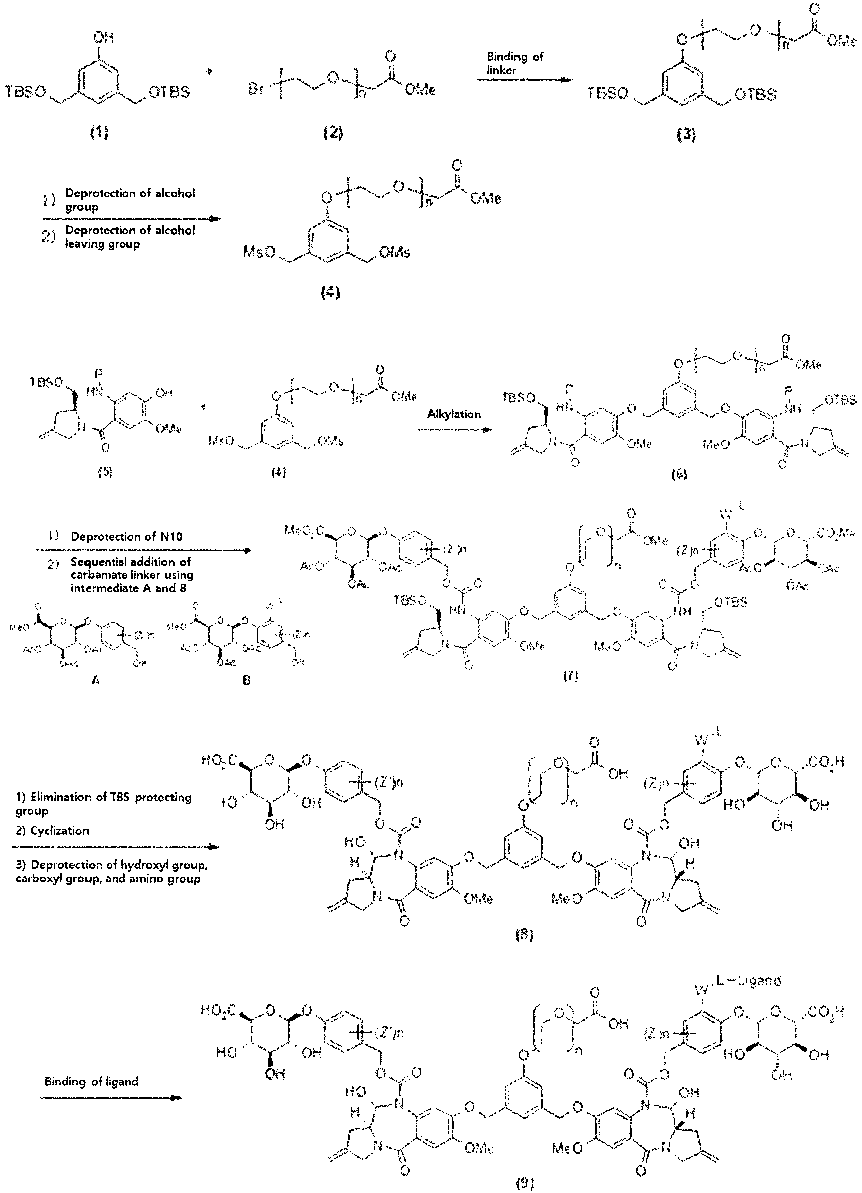

Synthetic pathway of pyrrolobenzodiazepine derivative and precursor thereof

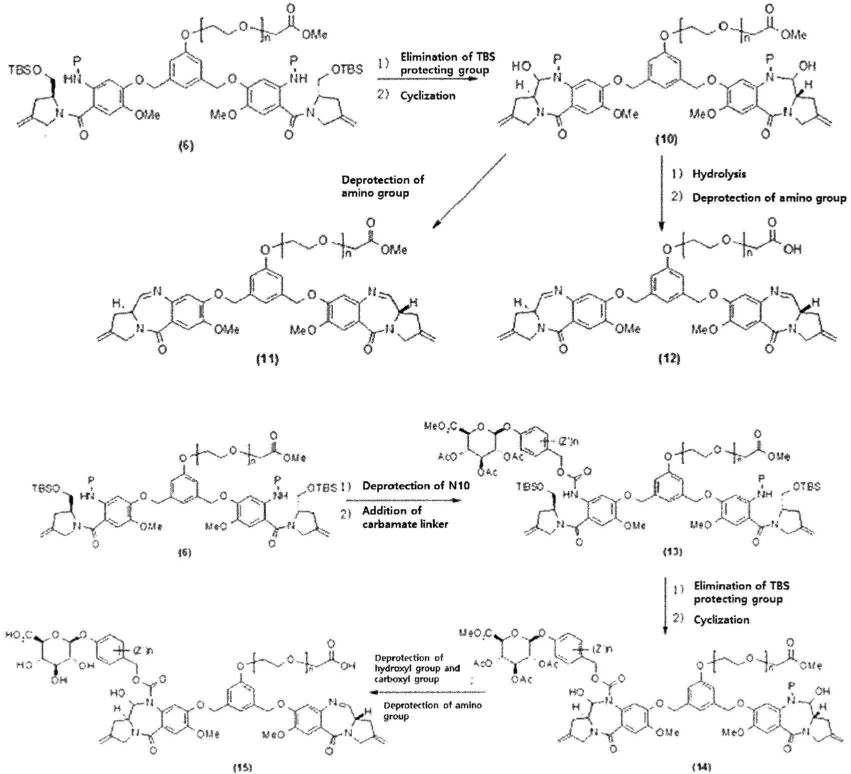

### [Examples]

Hereinafter, the present invention will be described in more detail with reference to Examples. However, the following Examples are provided to aid the understanding of the present invention, and are not intended to limit the scope of the present invention.

### <Example 1> Preparation of Compound 4

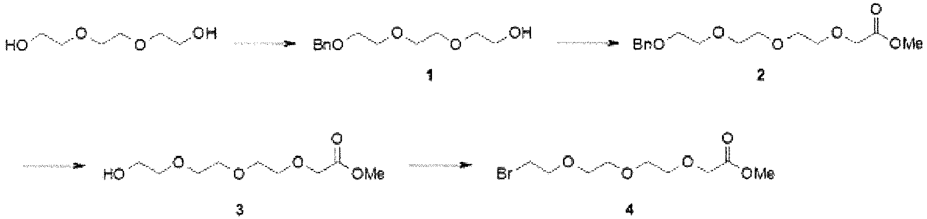

### Preparation of Compound 1

Triethylene glycol (7.0 g, 46.6 mmol) was dissolved in dichloromethane (50 mL), and then silver(I) oxide (16 g, 69.9 mmol) and benzyl bromide (6.1 mL, 51.3 mmol) were sequentially added thereto at 0°C in a nitrogen atmosphere. The reaction solution was heated to room temperature, stirred for 20 hours, and then filtered through Celite. The filtered solution was concentrated and then purified by column chromatography to obtain Compound 1 (8.2 g, 73%).

¹H-NMR (400 MHz, CDCl₃) δ 7.24-7.36 (m, 5H) , 4.57 (s, 2H), 3.76-3.56 (m, 12H), 2.60 (br s, 1H).

### Preparation of Compound 2

Compound 1 (8.0 g, 33.3 mmol) was diluted with toluene (40 mL), and then potassium t-butoxide (1 M tetrahydrofuran solution, 99 mL, 99 mmol) and methyl bromoacetate (15 mL, 166 mmol) were sequentially added thereto at 0°C in a nitrogen atmosphere. The reaction solution was heated to room temperature and stirred for 18 hours. Salt water (200 mL) was added to the reaction solution, and then extraction with dichloromethane (3 × 200 mL) was performed. The collected organic layer was washed with salt water (200 mL) and then dried over anhydrous sodium sulfate. Filtration, concentration, and purification by column chromatography were performed to obtain Compound 2 (5.9 g, 56%).

¹H-NMR (400 MHz, CDCl₃) δ 7.25-7.36 (m, 5H), 4.57 (s, 2H), 4.14 (s, 2H), 3.76-3.62 (m, 12H).

### Preparation of Compound 3

Compound 2 (9.0 g, 28.8 mmol) was dissolved in methanol (200 mL), and then palladium/charcoal (10% w/w, 900 mg) was added thereto. The reaction solution was stirred at room temperature in a hydrogen atmosphere for 2 hours. The reaction solution was filtered through Celite and then concentrated to obtain Compound 3 (5.18 g, 81%) .

¹H-NMR (400 MHz, CDCl₃) δ 4.18 (s, 2H), 3.78-3.65 (m, 12H), 3.64-3.60 (m, 1H).

### Preparation of Compound 4

Compound 3 (5.0 g, 22.5 mmol) was dissolved in tetrahydrofuran (50 mL), and then tetrabromomethane (11 g, 33.7 mmol) and triphenylphosphine (7.3 g, 27.0 mmol) were sequentially added thereto at 0°C in a nitrogen atmosphere. The reaction solution was stirred for 3 hours, then diluted with ethyl acetate (100 mL), and washed with distilled water (50 mL). The collected organic layer was dried over anhydrous sodium sulfate, then filtered, concentrated, and purified by column chromatography to obtain Compound 4 (3.4 g, 53%).

### <Example 2> Preparation of Compound 10

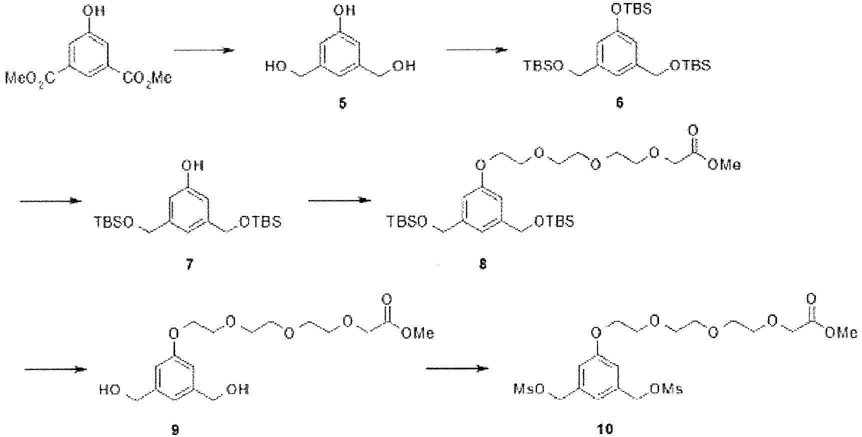

### Preparation of Compound 5

Dimethyl-5-hydroxyisophthalate (10 g, 47.6 mmol) was dissolved in tetrahydrofuran (250 mL), and then lithium aluminum hydride (1 M tetrahydrofuran solution, 95.2 mL, 95.2 mmol) was gradually added thereto at -30°C in a nitrogen atmosphere. The reaction solution was stirred for 5 hours while being gradually heated to room temperature, and then cooled to 0°C, and distilled water (3.6 mL), sodium hydroxide aqueous solution (15% w/w, 3.6 mL), and distilled water (10.8 mL) were gradually added to the reaction solution. The produced solid was filtered, and the residual organic layer was concentrated and purified by column chromatography to obtain Compound 5 (4.4 g, 60%).

¹H-NMR (400 MHz, CDCl₃) δ 9.18 (s, 1H), 6.65 (s, 1H), 6.57 (s, 2H), 5.05 (br s, 2H), 4.37 (s, 4H).

### Preparation of Compound 6

Compound 5 (6.8 g, 44.1 mmol) was dissolved in *N,N-*dimethylformamide (40 mL), and then imidazole (18.0 g, 265 mmol) and t-butyldimethylsilyl chloride (21.2 g, 141 mmol) were added thereto. The reaction solution was stirred at room temperature for 16 hours, and then ethyl acetate (200 mL) was added thereto. The organic layer was washed with distilled water (200 mL) and salt water (200 mL) in this order, and then dried over anhydrous sodium sulfate. Filtration, concentration, and purification by column chromatography were performed to obtain Compound 6 (20.6 g, 94%).

¹H-NMR (400 MHz, CDCl₃) δ 6.84 (s, 1H), 6.69 (s, 2H), 4.66 (s, 4H), 0.97 (s, 9H), 0.93 (s, 18H), 0.17 (s, 6H), 0.08 (s, 12H).

### Preparation of Compound 7

Compound 6 (11.0 g, 22.1 mmol) was dissolved in *N,N-*dimethylformamide/distilled water (150 mL/10 mL), then sodium acetate (2.0 g, 24.3 mmol) was added thereto, and the reaction solution was stirred at 70°C in a nitrogen atmosphere. After 3 hours, the reaction temperature was lowered to room temperature, and ethyl acetate (300 mL) was added thereto. The organic layer was washed with distilled water (200 mL) and slat water (200 mL) in this order, and then dried over anhydrous sodium sulfate. Filtration, concentration, and purification by column chromatography were performed to obtain Compound 7 (7.6 g, 89%).

¹H-NMR (400 MHz, CDCl₃) δ 6.81 (s, 1H), 6.69 (s, 2H), 5.09 (s, 1H), 4.68 (s, 4H), 0.93 (s, 18H), 0.10 (s, 12H).

### Preparation of Compound 8

Compound 7 (2.7 g, 7.3 mmol) and Compound 4 (2.5 g, 8.8 mmol) were dissolved in N,N-dimethylformamide (30 mL), then cesium carbonate (3.4 g, 11.0 mmol) was added thereto, and the mixture was stirred at room temperature for 16 hours in a nitrogen atmosphere. The reaction solution was diluted with distilled water (100 mL) and then subjected to extraction with ethyl acetate (2 ×100 mL). The extracted organic layer was washed with salt water (100 mL) and dried over anhydrous sodium sulfate. Filtration, concentration under reduced pressure, and purification by column chromatography were performed to obtain Compound 8 (2.3 g, 49%).

¹H-NMR (400 MHz, CDCl₃) δ 6.85 (s, 1H), 6.76 (s, 2H), 4.68 (s, 4H), 4.16 (s, 2H), 4.14-4.08 (m, 2H), 3.88-3.82 (m, 2H), 3.76-3.64 (m, 11H), 0.93 (s, 18H), 0.09 (s, 12H).

### Preparation of Compound 9

Compound 8 (2.25 g, 3.83 mmol) was dissolved in tetrahydrofuran (50 mL), and then tetrabutylammonium fluoride (1 M tetrahydrofuran solution, 8.0 mL, 8.0 mmol) was added thereto at 0°C in a nitrogen atmosphere. The mixture was stirred for 1 hour, then diluted with saturated aqueous ammonium chloride solution (100 mL), and subjected to extraction with ethyl acetate (2 ×100 mL). The collected organic layer was washed with salt water (100 mL) and dried over anhydrous sodium sulfate. Filtration, concentration under reduced pressure, and purification by column chromatography were performed to obtain Compound 9 (1.4 g, 100%).

¹H-NMR (400 MHz, CDCl₃) δ 6.98 (s, 1H), 6.83 (s, 2H), 4.62 (s, 4H), 4.16 (s, 2H), 4.12 (t, J = 4.8 Hz, 2H), 3.83 (t, J = 4.4 Hz, 2H), 3.74 (s, 3H), 3.74-3.64 (m, 13H).

### Preparation of Compound 10

Compound 9 (1.0 g, 2.79 mmol) was dissolved in dichloromethane (20 mL), and then triethylamine (1.2 mL, 8.37 mmol) and methanesulfonyl chloride (0.5 mL, 6.14 mmol) were added thereto at 0°C in a nitrogen atmosphere. The mixture was stirred for 1 hour, then diluted with distilled water (30 mL), and subjected to extraction with dichloromethane (2 × 50 mL). The organic layer was dried over anhydrous sodium sulfate, filtered, and then concentrated to obtain Compound 10 (crude 1.54 g, 100%).

¹H-NMR (400 MHz, CDCl₃) δ 7.01 (s, 1H), 6.97 (s, 2H), 5.18 (s, 4H), 4.16-4.12 (m, 4H), 3.83 (t, J = 4.8 Hz, 2H), 3.74-3.64 (m, 11H), 2.97 (s, 6H).

### <Example 3> Preparation of Compound 16

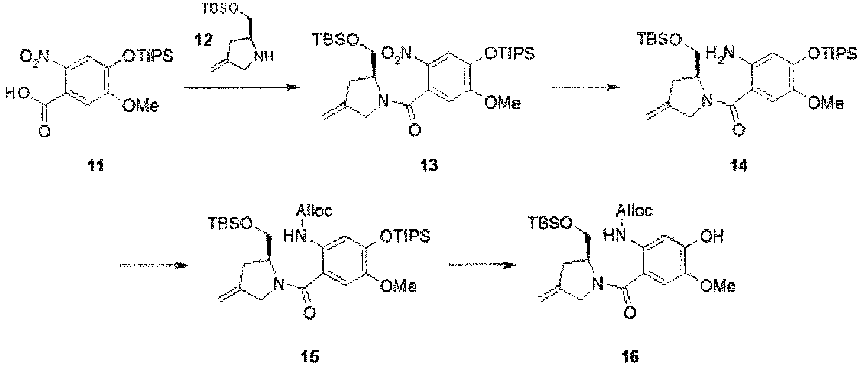

### Preparation of Compound 13

Compound 11 (48.7 g, 132 mmol) and Compound 12 (25 g, 110 mmol, Compound 12 was prepared by the method described in Korean Patent Publication No. 10-2018-0110645) were dissolved in dichloromethane (450 mL), and then 1-hydroxybenzotriazole (19.3 g, 143 mmol), N-(3-dimethylaminopropyl)-N'-ethylcarbodiimide hydrochloride (29.5 g, 154 mmol), and triethylamine (27 mL, 194 mmol) were sequentially added thereto at 0°C in a nitrogen atmosphere. The reaction solution was stirred at room temperature for 12 hours, then saturated aqueous ammonium chloride solution (400 mL) was added to the reaction solution, and extraction with dichloromethane (2 × 300 mL) was performed. The collected organic layer was washed with salt water (400 mL) and then dried over anhydrous sodium sulfate. Filtration, concentration, and purification by column chromatography were performed to obtain Compound 13 (57.1 g, 89%).

¹H-NMR (400 MHz, CDCl₃) (rotamers) δ 7.69 (s, 1H), 6.73 (s, 1H), 4.98 (s, 1H), 4.83 (s, 1H), 4.58 (bs, 1H), 3.89 (s, 4H), 3.75 (bs, 1H), 3.71 (s, 1H), 3.31-3.27 (m, 1H), 2.82-2.53 (m, 2H), 1.30-1.26 (m, 3H), 1.10 (d, J = 7.6 Hz, 18H), 0.91 (s, 9H), 0.09 (s, 6H).

### Preparation of Compound 14

Compound 13 (29.5 g, 51.0 mmol) was dissolved in ethanol (220 mL), and then zinc dust (Zinc dust, 66.6 g, 1.01 mol) and formic acid (5% in EtOH, 500 mL) were added thereto. The reaction solution was stirred at room temperature for 30 minutes, filtered through Celite, and ethyl acetate (1 L) was added to the filtered solution. The organic layer was washed with distilled water (500 mL), saturated aqueous sodium hydrogen carbonate solution (500 mL), and salt water (500 mL) in this order, and then dried over anhydrous sodium sulfate. Filtration, concentration, and purification by column chromatography were performed to obtain Compound 14 (27.9 g, 99%).

¹H-NMR (400 MHz, CDCl₃) (rotamers) δ 6.71 (s, 1H), 6.25 (s, 1H), 4.96 (s, 1H), 4.89 (s, 1H), 4.53 (bs, 1H), 4.21-4.09 (m, 4H), 3.71 (s, 3H), 3.62 (bs, 1H) 2.73-2.63 (m, 2H), 1.29-1.21 (m, 3H), 1.10 (d, J = 7.2 Hz, 18H), 0.87 (s, 9H), 0.02 (s, 6H).

### Preparation of Compound 15

Compound 14 (27.9 g, 50.9 mmol) was dissolved in dichloromethane (300 mL), and then pyridine (9.0 mL, 111.3 mmol) and allyl chloroformate (5.9 mL, 55.5 mmol) were added thereto at -78°C in a nitrogen atmosphere. The reaction solution was stirred for 1 hour, then the reaction temperature was raised to room temperature, and concentration and purification by column chromatography were performed to obtain Compound 15 (31.8 g, 98%).

¹H-NMR (400 MHz, CDCl₃) (rotamers) δ 8.67 (bs, 1H), 7.75 (s, 1H), 6.78 (s, 1H), 5.97-5.89 (m, 1H), 5.32 (d, J = 17.2 Hz, 1H), 5.21 (d, J = 10.4 Hz, 1H), 4.98 (s, 1H), 4.90 (bs, 1H), 4.66-4.61 (m, 3H), 4.19-4.15 (m, 1H), 4.01 (bs, 1H), 3.86 (bs, 1H), 3.76 (s, 3H), 3.65 (bs, 1H) 2.68 (s, 2H), 1.33-1.24 (m, 3H), 1.10 (d, J = 6.8 Hz, 18H), 0.87 (s, 9H), 0.03 (s, 6H).

### Preparation of Compound 16

Compound 15 (31.8 g, 50.3 mmol) was dissolved in N,N-dimethylformamide/distilled water (300 mL/6 mL), then sodium acetate (5.0 g, 60.0 mmol) was added thereto, and the reaction solution was stirred at 70°C in a nitrogen atmosphere. After 3 hours, the reaction temperature was lowered to room temperature, and ethyl acetate (300 mL) was added to the reaction solution. The organic layer was washed with distilled water (200 mL) and salt water (200 mL) in this order, and then dried over anhydrous sodium sulfate. Filtration, concentration, and purification by column chromatography were performed to obtain Compound 16 (18.6 g, 77%).

¹H-NMR (400 MHz, CDCl₃) (rotamers) δ 8.71 (bs, 1H), 7.75 (s, 1H), 6.78 (s, 1H), 6.14 (s, 1H), 5.94-5.90 (m, 1H), 5.32 (d, J = 17.2 Hz, 1H), 5.21 (d, J = 10.4 Hz, 1H), 4.97 (s, 1H), 4.90 (bs, 1H), 4.64-4.58 (m, 3H), 4.18-4.15 (m, 1H), 4.01 (bs, 1H), 3.83 (s, 4H), 3.65 (bs, 1H) 2.68 (s, 2H), 0.87 (s, 9H), 0.02 (s, 6H).

### <Example 4> Preparation of Compound 19

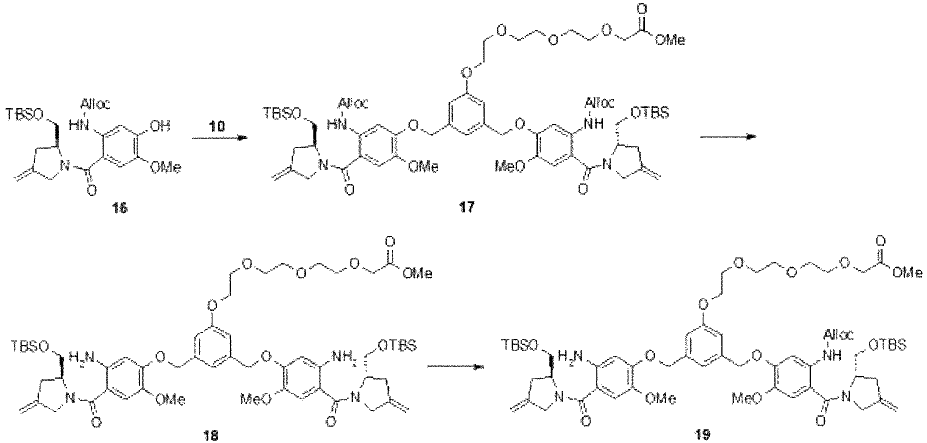

### Preparation of Compound 17

Compound 16 (3.2 g, 6.70 mmol) and Compound 10 (1.4 g, 2.79 mmol) were dissolved in *N,N*-dimethylformamide (10 mL), and then potassium carbonate (1.9 g, 13.6 mmol) was added thereto at 0°C, and the mixture was stirred at room temperature for 20 hours in a nitrogen atmosphere. Distilled water (30 mL) was added to the reaction solution, and then extraction with ethyl acetate (2 × 50 mL) was performed. The collected organic layer was washed with salt water (50 mL) and dried over anhydrous sodium sulfate. Filtration, concentration under reduced pressure, and purification by column chromatography were performed to obtain Compound 17 (2.72 g, 76%).

¹H-NMR (400 MHz, CDCl₃) δ 7.88 (br s, 2H), 7.24 (s, 2H), 7.13 (s, 1H), 6.99 (s, 2H), 6.81 (s, 2H), 5.97-5.87 (m, 2H), 5.32 (dd, *J* = 17.2, 1.2 Hz, 1H), 5.21 (dd, *J* = 10.0, 1.2 Hz, 1H), 5.12 (s, 4H), 4.96 (br, 2H), 4.89 (br, 2H), 4.64-4.54 (m, 5H), 4.21-4.12 (m, 7H), 3.84 (t, *J* = 4.4 Hz, 4H), 3.81 (s, 6H), 3.73-3.63 (m, 13H), 2.67 (br, 4H) 0.86 (s, 18H), 0.01 (s, 6H).

### Preparation of Compound 18

Compound 17 (2.7 g, 2.12 mmol) was dissolved in dichloromethane (20 mL), then pyrrolidine (0.43 mL, 5.29 mmol) and tetrakis(triphenylphosphine)palladium(0) (98 mg, 0.085 mmol) were added thereto, and the mixture was stirred at room temperature in a nitrogen atmosphere for 6 hours. Distilled water (30 mL) was added to the reaction solution, and then extraction with dichloromethane (2 × 50 mL) was performed. The collected organic layer was washed with salt water (50 mL) and dried over anhydrous sodium sulfate. Filtration, concentration under reduced pressure, and purification by column chromatography were performed to obtain Compound 18 (1.7 g, 72%).

EI-MS m/z: [M+H]⁺ 1108.2, 1/2[M+H]⁺ 554.7.

### Preparation of Compound 19

Compound 18 (1.6 g, 1.44 mmol) was dissolved in dichloromethane (100 mL), then pyridine (0.3 mL, 3.03 mmol) and allyl chloroformate (0.13 mL, 1.30 mmol) were added thereto at -78°C in a nitrogen atmosphere, and the reaction solution was stirred for 1 hour. Distilled water (100 mL) was added to the reaction solution, and then extraction with dichloromethane (2 × 100 mL) was performed. The collected organic layer was washed with salt water (100 mL) and dried over anhydrous sodium sulfate. Filtration, concentration under reduced pressure, and purification by column chromatography were performed to obtain Compound 19 (985 mg, 57%).

¹H-NMR (400 MHz, CDCl₃) δ 7.91 (br s, 1H), 7.26 (s, 1H), 7.09 (s, 1H), 6.97 (s, 1H), 6.95 (s, 1H), 6.83 (s, 1H), 6.74 (s, 1H), 6.23 (s, 1H), 5.99-5.88 (m, 1H), 5.33 (dd, *J* = 17.2, 1.6 Hz, 1H), 5.23 (dd, *J* = 10.4, 1.2 Hz, 1H), 5.14 (s, 2H), 5.10 (s, 2H), 4.97 (br, 2H), 4.90 (br, 2H), 4.65-4.58 (m, 3H), 4.22-4.10 (m, 8H), 3.87-3.82 (m, 4H), 3.82 (s, 3H), 3.75 (s, 3H), 3.74-3.64 (m, 16H), 2.68 (br, 4H) 0.87 (s, 18H), 0.02 (s, 6H).

### <Example 5> Preparation of Compound 24

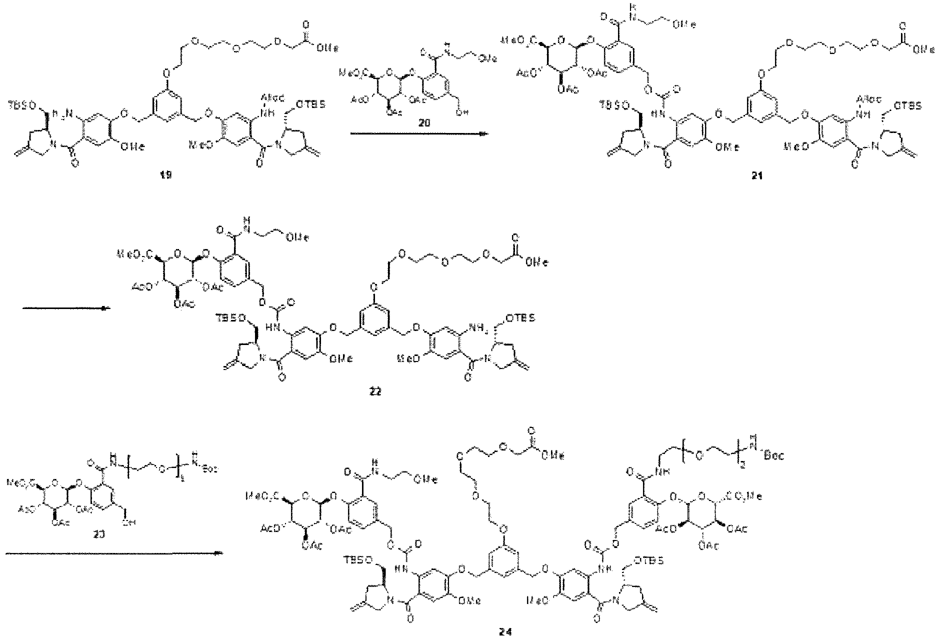

### Preparation of Compound 21

Compound 19 (980 mg, 0.82 mmol) was dissolved in dry tetrahydrofuran (40 mL), then triphosgene (98 mg, 0.33 mmol) and triethylamine (0.18 mL, 1.32 mmol) were added thereto at -10°C, and the mixture was stirred in a nitrogen atmosphere for 1 hour. Compound 20 (490 mg, 0.90 mmol, Compound 20 was prepared by the method described in Korean Patent Publication No. 10-2018-0110645) was dissolved in dry tetrahydrofuran (5 mL), triethylamine (0.18 mL, 1.32 mmol) was added thereto, and then this solution was gradually added to the reaction solution. After 1 hour, the reaction solution was heated to reflux and stirred for 12 hours. The reaction solution was diluted with ethyl acetate (30 mL), then washed with salt water (20 mL), and dried over anhydrous sodium sulfate. Filtration, concentration under reduced pressure, and purification by column chromatography were performed to obtain Compound 21 (580 mg, 40%).

EI-MS m/z: [M+H]⁺ 1760.7, 1/2[M+H]⁺ 880.6.

### Preparation of Compound 22

Compound 21 (580 mg, 0.33 mmol) was dissolved in dichloromethane (10 mL), then pyrrolidine (0.035 mL, 0.43 mmol) and tetrakis(triphenylphosphine)palladium(0) (9 mg, 0.0082 mmol) were added thereto, and the mixture was stirred at room temperature in a nitrogen atmosphere for 2 hours. Distilled water (20 mL) was added to the reaction solution, and then extraction with dichloromethane (2 × 20 mL) was performed. The collected organic layer was washed with salt water (20 mL) and dried over anhydrous sodium sulfate. Filtration, concentration under reduced pressure, and purification by column chromatography were performed to obtain Compound 22 (455 mg, 82%).

EI-MS m/z: [M+H]⁺ 1676.7, 1/2[M+H]⁺ 838.6.

### Preparation of Compound 24

Compound 22 (450 mg, 0.27 mmol) was dissolved in dry tetrahydrofuran (15 mL), then triphosgene (32 mg, 0.11 mmol) and triethylamine (0.12 mL, 0.81 mmol) were added thereto at -10°C, and the mixture was stirred in a nitrogen atmosphere for 1 hour. Compound 23 (216 mg, 0.30 mmol, Compound 23 was prepared by the method described in Korean Patent Publication No. 10-2018-0110645) was dissolved in dry tetrahydrofuran (3 mL), triethylamine (0.12 mL, 0.81 mmol) was added thereto, and then this solution was gradually added to the reaction solution. After 1 hour, the reaction solution was heated to reflux and stirred for 16 hours. The reaction solution was diluted with ethyl acetate (30 mL), washed with salt water (20 mL), and dried over anhydrous sodium sulfate. Filtration, concentration under reduced pressure, and purification by column chromatography were performed to obtain Compound 24 (293 mg, 45%).

EI-MS m/z: [M+H]⁺ 2243.8, 1/2[M+H]⁺ 1122.5.

### <Example 6> Preparation of Compound 28

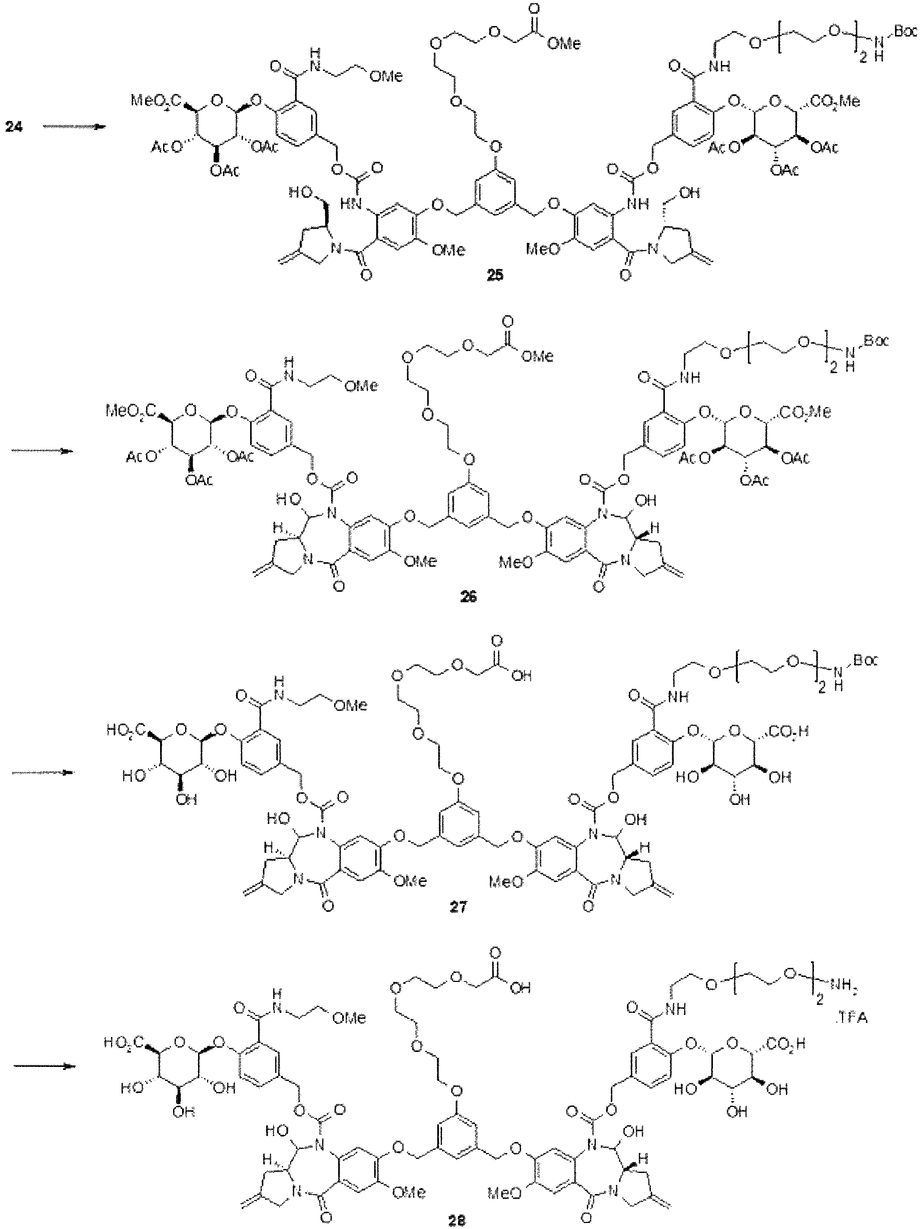

### Preparation of Compound 25

Compound 24 (293 mg, 0.12 mmol) was dissolved in tetrahydrofuran/distilled water (3 mL/3 mL), and acetic acid (8 mL) was added thereto, and then the mixture was stirred at room temperature in a nitrogen atmosphere for 16 hours. The reaction solution was concentrated under reduced pressure and purified by column chromatography to obtain Compound 25 (180 mg, 68%).

EI-MS m/z: [M+H]⁺ 2204.4, 1/2[M+H]⁺ 1102.8.

### Preparation of Compound 26

Compound 25 (180 mg, 0.082 mmol) was dissolved in dichloromethane (5 mL), then Dess-Martin periodinane (76 mg, 0.18 mmol) was added thereto, and the mixture was stirred at room temperature in a nitrogen atmosphere for 24 hours. The reaction solution was concentrated under reduced pressure and then purified by column chromatography to obtain Compound 26 (120 mg, 66%).

EI-MS m/z: [M+H]⁺ 2199.7, [M+Na]⁺ 2222.5.

### Preparation of Compound 27

Compound 26 (120 mg, 0.055 mmol) was dissolved in methanol/tetrahydrofuran (1 mL/3 mL), and then a solution prepared by dissolving lithium hydroxide (20 mg, 0.55 mmol) in distilled water (1 mL) was gradually added thereto at -40°C. The mixture was stirred for 2 hours while gradually raising the reaction temperature to 0°C. The reaction solution was neutralized with acetic acid, then concentrated under reduced pressure, purified by HPLC, and freeze-dried to obtain Compound 27 (32 mg, 31%) as a white solid.

EI-MS m/z: [M+H]⁺ 1905.4, 1/2[M+H]⁺ 953.8.

### Preparation of Compound 28

Compound 27 (32 mg, 0.017 mmol) was diluted with dichloromethane (3 mL), then trifluoroacetic acid (1 mL) was added thereto at 0°C, and the mixture was stirred for 2 hours. The reaction solution was concentrated under reduced pressure, purified by HPLC, and freeze-dried to obtain Compound 28 as a white solid (5.6 mg).

EI-MS m/z: [M+H]⁺ 1805.4, 1/2[M+H]⁺ 903.5.

### <Example 7> Preparation of Compound 32

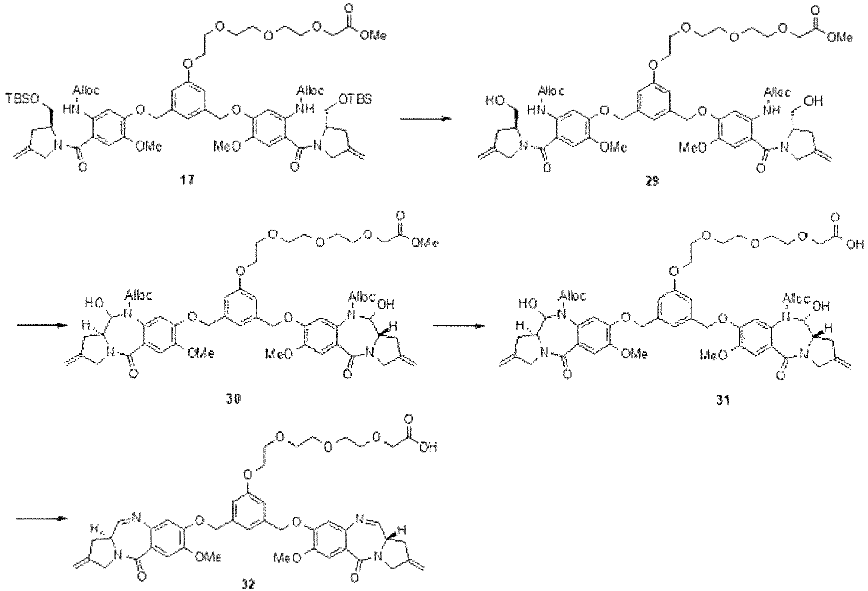

### Preparation of Compound 29

Compound 17 (350 mg, 0.27 mmol) was dissolved in tetrahydrofuran/distilled water (3 mL/3 mL), acetic acid (6 mL) was added thereto, and then the mixture was stirred at room temperature in a nitrogen atmosphere for 16 hours. Distilled water (30 mL) was added to the reaction solution, and then extraction with ethyl acetate (2 × 30 mL) was performed. The collected organic layer was washed with salt water (30 mL) and dried over anhydrous sodium sulfate. Filtration, concentration under reduced pressure, and purification by column chromatography were performed to obtain Compound 29 (197 mg, 70%).

¹H-NMR (400 MHz, CDCl₃) δ 8.40 (br s, 2H), 7.29 (s, 2H), 6.93 (s, 1H), 6.87 (s, 2H), 6.74 (s, 2H), 5.95-5.85 (m, 2H), 5.29 (dd, *J* = 17.2, 1.2 Hz, 2H), 5.23-5.16 (m, 6H), 4.98 (br s, 2H), 4.89 (br s, 2H), 4.64-4.52 (m, 5H), 4.16 (s, 4H), 4.08-4.00 (m, 3H), 3.88-3.83 (m, 4H), 3.80 (s, 6H), 3.74-3.64 (m, 13H), 2.76-2.66 (m, 2H), 2.48-2.40 (m, 2H).

### Preparation of Compound 30

Compound 29 (190 mg, 0.18 mmol) was dissolved in dichloromethane (5 mL), then Dess-Martin periodinane (185 mg, 0.44 mmol) was added thereto, and the mixture was stirred at room temperature in a nitrogen atmosphere for 18 hours. The reaction solution was concentrated under reduced pressure and purified by HPLC to obtain Compound 30 (168 mg, 89%).

### Preparation of Compound 31

Compound 30 (100 mg, 0.096 mmol) was dissolved in methanol/tetrahydrofuran (1 mL/3 mL), and then a solution prepared by dissolving lithium hydroxide (5 mg, 0.12 mmol) in distilled water (1 mL) was gradually added thereto at -40°C. The mixture was stirred for 2 hours while gradually raising the reaction temperature to 0°C. The reaction solution was neutralized with acetic acid, then concentrated under reduced pressure, purified by HPLC, and freeze-dried to obtain Compound 31 (95 mg, 96%).

EI-MS m/z: [M+H]⁺ 1029.9, [M+Na]⁺ 1051.8.

### Preparation of Compound 32

Compound 31 (40 mg, 0.039 mmol) was dissolved in dichloromethane (3 mL), then pyrrolidine (0.01 mL, 0.12 mmol) and tetrakis(triphenylphosphine)palladium(0) (2 mg, 0.0017 mmol) were added thereto, and the mixture was stirred at room temperature in a nitrogen atmosphere for 1 hour. The reaction solution was concentrated under reduced pressure and purified by HPLC to obtain Compound 32 (8.1 mg, 25%).

EI-MS m/z: [M+H]⁺ 825.7, 1/2[M+H]⁺ 413.4.

### <Example 8> Preparation of Compound 33

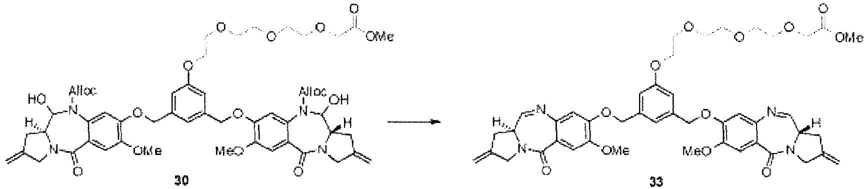

### Preparation of Compound 33

Compound 30 (50 mg, 0.048 mmol) was dissolved in dichloromethane (3 mL), then pyrrolidine (0.01 mL, 0.12 mmol) and tetrakis(triphenylphosphine)palladium(0) (2 mg, 0.0017 mmol) were added thereto, and the mixture was stirred at room temperature in a nitrogen atmosphere for 1 hour. The reaction solution was concentrated under reduced pressure and purified by HPLC to obtain Compound 33 (5.4 mg, 13%).

EI-MS m/z: [M+H]⁺ 839.7, 1/2[M+H]⁺ 420.4.

### <Example 9> Preparation of Compound 38

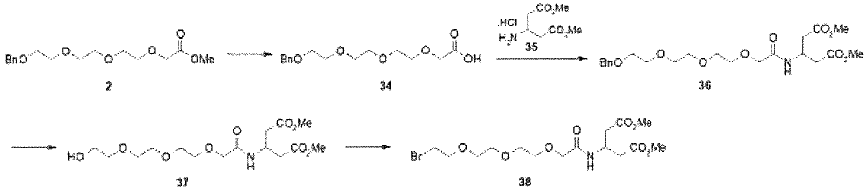

### Preparation of Compound 34

Compound 2 (3.6 g, 11.5 mmol) was dissolved in methanol/tetrahydrofuran (5 mL/10 mL), and then a solution prepared by dissolving lithium hydroxide (725 mg, 17.3 mmol) in distilled water (5 mL) was gradually added thereto at 0°C. The mixture was stirred for 2 hours, then the pH thereof was adjusted to about 2 with 1 N aqueous hydrochloric acid solution, and then extraction with ethyl acetate (2 × 30 mL) was performed. The collected organic layer was dried over anhydrous sodium sulfate, then filtered, and concentrated to obtain Compound 34 (2.8 g, 81%).

¹H-NMR (400 MHz, CDCl₃) δ 7.38-7.20 (m, 5H), 4.56 (s, 2H), 4.12 (s, 2H), 3.77-3.58 (m, 12H).

### Preparation of Compound 36

Compound 34 (2.8 g, 9.36 mmol) and Compound 35 (1.8 g, 8.51 mmol, Compound 35 was prepared by the method described in Korean Patent Publication No. 10-2018-0078329) were dissolved in *N,N*-dimethylformamide (6 mL), and then 1-hydroxybenzotriazole (1.7 g, 12.8 mmol), *N*-(3-dimethylaminopropyl)-*N*'-ethylcarbodiimide hydrochloride (2.4 g, 12.8 mmol), and triethylamine (9 mL, 34.0 mmol) were sequentially added thereto at 0°C in a nitrogen atmosphere. The reaction solution was stirred at room temperature for 24 hours, then saturated aqueous ammonium chloride solution (100 mL) was added to the reaction solution, and extraction with ethyl acetate (2 × 100 mL) was performed. The collected organic layer was washed with salt water (100 mL) and dried over anhydrous sodium sulfate. Filtration, concentration, and purification by column chromatography were performed to obtain Compound 36 (3.35 g, 78%).

¹H-NMR (400 MHz, CDCl₃) δ 7.44 (d, *J* = 8.0 Hz, 1H), 7.38-7.21 (m, 5H), 4.71-4.60 (m, 1H), 4.54 (d, *J* = 2.0 Hz, 1H), 3.95 (d, *J* = 2.4 Hz, 1H), 3.72-3.58 (m, 18H), 2.74-2.58 (m, 4H).

### Preparation of Compound 37

Compound 36 (3.3 g, 7.25 mmol) was dissolved in methanol (50 mL), and then palladium/charcoal (10% w/w, 330 mg) was added thereto. The reaction solution was stirred at room temperature in a hydrogen atmosphere for 18 hours. The reaction solution was filtered through Celite and then concentrated to obtain Compound 37 (2.14 g, 79%).

¹H-NMR (400 MHz, CDCl₃) δ 7.55 (d, *J* = 6.4 Hz, 1H), 4.65 (d, *J* = 5.6 Hz, 1H), 3.99 (s, 2H), 3.78-3.58 (m, 18H), 3.00 (bs, 1H), 2.79-2.62 (m, 4H).

### Preparation of Compound 38

Compound 37 (0.50 g, 1.37 mmol) was dissolved in tetrahydrofuran (5 mL), and then tetrabromomethane (0.545 g, 1.64 mmol) and triphenylphosphine (368 mg, 1.37 mmol) were sequentially added thereto at 0°C in a nitrogen atmosphere. The reaction solution was stirred for 6 hours, then diluted with ethyl acetate (50 mL), and washed with distilled water (50 mL). The collected organic layer was dried over anhydrous sodium sulfate, then filtered, concentrated, and purified by column chromatography to obtain Compound 38 (350 mg, 64%).

EI-MS m/z: [M+H]⁺ 398.3.

### <Example 10> Preparation of Compound 41

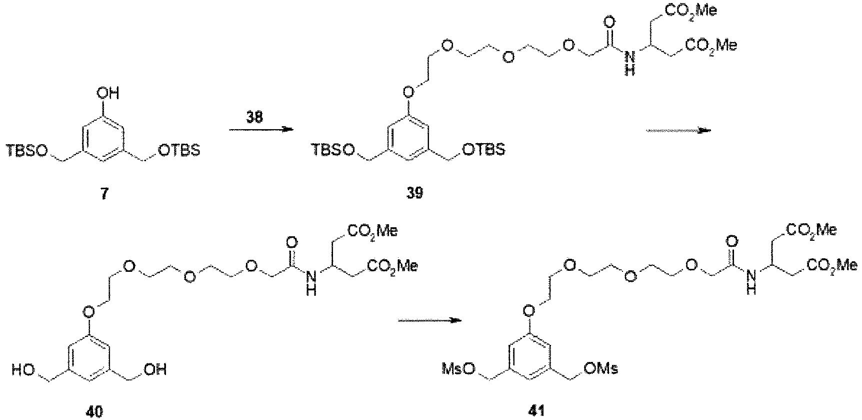

### Preparation of Compound 39

Compound 7 (1.2 g, 3.01 mmol) and Compound 38 (960 mg, 2.51 mmol) were dissolved in *N,N*-dimethylformamide (25 mL), then cesium carbonate (1.22 g, 3.76 mmol) was added thereto, and the mixture was stirred at room temperature in a nitrogen atmosphere for 16 hours. The reaction solution was diluted with distilled water (100 mL), and then extraction with ethyl acetate (2 × 100 mL) was performed. The extracted organic layer was washed with salt water (100 mL) and dried over anhydrous sodium sulfate. Filtration, concentration under reduced pressure, and purification by column chromatography were performed to obtain Compound 39 (1.65 g, 90%).

EI-MS m/z: [M+H]⁺ 731.1.

### Preparation of Compound 40

Compound 39 (1.65 g, 2.26 mmol) was dissolved in tetrahydrofuran (50 mL), and then tetrabutylammonium fluoride (1 M tetrahydrofuran solution, 5.2 mL, 5.2 mmol) was added thereto at 0°C in a nitrogen atmosphere. The mixture was stirred for 1 hour, then diluted with saturated aqueous ammonium chloride solution (100 mL), and subjected to extraction with ethyl acetate (2 × 100 mL). The collected organic layer was washed with salt water (100 mL) and dried over anhydrous sodium sulfate. Filtration, concentration under reduced pressure, and purification by column chromatography were performed to obtain Compound 40 (1.10 g, 97%).

EI-MS m/z: [M+H]⁺ 502.6.

### Preparation of Compound 41

Compound 40 (1.1 g, 2.19 mmol) was dissolved in dichloromethane (20 mL), and then triethylamine (0.76 mL, 5.48 mmol) and methanesulfonyl chloride (0.37 mL, 4.82 mmol) were added thereto at 0°C in a nitrogen atmosphere. The mixture was stirred for 1 hour, diluted with distilled water (30 mL), and subjected to extraction with dichloromethane (2 × 50 mL). The organic layer was dried over anhydrous sodium sulfate, filtered, and then concentrated to obtain Compound 41 (crude 1.4 g, 100%).

EI-MS m/z : [M+H]⁺ 658.5.

### <Example 11> Preparation of Compound 44

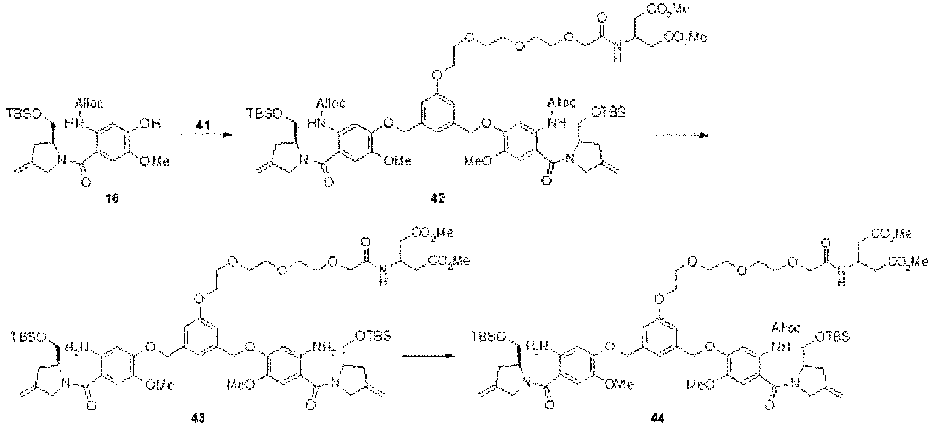

### Preparation of Compound 42

Compound 16 (2.4 g, 5.11 mmol) and Compound 41 (1.4 g, 2.13 mmol) were dissolved in *N,N*-dimethylformamide (10 mL), then potassium carbonate (1.45 g, 10.6 mmol) was added thereto at 0°C, and the mixture was stirred at room temperature for 20 hours in a nitrogen atmosphere. Distilled water (30 mL) was added to the reaction solution, and then extraction with ethyl acetate (2 × 50 mL) was performed. The collected organic layer was washed with salt water (50 mL) and dried over anhydrous sodium sulfate. Filtration, concentration under reduced pressure, and purification by column chromatography were performed to obtain Compound 42 (2.12 g, 70%).

¹H-NMR (400 MHz, CDCl₃) δ 8.81 (brs, 1H), 7.89 (brs, 1H), 7.43 (d, *J* = 6.4 Hz, 1H), 7.24 (s, 1H), 7.14 (s, 1H), 6.99 (s, 2H), 6.81 (s, 2H), 6.02-5.84 (m, 2H), 5.34 (d, *J* = 18.0 Hz, 2H), 5.22 (d, *J* = 10.8 Hz, 1H), 5.18-5.06 (m, 4H), 5.00-4.82 (m, 4H), 4.52-4.40 (m, 7H), 4.22-4.16 (m, 4H), 3.88-3.84 (m, 4H), 3.81 (s, 6H), 3.77-3.72 (m, 2H), 3.70-3.62 (m, 16H), 2.78-2.60 (m, 8H), 0.86 (s, 18H), 0.02 (s, 12H).

### Preparation of Compound 43

Compound 42 (2.0 g, 1.41 mmol) was dissolved in dichloromethane (20 mL), then pyrrolidine (0.29 mL, 3.52 mmol) and tetrakis(triphenylphosphine)palladium(0) (65 mg, 0.056 mmol) were added thereto, and the mixture was stirred at room temperature in a nitrogen atmosphere for 3 hours. Distilled water (30 mL) was added to the reaction solution, and then extraction with dichloromethane (2 × 50 mL) was performed. The collected organic layer was washed with salt water (50 mL) and dried over anhydrous sodium sulfate. Filtration, concentration under reduced pressure, and purification by column chromatography were performed to obtain Compound 43 (1.42 g, 81%).

EI-MS m/z: [M+H]⁺ 1251.5, 1/2[M+H]⁺ 626.4.

### Preparation of Compound 44

Compound 43 (1.3 g, 1.04 mmol) was dissolved in dichloromethane (50 mL), then pyridine (0.16 mL, 2.08 mmol) and allyl chloroformate (0.088 mL, 0.83 mmol) were added thereto at -78°C in a nitrogen atmosphere, and the reaction solution was stirred for 1 hour. Distilled water (50 mL) was added to the reaction solution, and then extraction with dichloromethane (2 × 50 mL) was performed. The collected organic layer was washed with salt water (50 mL) and dried over anhydrous sodium sulfate. Filtration, concentration under reduced pressure, and purification by column chromatography were performed to obtain Compound 44 (450 mg, 32%).

EI-MS m/z: [M+H]⁺ 1903.8, 1/2[M+H]⁺ 952.4.

### <Example 12> Preparation of Compound 47

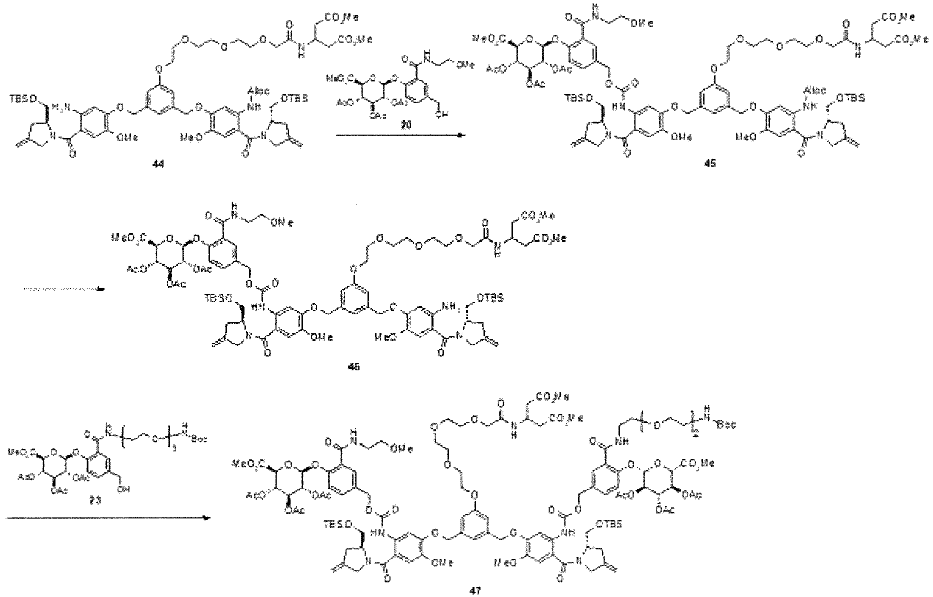

### Preparation of Compound 45

Compound 44 (440 mg, 0.33 mmol) was dissolved in dry tetrahydrofuran (20 mL), then triphosgene (40 mg, 0.14 mmol) and triethylamine (0.074 mL, 0.53 mmol) were added thereto at -10°C, and the mixture was stirred in a nitrogen atmosphere for 1 hour. Compound 20 (196 mg, 0.36 mmol) was dissolved in dry tetrahydrofuran (5 mL), triethylamine (0.074 mL, 0.53 mmol) was added thereto, and then this solution was gradually added to the reaction solution. After 1 hour, the reaction solution was heated to reflux and stirred for 12 hours. The reaction solution was diluted with ethyl acetate (30 mL), washed with salt water (20 mL), and dried over anhydrous sodium sulfate. Filtration, concentration under reduced pressure, and purification by column chromatography were performed to obtain Compound 45 (295 mg, 47%).

EI-MS m/z: [M+H^{]+} 1903.8, 1/2[M+H]⁺ 952.4.

### Preparation of Compound 46

Compound 45 (280 mg, 0.15 mmol) was dissolved in dichloromethane (10 mL), then pyrrolidine (0.016 mL, 0.19 mmol) and tetrakis(triphenylphosphine)palladium(0) (4 mg, 0.0029 mmol) were added thereto, and the mixture was stirred at room temperature in a nitrogen atmosphere for 2 hours. Distilled water (20 mL) was added to the reaction solution, and then extraction with dichloromethane (2 × 20 mL) was performed. The collected organic layer was washed with salt water (20 mL) and dried over anhydrous sodium sulfate. Filtration, concentration under reduced pressure, and purification by column chromatography were performed to obtain Compound 46 (250 mg, 93%).

EI-MS m/z: [M+H]⁺ 1819.4, 1/2[M+H]⁺ 910.3.

### Preparation of Compound 47

Compound 46 (250 mg, 0.14 mmol) was dissolved in dry tetrahydrofuran (10 mL), then triphosgene (16 mg, 0.055 mmol) and triethylamine (0.020 mL, 0.41 mmol) were added thereto at -10°C, and the mixture was stirred in a nitrogen atmosphere for 1 hour. Compound 23 (100 mg, 0.14 mmol) was dissolved in dry tetrahydrofuran (3 mL), triethylamine (0.020 mL, 0.41 mmol) was added thereto, and then this solution was gradually added to the reaction solution. After 1 hour, the reaction solution was heated to reflux and stirred for 12 hours. The reaction solution was diluted with ethyl acetate (30 mL), washed with salt water (20 mL), and dried over anhydrous sodium sulfate. Filtration, concentration under reduced pressure, and purification by column chromatography were performed to obtain Compound 47 (208 mg, 72%).

EI-MS m/z: [M+H]⁺ 2575.7, 1/2[M+H]⁺ 1288.8.

### <Example 13> Preparation of Compound 48

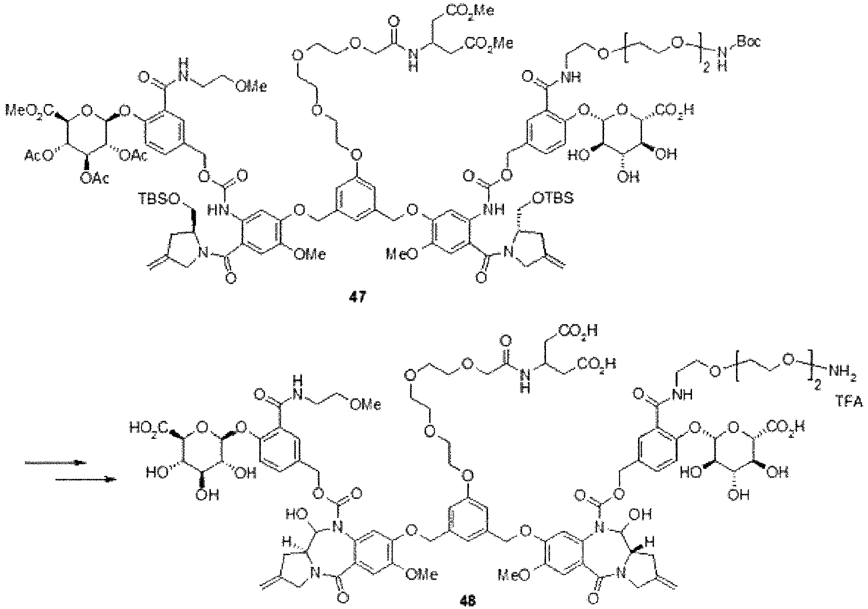

Compound 48 was prepared from Compound 47 by a method similar to that for the synthesis of Compound 28.

### <Example 14> Preparation of Compound 52

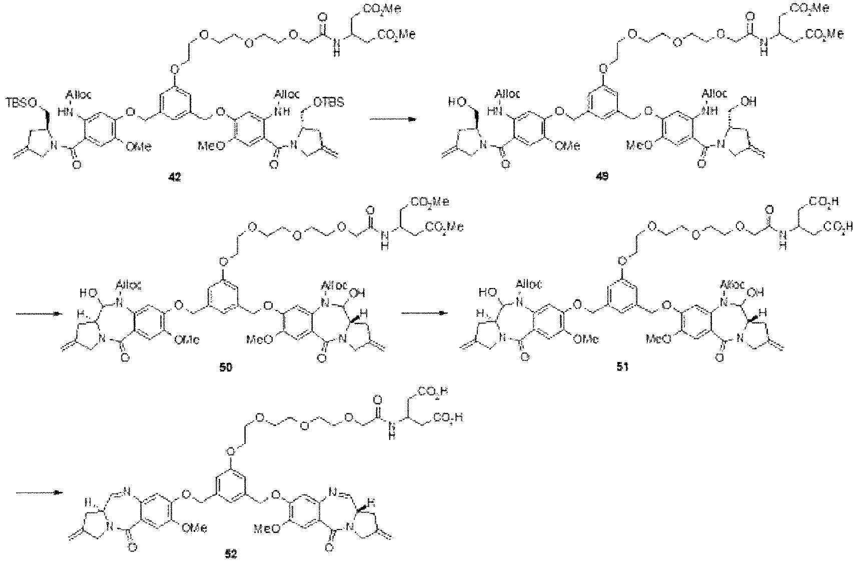

### Preparation of Compound 49

Compound 42 (250 mg, 0.18 mmol) was dissolved in tetrahydrofuran/distilled water (1.5 mL/1.5 mL), acetic acid (3 mL) was added thereto, and the mixture was stirred at room temperature in a nitrogen atmosphere for 16 hours. Distilled water (30 mL) was added to the reaction solution, and then extraction with ethyl acetate (2 × 30 mL) was performed. The collected organic layer was washed with salt water (30 mL) and dried over anhydrous sodium sulfate. Filtration, concentration under reduced pressure, and purification by column chromatography were performed to obtain Compound 49 (190 mg, 91%).

EI-MS m/z: [M+H]⁺ 1191.2, [M+Na]⁺ 1213.0.

### Preparation of Compound 50

Compound 49 (190 mg, 0.16 mmol) was dissolved in dichloromethane (5 mL), then Dess-Martin periodinane (162 mg, 0.39 mmol) was added thereto, and the mixture was stirred at room temperature in a nitrogen atmosphere for 15 hours. The reaction solution was concentrated under reduced pressure and then purified by HPLC to obtain Compound 50 (151 mg, 79%).

EI-MS m/z: [M+H]⁺ 1187.3, [M+Na]⁺ 1208.8.

### Preparation of Compound 51

Compound 50 (70 mg, 0.059 mmol) was dissolved in methanol/tetrahydrofuran (1 mL/2 mL), and then a solution prepared by dissolving lithium hydroxide (6.2 mg, 0.15 mmol) in distilled water (1 mL) was gradually added thereto at 0°C. The mixture was stirred for 16 hours, the reaction solution was neutralized with acetic acid, concentrated under reduced pressure, then purified by HPLC, and freeze-dried to obtain Compound 51 (61 mg, 89%).

EI-MS m/z: [M+H]⁺ 1159.0, [M+Na^{]+} 1181.0.

### Preparation of Compound 52

Compound 51 (60 mg, 0.052 mmol) was dissolved in dichloromethane (3 mL), then pyrrolidine (0.011 mL, 0.13 mmol) and tetrakis(triphenylphosphine)palladium(0) (2.3 mg, 0.0021 mmol) were added thereto, and the mixture was stirred at room temperature in a nitrogen atmosphere for 3 hours. The reaction solution was concentrated under reduced pressure and then purified by HPLC to obtain Compound 52 (23 mg, 47%).

EI-MS m/z: [M+H]⁺ 954.6, 1/2[M+H]⁺ 477.9.

### <Example 15> Preparation of Compound 53

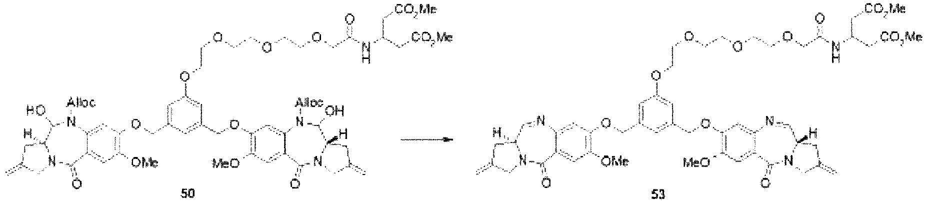

### Preparation of Compound 53

Compound 50 (30 mg, 0.025 mmol) was dissolved in dichloromethane (3 mL), then pyrrolidine (0.005 mL, 0.063 mmol) and tetrakis(triphenylphosphine)palladium(0) (1 mg, 0.001 mmol) were added thereto, and the mixture was stirred at room temperature in a nitrogen atmosphere for 1 hour. The reaction solution was concentrated under reduced pressure and then purified by HPLC to obtain Compound 53 (11.2 mg, 45%).

EI-MS m/z: [M+H]⁺ 982.9, 1/2[M+H]⁺ 491.9.

### <Example 16> Preparation of Compound 56

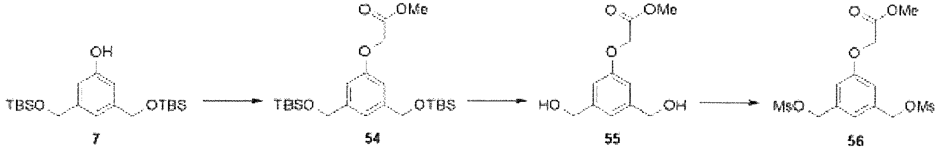

### Preparation of Compound 54

Compound 7 (3.5 g, 9.14 mmol) and methyl bromoacetate (1.68 g, 10.97 mmol) were dissolved in *N,N-*dimethylformamide (35 mL), then cesium carbonate (4.5 g, 13.71 mmol) was added thereto, and the mixture was stirred at room temperature in a nitrogen atmosphere for 16 hours. The reaction solution was diluted with ethyl acetate (100 mL) and washed with distilled water (3 × 100 mL). The washed organic layer was washed with salt water (100 mL) and dried over anhydrous sodium sulfate. Filtration and concentration under reduced pressure were performed to obtain Compound 54 (3.83 g, 92%).

¹H-NMR (400 MHz, CDCl₃) δ 6.89 (s, 1H), 6.77 (s, 2H), 4.70 (s, 4H), 4.64 (s, 2H), 3.80 (s, 3H).

### Preparation of Compound 55

Compound 54 (3.8 g, 8.35 mmol) was dissolved in methanol (30 mL), and then camphorsulfonic acid (776 mg, 3.34 mmol) was added thereto at 0°C in a nitrogen atmosphere. The mixture was stirred at room temperature for 5 hours, then triethylamine (0.8 mL) was added thereto, the solvent was concentrated under reduced pressure, and purification by column chromatography was performed to obtain Compound 55 (1.83 g, 97%).

¹H-NMR (400 MHz, CDCl₃) δ 6.99 (s, 1H), 6.85 (s, 2H), 4.67 (s, 4H), 4.66 (s, 2H), 3.81 (s, 3H).

### Preparation of Compound 56

Compound 55 (700 mg, 3.09 mmol) was dissolved in dichloromethane (25 mL), then triethylamine (1.3 mL, 9.27 mmol) and methanesulfonyl chloride (0.53 mL, 6.81 mmol) were added thereto at 0°C in a nitrogen atmosphere, and then the mixture was stirred for 1 hour. The reaction solution was diluted with dichloromethane (50 mL) and washed with distilled water (50 mL). The washed organic layer was washed with salt water (50 mL) and dried over anhydrous sodium sulfate. Filtration, concentration, and purification by column chromatography were performed to obtain Compound 56 (0.95 g, 81%).

¹H-NMR (400 MHz, CDCl₃) δ 7.09 (s, 1H), 6.98 (s, 2H), 5.21 (s, 4H), 4.68 (s, 2H), 3.82 (s, 3H), 2.99 (s, 6H).

### <Example 17> Preparation of Compound 60

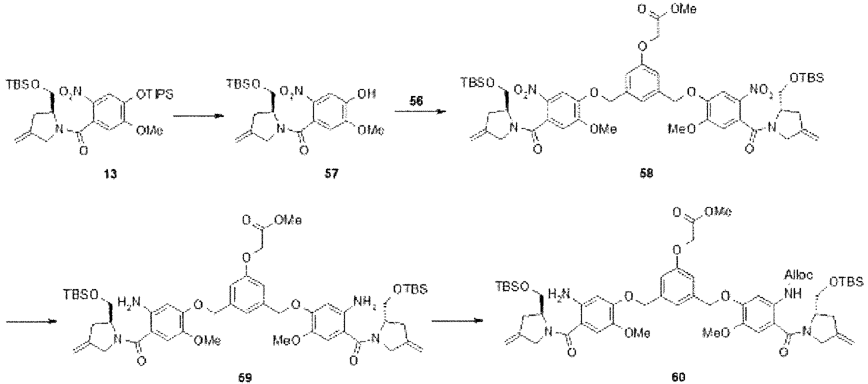

### Preparation of Compound 57

Compound 13 (36 g, 62.1 mmol) was dissolved in *N,N-*dimethylformamide/distilled water (300 mL/6 mL), then sodium acetate (6.1 g, 74.6 mmol) was added thereto, and the reaction solution was stirred at room temperature in a nitrogen atmosphere. After 3 hours, distilled water (500 mL) was added thereto, and extraction with ethyl acetate (2 × 200 mL) was performed. The collected organic layer was washed with distilled water (200 mL) and salt water (200 mL) in this order, and then dried over anhydrous sodium sulfate. Filtration, concentration, and purification by column chromatography were performed to obtain Compound 57 (26 g, 99%).

¹H-NMR (400 MHz, CDCl₃) (rotamers) δ 7.77-7.76 (m, 1H), 6.81-6.76 (m, 1H), 6.05-6.01 (m, 1H), 5.10-4.83 (m, 2H), 4.58-4.54 (m, 1H), 3.99 (s, 3H), 3.89-3.56 (m, 3H), 3.34-3.26 (m, 1H), 2.81-2.67 (m, 2H), 0.89 (s, 9H), 0.09 (s, 6H).

### Preparation of Compound 58

Compound 57 (2.31 g, 5.46 mmol) and Compound 56 (0.95 g, 2.48 mmol) were dissolved in *N,N-*dimethylformamide (20 mL), then potassium carbonate (1.71 g, 12.40 mmol) was added thereto at 0°C, and the mixture was stirred at room temperature in a nitrogen atmosphere for 20 hours. The reaction solution was diluted with ethyl acetate (100 mL) and washed with distilled water (3 × 100 mL). The washed organic layer was washed with salt water (100 mL) and dried over anhydrous sodium sulfate. Filtration, concentration under reduced pressure, and purification by column chromatography were performed to obtain Compound 58 (2.45 g, 95%).

EI-MS m/z: [M+H]⁺ 1035.6.

### Preparation of Compound 59

Compound 58 (2.45 g, 2.36 mmol) was dissolved in ethyl acetate (12 mL) and ethanol (12 mL), then zinc dust (9.28 g, 141.98 mmol) was added thereto, and formic acid (3.56 mL, 94.4 mmol) diluted with ethanol (12 mL) was added thereto. The mixture was stirred at room temperature for 4 hours, diluted with ethyl acetate (30 mL), and filtered through Celite, and ethyl acetate (100 mL) was added to the filtered solution. The organic layer was washed with distilled water (150 mL), saturated aqueous sodium hydrogen carbonate solution (150 mL), and salt water (100 mL) in this order, and then dried over anhydrous sodium sulfate. Filtration, concentration, and purification by column chromatography were performed to obtain Compound 59 (1.89 g, 82%).

EI-MS m/z: [M+H]⁺ 975.3, 1/2[M+H]⁺ 488.4.

### Preparation of Compound 60

Compound 59 (1.89 g, 1.94 mmol) was dissolved in dichloromethane (150 mL), then pyridine (0.31 mL, 3.88 mmol) and allyl chloroformate (0.19 mL, 1.74 mmol) were added thereto at -78°C in a nitrogen atmosphere, and the reaction solution was stirred for 3 hours. The reaction solution was concentrated under reduced pressure and then purified by column chromatography to obtain Compound 60 (923 mg, 44%).

### <Example 18> Preparation of Compound 63

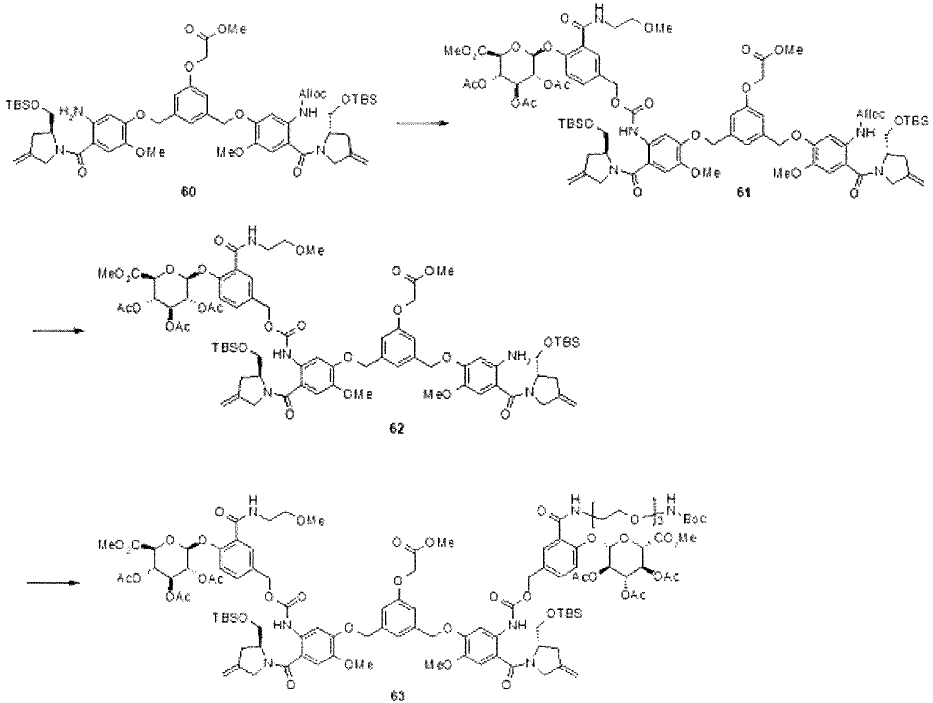

### Preparation of Compound 61

Compound 60 (923 mg, 0.87 mmol) and triphosgene (93 mg, 0.31 mmol) were dissolved in dry dichloromethane (45 mL), then N-methylimidazole (0.24 mL, 4.35 mmol) was added thereto at -10°C, and the mixture was stirred in a nitrogen atmosphere for 1 hour. Compound 20 (196 mg, 0.36 mmol) was dissolved in dry dichloromethane (15 mL), *N-*methylimidazole (0.06 mL, 1.04 mmol) was added thereto, then this solution was gradually added to the reaction solution, and the mixture was stirred at room temperature for 17 hours. The reaction solution was diluted with dichloromethane (20 mL), washed with distilled water (30 mL), and dried over anhydrous sodium sulfate. Filtration, concentration under reduced pressure, and purification by column chromatography were performed to obtain Compound 61 (1.01 g, 71%).

EI-MS m/z: [M+H]⁺ 1624.3, 1/2[M+H]⁺ 814.0.

### Preparation of Compound 62

Compound 61 (1.01 g, 0.62 mmol) was dissolved in dichloromethane (60 mL), then sodium 2-ethylhexanoate (155 mg, 0.93 mmol) and tetrakis(triphenylphosphine)palladium(0) (36 mg, 0.03 mmol) were added thereto, the mixture was stirred at room temperature in a nitrogen atmosphere for 2 hours, and then sodium 2-ethylhexanoate (50 mg, 0.30 mmol) and tetrakis(triphenylphosphine)palladium(0) (12 mg, 0.01 mmol) were further added thereto. The reaction product was concentrated under reduced pressure and then purified by column chromatography to obtain Compound 62 (900 mg, 94%).

EI-MS m/z: [M+H]⁺ 1543.5, 1/2[M+H]⁺ 772.0.

### Preparation of Compound 63

Compound 62 (900 mg, 0.58 mmol) and triphosgene (62 mg, 0.21 mmol) were dissolved in dry dichloromethane (30 mL), then *N*-methylimidazole (0.16 mL, 2.90 mmol) was added thereto at -10°C, and the mixture was stirred in a nitrogen atmosphere for 1 hour. Compound 23 (508 mg, 0.69 mmol) was dissolved in dry dichloromethane (10 mL), N-methylimidazole (0.04 mL, 0.69 mmol) was added thereto, then this solution was gradually added to the reaction solution, and the mixture was stirred at room temperature for 17 hours. The reaction solution was diluted with dichloromethane (20 mL), washed with distilled water (30 mL), and dried over anhydrous sodium sulfate. Filtration, concentration under reduced pressure, and purification by column chromatography were performed to obtain Compound 63 (827 mg, 62%).

EI-MS m/z: [M+H]⁺ 2299.79, 1/2[M+H]⁺ 1150.3.

### <Example 19> Preparation of Compound 64

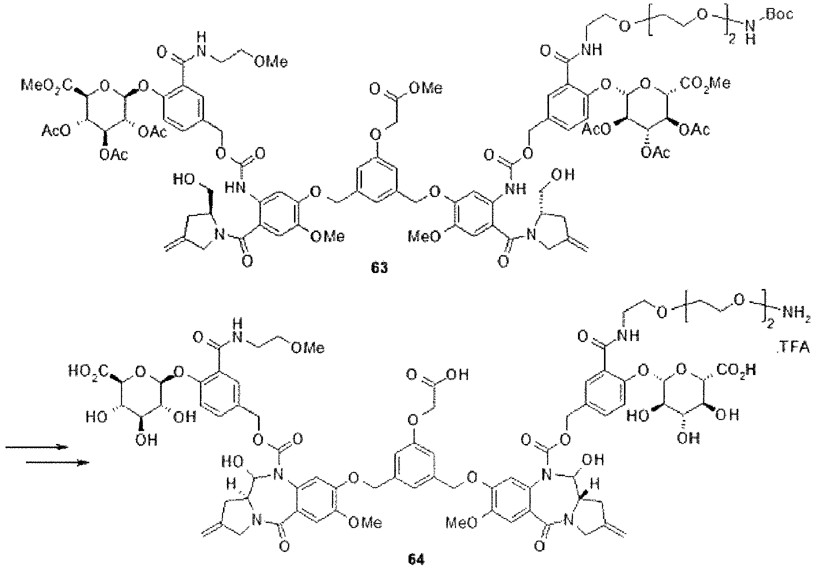

Compound 64 was prepared from Compound 63 by a method similar to that for the synthesis of Compound 28.

### <Example 20> Preparation of Compound 68

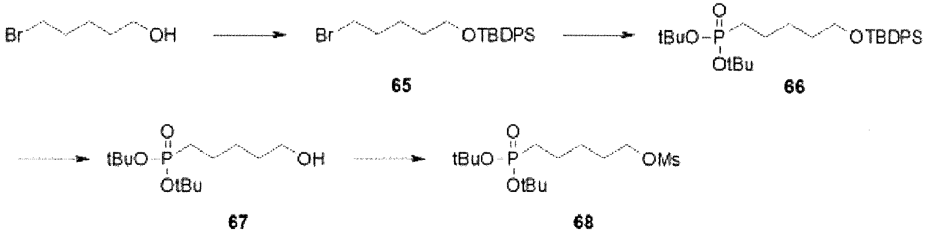

### Preparation of Compound 65

After 5-bromopentan-1-ol (5 g, 29.93 mmol) was dissolved in tetrahydrofuran (70 mL), imidazole (5.09 g, 47.82 mmol) and t-butyldiphenylchlorosilane (8.6 mL, 32.92 mmol) were sequentially added thereto at 0°C, and the mixture was stirred for 4 hours. The solvent was concentrated under reduced pressure, and the resultant was diluted with ethyl acetate (100 mL), washed with distilled water (100 mL), and dried over anhydrous sodium sulfate. Filtration, concentration under reduced pressure, and purification by column chromatography were performed to obtain Compound 65 (11.2 g, 92%).

¹H-NMR (400 MHz, CDCl₃) δ 7.68-7.64 (m, 4H), 7.45-7.35 (m, 6H), 3.66 (t, *J* = 6.0 Hz, 2H), 3.38 (t, *J* = 6.8 Hz, 2H), 1.84 (m, 2H), 1.61-1.47 (m, 6H), 1.05 (s, 9H).

### Preparation of Compound 66

Di-t-butyl phosphite (2 mL, 10 mmol) was diluted with *N,N*-dimethylformamide (8 mL), sodium hydride (0.24 g, 10 mmol) was added thereto, and the mixture was stirred at 0°C for 30 minutes. Compound 65 (2 g, 5 mmol) diluted with *N,N*-dimethylformamide (8 mL) was added thereto, and the mixture was stirred in a nitrogen atmosphere for 16 hours. Methanol (0.5 mL) was added thereto, the mixture was diluted with ethyl acetate (70 mL), washed with saturated ammonium chloride solution (50 mL) and salt water (50 mL) in this order, and dried over anhydrous sodium sulfate. Filtration, concentration under reduced pressure, and purification by column chromatography were performed to obtain Compound 66 (2.35 g, 90%).

¹H-NMR (400 MHz, CDCl₃) δ 7.67-7.65 (m, 4H), 7.44-7.35 (m, 6H), 3.66 (t, *J* = 6.4 Hz, 2H), 1.65-1.40 (m, 24H), 1.20 (s, 9H).

### Preparation of Compound 67

Compound 66 (3.3 g, 6.36 mmol) was dissolved in tetrahydrofuran (40 mL), and then tetrabutylammonium fluoride (1 M tetrahydrofuran solution, 7.63 mL, 7.63 mmol) was added thereto at 0°C in a nitrogen atmosphere. The mixture was stirred for 2 hours, then concentrated under reduced pressure, and purified by column chromatography to obtain Compound 67 (1.76 g, 99%).

¹H-NMR (400 MHz, CDCl₃) δ 3.65 (t, *J* = 6.4 Hz, 2H), 1.72-1.33 (m, 26H).

### Preparation of Compound 68

Compound 67 (1.76 g, 6.28 mmol) was dissolved in dichloromethane (40 mL), and then triethylamine (1.3 mL, 9.42 mmol) and methanesulfonyl chloride (0.58 mL, 7.53 mmol) were added thereto at 0°C in a nitrogen atmosphere. The mixture was stirred for 2 hours, then diluted with dichloromethane (30 mL), and washed with distilled water (40 mL). The organic layer was dried over anhydrous sodium sulfate, filtered, and then concentrated to obtain Compound 68 (crude 2.37 g, 100%).

¹H-NMR (400 MHz, CDCl₃) δ 4.23 (t, *J* = 6.0 Hz, 2H), 3.01 (s, 3H), 1.77 (m, 2H), 1.68-1.56 (m, 4H), 1.53-1.40 (m, 20H).

### <Example 21> Preparation of Compound 71

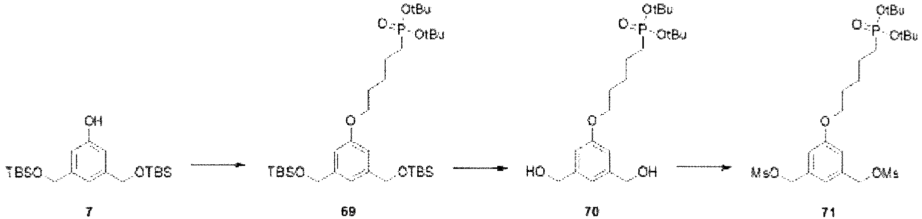

### Preparation of Compound 69

Compound 7 (1.87 g, 4.90 mmol) and Compound 68 (2.11 g, 5.88 mmol) were dissolved in *N,N*-dimethylformamide (20 mL), then cesium carbonate (2.39 g, 7.35 mmol) and sodium iodide (147 mg, 0.98 mmol) were added thereto, and the mixture was stirred at room temperature in a nitrogen atmosphere for 4 hours and then at 60°C for 15 hours. The reaction solution was diluted with ethyl acetate (100 mL) and washed with distilled water (3 × 80 mL). The washed organic layer was washed with salt water (70 mL) and dried over anhydrous sodium sulfate. Filtration and concentration under reduced pressure were performed to obtain Compound 69 (crude 3.56 g, 100%).

¹H-NMR (400 MHz, CDCl₃) δ 6.84 (s, 1H), 6.75 (s, 2H), 4.69 (s, 4H), 3.95 (t, *J* = 6.4 Hz, 2H), 1.84-1.72 (m, 2H), 1.72-1.42 (m, 28H), 0.94 (s, 18H), 0.09 (s, 12H).

### Preparation of Compound 70

Compound 69 (crude 3.56 g, 4.90 mmol) was dissolved in tetrahydrofuran (50 mL), and then tetrabutylammonium fluoride (1 M tetrahydrofuran solution, 16.6 mL, 16.6 mmol) was added thereto at 0°C in a nitrogen atmosphere. The mixture was stirred for 2 hours and then concentrated under reduced pressure to obtain Compound 70 (crude 2.04 g, 100%).

¹H-NMR (400 MHz, CDCl₃) δ 6.92 (s, 1H), 6.85 (s, 2H), 4.67 (s, 2H), 4.65 (s, 2H), 3.99 (t, *J* = 6.4 Hz, 2H), 2.05 (t, *J* = 5.6 Hz, 2H), 1.78 (m, 2H), 1.71-1.48 (m, 26H) .

### Preparation of Compound 71

Compound 70 (crude 2.04 g, 4.90 mmol) was dissolved in dichloromethane (50 mL), and then triethylamine (2 mL, 14.40 mmol) and methanesulfonyl chloride (0.82 mL, 10.56 mmol) were added thereto at 0°C in a nitrogen atmosphere. The mixture was stirred for 2 hours, then diluted with dichloromethane (30 mL), and washed with distilled water (50 mL). The organic layer was dried over anhydrous sodium sulfate, filtered, and then concentrated to obtain Compound 71 (crude 2.91 g, 100%).

¹H-NMR (400 MHz, CDCl₃) δ 7.02 (s, 1H), 6.95 (s, 2H), 5.20 (s, 4H), 3.98 (t, *J* = 6.8 Hz, 2H), 2.99 (s, 6H), 1.83 (m, 2H), 1.71-1.51 (m, 6H), 1.50 (s, 18H).

### <Example 22> Preparation of Compound 74

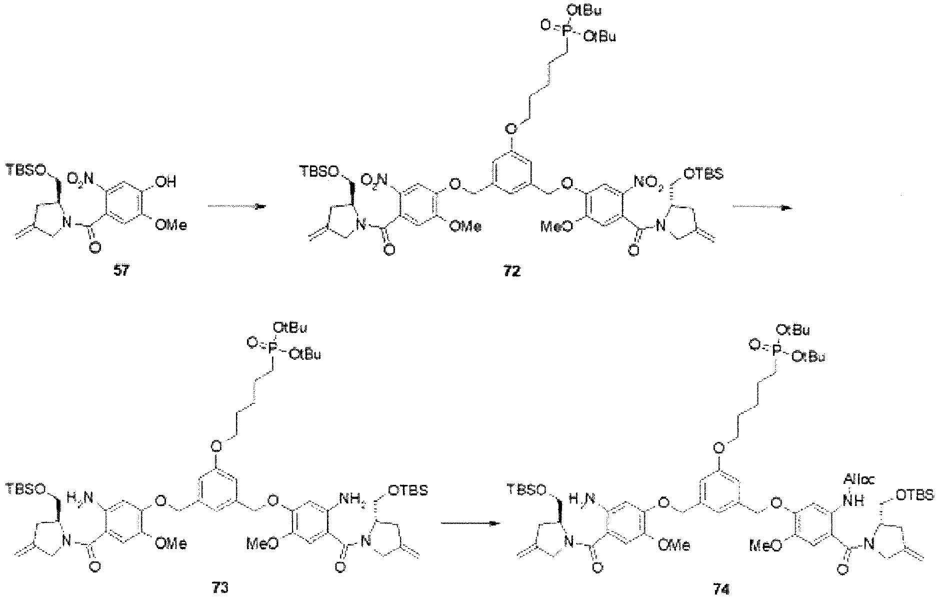

### Preparation of Compound 72

Compound 57 (1.2 g, 2.84 mmol) and Compound 71 (766 mg, 1.29 mmol) were dissolved in *N,N*-dimethylformamide (20 mL), then potassium carbonate (0.89 g, 6.45 mmol) was added thereto at 0°C, and the mixture was stirred at room temperature for 20 hours in a nitrogen atmosphere. The reaction solution was diluted with ethyl acetate (100 mL) and washed with distilled water (3 × 100 mL). The washed organic layer was washed with salt water (100 mL) and dried over anhydrous sodium sulfate. Filtration, concentration under reduced pressure, and purification by column chromatography were performed to obtain Compound 72 (0.85 g, 53%).

### Preparation of Compound 73

Compound 72 (850 mg, 0.69 mmol) was dissolved in ethyl acetate (3 mL) and ethanol (3 mL), then zinc dust (2.72 g, 41.6 mmol) was added thereto, and formic acid (1 mL, 27.6 mmol) diluted with ethanol (4 mL) was added thereto. The mixture was stirred at room temperature for 5 hours, then diluted with ethyl acetate (30 mL), and filtered through Celite, and ethyl acetate (70 mL) was added to the filtered solution. The organic layer was washed with distilled water (50 mL), saturated aqueous sodium hydrogen carbonate solution (50 mL), and salt water (50 mL) in this order, and dried over anhydrous sodium sulfate. Filtration, concentration, and purification by column chromatography were performed to obtain Compound 73 (565 mg, 70%).

### Preparation of Compound 74

Compound 73 (2.03 g, 1.74 mmol) was dissolved in dichloromethane (150 mL), then pyridine (0.28 mL, 3.48 mmol) was added thereto, allyl chloroformate (0.17 mL, 1.57 mmol) diluted with dichloromethane (60 mL) was added thereto at -78°C in a nitrogen atmosphere, and the reaction solution was stirred for 3 hours. The reaction solution was concentrated under reduced pressure and then purified by column chromatography to obtain Compound 74 (1.06 g, 49%).

### <Example 23> Preparation of Compound 77

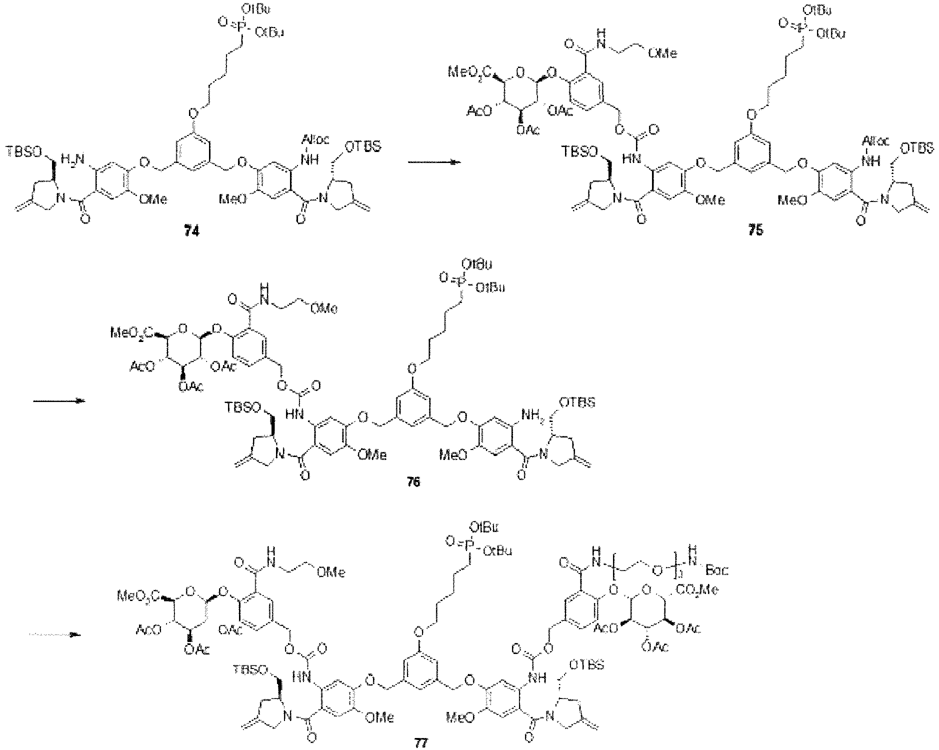

### Preparation of Compound 75

Compound 74 (1.06 g, 0.87 mmol) and triphosgene (91 mg, 0.31 mmol) were dissolved in dry dichloromethane (45 mL), then *N*-methylimidazole (0.23 mL, 4.20 mmol) was added thereto at -10°C, and the mixture was stirred in a nitrogen atmosphere for 1 hour. Compound 20 (500 mg, 0.92 mmol) was dissolved in dry dichloromethane (15 mL), *N-*methylimidazole (0.06 mL, 1.00 mmol) was added thereto, then this solution was gradually added to the reaction solution, and the mixture was stirred at room temperature for 17 hours. The reaction solution was diluted with dichloromethane (20 mL), washed with distilled water (30 mL), and dried over anhydrous sodium sulfate. Filtration, concentration under reduced pressure, and purification by column chromatography were performed to obtain Compound 75 (1.27 g, 83%).

EI-MS m/z: [M+H]⁺ 1818.4, 1/2[M+H]⁺ 909.6.

### Preparation of Compound 76

Compound 75 (1.27 mg, 0.69 mmol) was dissolved in dichloromethane (60 mL), then pyrrolidine (0.34 mL, 4.19 mmol) and tetrakis(triphenylphosphine)palladium(0) (40 mg, 0.035 mmol) were added thereto, and the mixture was stirred at room temperature in a nitrogen atmosphere for 1 hour. The reaction solution was concentrated under reduced pressure and then purified by column chromatography to obtain Compound 76 (1.1 g, 90%).

EI-MS m/z : [M+H]⁺ 1733.8, 1/2[M+H]⁺ 867.3.

### Preparation of Compound 77

Compound 76 (1.1 g, 0.63 mmol) and triphosgene (68 mg, 0.22 mmol) were dissolved in dry dichloromethane (30 mL), then *N*-methylimidazole (0.17 mL, 3.15 mmol) was added thereto at -10°C, and the mixture was stirred in a nitrogen atmosphere for 1 hour. Compound 23 (506 mg, 0.69 mmol) was dissolved in dry dichloromethane (10 mL), *N-*methylimidazole (0.04 mL, 0.75 mmol) was added thereto, then this solution was gradually added to the reaction solution, and the mixture was stirred at room temperature for 17 hours. The reaction solution was diluted with dichloromethane (20 mL), washed with distilled water (30 mL), and dried over anhydrous sodium sulfate. Filtration, concentration under reduced pressure, and purification by column chromatography were performed to obtain Compound 77 (500 mg, 32%).

EI-MS m/z: [M+H]⁺ 2490.2, 1/2[M+H]⁺ 1245.7.

### <Example 24> Preparation of Compound 81

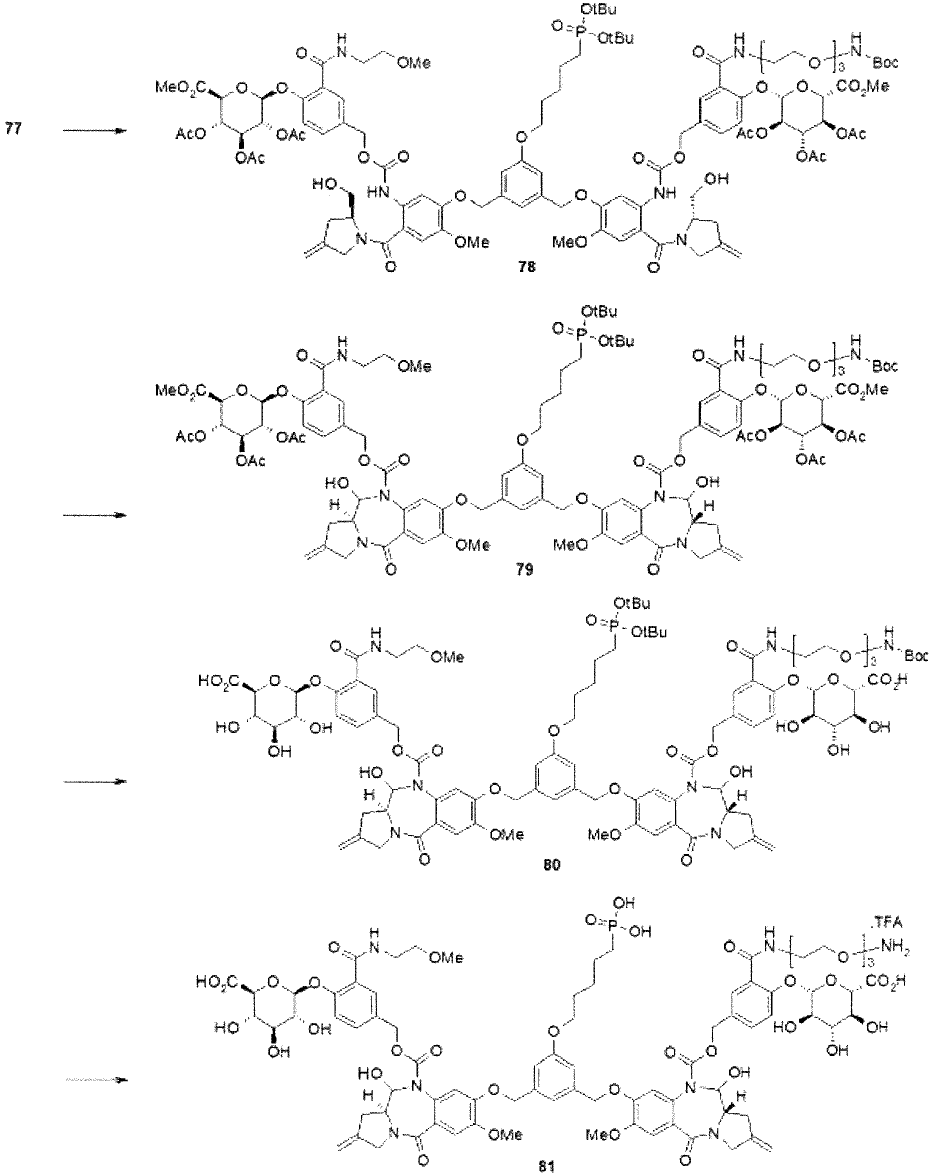

### Preparation of Compound 78

Compound 77 (500 mg, 0.20 mmol) was dissolved in tetrahydrofuran/distilled water (5 mL/5 mL), acetic acid (10 mL) was added thereto, and the mixture was stirred at room temperature in a nitrogen atmosphere for 16 hours. The reaction solution was diluted with ethyl acetate (60 mL), washed with distilled water (50 mL) and saturated sodium carbonate solution (2 × 50 mL), and then dried over anhydrous sodium sulfate. Filtration, concentration under reduced pressure, and purification by column chromatography were performed to obtain Compound 78 (240 mg, 52%).

EI-MS m/z: [M+H]⁺ 2262.2, [M+Na]⁺ 2284.0.

### Preparation of Compound 79

Compound 78 (240 mg, 0.106 mmol) was dissolved in dichloromethane (10 mL), then Dess-Martin periodinane (104 mg, 0.24 mmol) was added thereto, and the mixture was stirred at room temperature in a nitrogen atmosphere for 90 minutes. The reaction solution was diluted with dichloromethane (20 mL), washed with saturated aqueous sodium hydrogen carbonate solution (30 mL), and dried over anhydrous sodium sulfate. Filtration, concentration under reduced pressure, and purification by column chromatography were performed to obtain Compound 79 (134 mg, 56%).

EI-MS m/z: [M+H]⁺ 2258.1, 1/2[M+H]⁺ 1129.3.

### Preparation of Compound 80

Compound 79 (134 mg, 0.059 mmol) was dissolved in methanol (4.2 mL), and then a solution prepared by dissolving lithium hydroxide (22 mg, 0.534 mmol) in distilled water (4.2 mL) was gradually added thereto at - 50°C, and the mixture was stirred at room temperature for 3 hours. The reaction solution was neutralized with formic acid, concentrated under reduced pressure, and then freeze-dried to obtain Compound 80 (crude 147 mg, 100%).

### Preparation of Compound 81

Compound 80 (crude 147 mg, 0.059 mmol) was diluted with dichloromethane (4 mL), then trifluoroacetic acid (1 mL) was added thereto, and the mixture was stirred for 1 hour. The reaction solution was concentrated under reduced pressure, then purified by HPLC, and freeze-dried to obtain Compound 81 as a white solid (45 mg, 41%).

EI-MS m/z : [M+H]⁺ 1765.3, 1/2[M+H]⁺ 883.1.

### <Example 25> Preparation of Compound 87

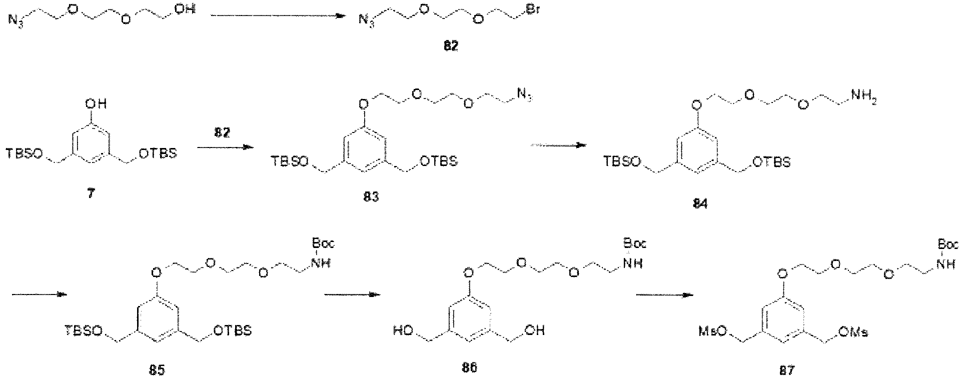

### Preparation of Compound 82

After 2-[2-(2-azidoethoxy)ethoxy]ethanol (21.0 g, 119.8 mmol) was dissolved in tetrahydrofuran (400 mL), methanesulfonic anhydride (31.3 g, 179.7 mmol) and *N-*methyl morpholine (23.7 mL, 215.7 mmol) were added thereto at 0°C, and the mixture was stirred at room temperature in a nitrogen atmosphere for 4 hours. The reaction solution was filtered, distilled under reduced pressure, and then dissolved in tetrahydrofuran (200 mL), then lithium bromide (20.8 g, 239.7 mmol) was added thereto, and the mixture was stirred at 100°C for 12 hours. The reaction solution was cooled to room temperature, diluted with dichloromethane (200 mL), washed with distilled water (500 mL), and then dried over anhydrous sodium sulfate. Filtration, concentration under reduced pressure, and purification by column chromatography were performed to obtain Compound 82 (26.0 g, 91%).

¹H-NMR (400 MHz, CDCl₃) δ 3.84-.3.80 (m, 2H), 3.70 (s, 6H), 3.49-3.47 (m, 2H), 3.47-3.39 (m, 2H).

### Preparation of Compound 83

Compound 82 (2.12 g, 8.90 mmol) and Compound 7 (2.84 g, 7.42 mmol) were dissolved in *N,N*-dimethylformamide (30 mL), then cesium carbonate (3.62 g, 11.13 mmol) was added thereto, and the mixture was stirred at room temperature in a nitrogen atmosphere for 12 hours. The reaction solution was diluted with ethyl acetate (50 mL) and washed with distilled water (80 mL). The washed organic layer was washed with salt water (70 mL) and dried over anhydrous sodium sulfate. Filtration, concentration under reduced pressure, and purification by column chromatography were performed to obtain Compound 83 (26.0 g, 91%).

¹H-NMR (400 MHz, CDCl₃) δ 6.84 (s, 1H), 6.76 (s, 2H), 4.67 (s, 4H), 4.11 (t, J = 4.8 Hz, 2H), 3.85 (t, J = 4.8 Hz, 2H), 3.74-3.71 (m, 2H), 3.68-3.65 (m, 6H), 3.38 (t, J = 4.8 Hz, 2H), 0.87 (s, 18H), 0.07 (s, 12H).

### Preparation of Compound 84

Compound 83 (3.73 g, 6.90 mmol) was dissolved in tetrahydrofuran (20 mL), then distilled water (1.2 mL, 69.0 mmol) and triphenyl phosphine (2.0 g, 7.59 mmol) were added thereto, and the mixture was stirred for 12 hours in a nitrogen atmosphere at room temperature. The reaction solution was dried over anhydrous sodium sulfate, filtered, concentrated under reduced pressure, and then purified by column chromatography to obtain Compound 84 (2.67 g, 75%).

¹H-NMR (400 MHz, CDCl₃) δ 6.86 (s, 1H), 6.77 (s, 2H), 4.69 (s, 4H), 4.13 (t, J = 4.8 Hz, 2H), 3.6 (t, J = 4.8 Hz, 2H), 3.74-3.72 (m, 2H), 3.66-3.64 (m, 2H), 3.54 (t, J = 4.8 Hz, 2H), 2.89 (t, J = 4.8 Hz, 2H), 2.21 (br, 2H), 0.94 (s, 18H), 0.09 (s, 12H). EI-MS m/z : [M+H]⁺ 514.4.

### Preparation of Compound 85

Compound 84 (2.67 g, 5.19 mmol) was dissolved in tetrahydrofuran (30 mL), then triethylamine (0.72 mL, 5.19 mmol) and di-t-butyl dicarbonate (1.1 g, 5.19 mmol) were added thereto at 0°C, and the mixture was stirred for 12 hours in a nitrogen atmosphere at room temperature. The reaction solution was diluted with ethyl acetate (50 mL), washed with distilled water (80 mL), and dried over anhydrous sodium sulfate. Filtration, concentration under reduced pressure, and purification by column chromatography were performed to obtain Compound 85 (2.67 g, 84%).

¹H-NMR (400 MHz, CDCl₃) δ 6.86 (s, 1H), 6.77 (s, 2H), 5.00 (br, 1H), 4.69 (s, 4H), 4.13 (t, *J* = 4.8 Hz, 2H), 3.85 (t, *J* = 4.8 Hz, 2H), 3.73-3.70 (m, 2H), 3.65-3.63 (m, 2H), 3.55 (t, *J* = 4.8 Hz, 2H), 3.32 (q, 2H), 1.46 (s, 9H), 0.93 (s, 18H), 0.09 (s, 12H).

### Preparation of Compound 86

Compound 85 (2.67 g, 4.35 mmol) was dissolved in methanol (50 mL), and then camphorsulfonic acid (404 mg, 1.74 mmol) was added thereto at 0°C in a nitrogen atmosphere. The mixture was stirred at room temperature for 2 hours, triethylamine (0.4 mL) was added thereto, the solvent was concentrated under reduced pressure, and the resultant was purified by column chromatography to obtain Compound 86 (1.2 g, 72%).

¹H-NMR (400 MHz, CDCl₃) δ 6.94 (s, 1H), 6.88 (s, 2H), 5.03 (brs, 1H), 4.67 (s, 2H), 4.66 (s, 2H), 4.17 (t, *J* = 4.8 Hz, 2H), 3.85 (t, *J* = 4.8 Hz, 2H), 3.72-3.70 (m, 2H), 3.70-3.60 (m, 2H), 3.60-3.50 (m, 2H), 3.34-3.28 (m, 2H), 1.92 (brs, 2H), 1.43 (s, 9H).

### Preparation of Compound 87

Compound 86 (1.2 g, 3.11 mmol) was dissolved in dichloromethane (30 mL), then triethylamine (1.3 mL, 9.33 mmol) and methanesulfonyl chloride (0.53 mL, 6.84 mmol) were added thereto at 0°C in a nitrogen atmosphere, and the mixture was stirred for 1 hour. The reaction solution was diluted with dichloromethane (40 mL) and washed with distilled water (50 mL). The washed organic layer was washed with salt water (50 mL) and dried over anhydrous sodium sulfate. Filtration, concentration, and purification by column chromatography were performed to obtain Compound 87 (1.57 g, 93%).

¹H-NMR (400 MHz, CDCl₃) δ 7.04 (s, 1H), 7.00 (s, 2H), 5.20 (s, 4H), 4.98 (brs, 1H), 4.17 (t, *J* = 4.8 Hz, 2H), 3.87 (t, *J* = 4.8 Hz, 2H), 3.73-3.71 (m, 2H), 3.66-3.64 (m, 2H), 3.55 (t, *J* = 5.2 Hz, 2H), 3.36-3.25 (m, 2H), 2.99 (s, 6H), 1.43 (s, 9H).

### <Example 26> Preparation of Compound 90

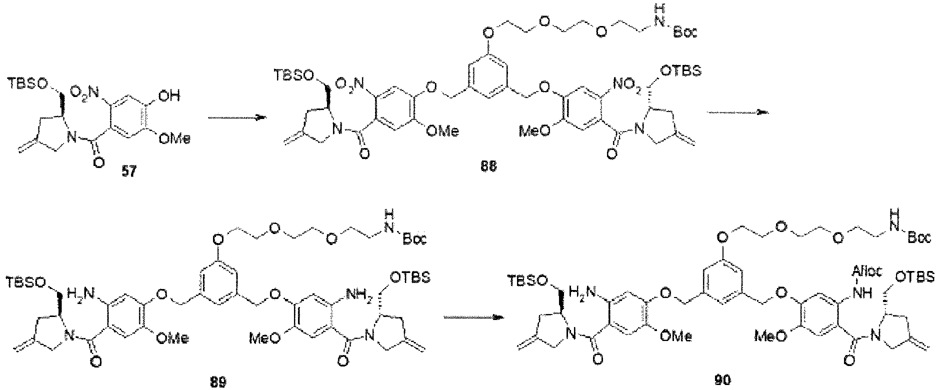

### Preparation of Compound 88

Compound 87 (1.57 g, 2.89 mmol) and Compound 57 (2.69 g, 6.36 mmol) were dissolved in *N,N-*dimethylformamide (30 mL), then potassium carbonate (2 g, 14.45 mmol) was added thereto at 0°C, and the mixture was stirred at room temperature for 20 hours in a nitrogen atmosphere. The reaction solution was diluted with ethyl acetate (100 mL) and washed with distilled water (3 × 80 mL). The washed organic layer was washed with salt water (100 mL) and dried over anhydrous sodium sulfate. Filtration, concentration under reduced pressure, and purification by column chromatography were performed to obtain Compound 88 (2.73 g, 79%).

### Preparation of Compound 89

Compound 88 (2.1 g, 1.76 mmol) was dissolved in ethyl acetate (10 mL) and ethanol (10 mL), then zinc dust (6.9 g, 105.60 mmol) was added thereto, and formic acid (2.66 mL, 70.4 mmol) diluted with ethanol (10 mL) was added thereto. The mixture was stirred at room temperature for 3 hours, then diluted with ethyl acetate (30 mL), and filtered through Celite, and ethyl acetate (100 mL) was added to the filtered solution. The organic layer was washed with distilled water (150 mL), saturated aqueous sodium hydrogen carbonate solution (150 mL), and salt water (100 mL) in this order, and then dried over anhydrous sodium sulfate. Filtration, concentration, and purification by column chromatography were performed to obtain Compound 89 (crude 1.99 g, 100%).

### Preparation of Compound 90

Compound 89 (2.8 g, 2.46 mmol) was dissolved in dichloromethane (200 mL), pyridine (0.39 mL, 4.93 mmol) was added thereto, allyl chloroformate (0.23 mL, 2.22 mmol) diluted with dichloromethane (50 mL) was added thereto at -78°C in a nitrogen atmosphere, and the reaction solution was stirred for 3 hours. The reaction solution was concentrated under reduced pressure and then purified by column chromatography to obtain Compound 90 (1.5 g, 50%).

EI-MS m/z: [M+H]⁺ 1219.3.

### <Example 27> Preparation of Compound 93

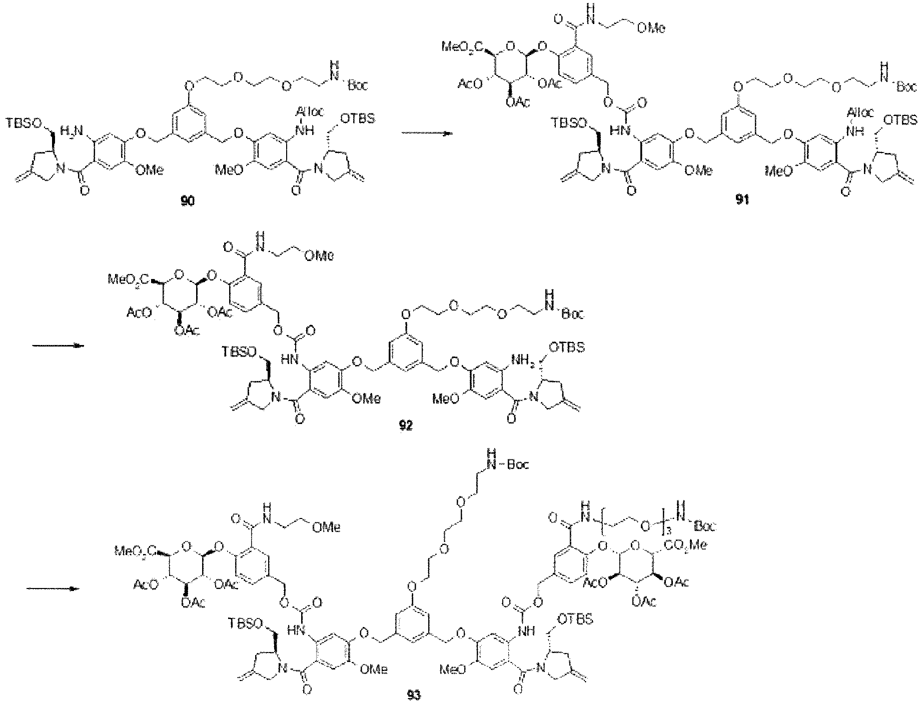

### Preparation of Compound 91

Compound 90 (1.5 g, 1.23 mmol) and triphosgene (131 mg, 0.44 mmol) were dissolved in dry dichloromethane (60 mL), then *N*-methylimidazole (0.49 mL, 6.15 mmol) was added thereto at -10°C, and the mixture was stirred in a nitrogen atmosphere for 1 hour. Compound 20 (799 mg, 1.47 mmol) was dissolved in dry dichloromethane (20 mL), *N-*methylimidazole (0.12 mL, 1.47 mmol) was added thereto, then this solution was gradually added to the reaction solution, and the mixture was stirred at room temperature for 17 hours. The reaction solution was diluted with dichloromethane (20 mL), washed with distilled water (30 mL), and dried over anhydrous sodium sulfate. Filtration, concentration under reduced pressure, and purification by column chromatography were performed to obtain Compound 91 (1.83 g, 82%).

EI-MS m/z: [M+H]⁺ 1786.3, 1/2[M+H]⁺ 893.6.

### Preparation of Compound 92

Compound 91 (1.83 g, 1.02 mmol) was dissolved in dichloromethane (80 mL), then pyrrolidine (0.17 mL, 2.05 mmol) and tetrakis(triphenylphosphine)palladium(0) (23.6 mg, 0.020 mmol) were added thereto, and the mixture was stirred at room temperature in a nitrogen atmosphere for 1 hour. The reaction solution was concentrated under reduced pressure and then purified by column chromatography to obtain Compound 92 (1.26 g, 72%).

EI-MS m/z : [M+H]⁺ 1702.5, 1/2[M+H]⁺ 851.6.

### Preparation of Compound 93

Compound 92 (1.26 g, 0.74 mmol) and triphosgene (79 mg, 0.27 mmol) were dissolved in dry dichloromethane (30 mL), then *N*-methylimidazole (0.29 mL, 3.70 mmol) was added thereto at -10°C, and the mixture was stirred in a nitrogen atmosphere for 1 hour. Compound 23 (649 mg, 0.88 mmol) was dissolved in dry dichloromethane (10 mL), *N-*methylimidazole (0.07 mL, 0.88 mmol) was added thereto, then this solution was gradually added to the reaction solution, and the mixture was stirred at room temperature for 17 hours. The reaction solution was diluted with dichloromethane (20 mL), washed with distilled water (30 mL), and dried over anhydrous sodium sulfate. Filtration, concentration under reduced pressure, and purification by column chromatography were performed to obtain Compound 93 (910 mg, 50 %).

EI-MS m/z: [M+H]⁺ 2458.9, 1/2[M+H]⁺ 1229.9.

### <Example 28> Preparation of Compound 97

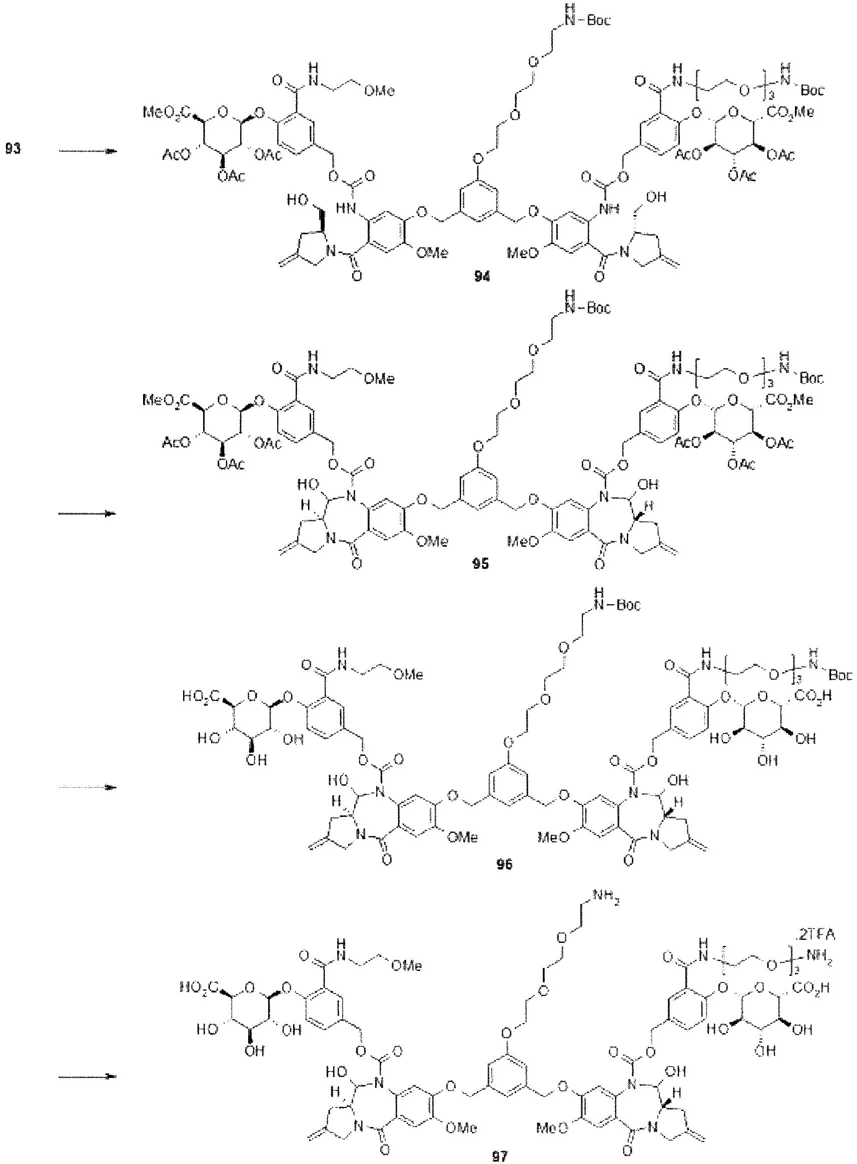

### Preparation of Compound 94

Compound 93 (910 mg, 0.37 mmol) was dissolved in tetrahydrofuran/distilled water (10 mL/10 mL), acetic acid (20 mL) was added thereto, and then the mixture was stirred at room temperature in a nitrogen atmosphere for 16 hours. The reaction solution was diluted with ethyl acetate (60 mL), washed with distilled water (50 mL) and saturated sodium carbonate solution (2 × 50 mL), and then dried over anhydrous sodium sulfate. Filtration, concentration under reduced pressure, and purification by column chromatography were performed to obtain Compound 94 (620 mg, 72%).

EI-MS m/z: [M+H]⁺ 2230.4, [M+Na]⁺ 2252.2.

### Preparation of Compound 95

Compound 94 (620 mg, 0.27 mmol) was dissolved in dichloromethane (25 mL), then Dess-Martin periodinane (235 mg, 0.55 mmol) was added thereto, and the mixture was stirred at room temperature in a nitrogen atmosphere for 90 minutes. The reaction solution was diluted with dichloromethane (20 mL), washed with saturated aqueous sodium hydrogen carbonate solution (30 mL), and dried over anhydrous sodium sulfate. Filtration, concentration under reduced pressure, and purification by column chromatography were performed to obtain Compound 95 (380 mg, 62%).

EI-MS m/z: [M+H]⁺ 2225.8, [M+Na]⁺ 2248.0.

### Preparation of Compound 96

Compound 95 (380 mg, 0.17 mmol) was dissolved in methanol (10 mL), then a solution prepared by dissolving lithium hydroxide (64 mg, 1.53 mmol) in distilled water (10 mL) was gradually added thereto at -50°C, and the mixture was stirred at room temperature for 3 hours. The reaction solution was neutralized with formic acid, concentrated under reduced pressure, then purified by HPLC, and freeze-dried to obtain Compound 96 (50 mg, 14%).

EI-MS m/z: [M+H]⁺ 1946.0, [M+Na]⁺ 1969.1.

### Preparation of Compound 97

Compound 96 (50 mg, 0.025 mmol) was diluted with dichloromethane (4 mL), then trifluoroacetic acid (1 mL) was added thereto at 0°C, and the mixture was stirred for 1 hour. The reaction solution was concentrated under reduced pressure, then purified by HPLC, and freeze-dried to obtain Compound 97 as a white solid (27 mg, 52%).

EI-MS m/z : [M+H]⁺ 1745.4, 1/2[M+H]⁺ 874.1.

### <Example 29> Preparation of Compound 100

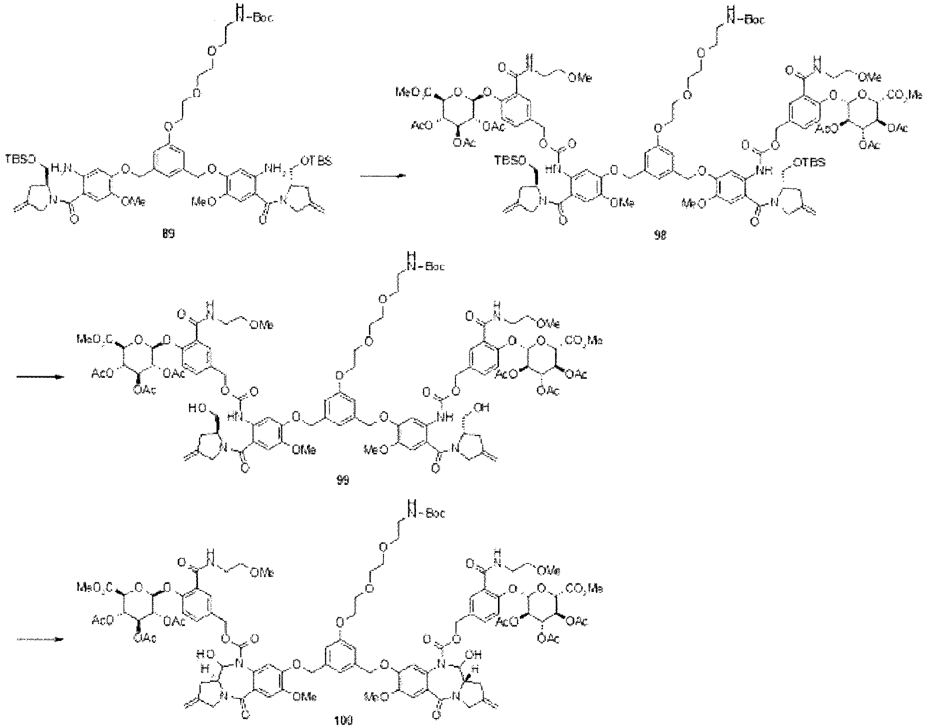

### Preparation of Compound 98

Compound 89 (1.2 g, 1.06 mmol) and triphosgene (226 mg, 0.76 mmol) were dissolved in dry dichloromethane (50 mL), *N*-methylimidazole (0.58 mL, 10.60 mmol) was added thereto at -10°C, and the mixture was stirred in a nitrogen atmosphere for 1 hour. Compound 20 (1.26 g, 2.33 mmol) was dissolved in dry dichloromethane (20 mL), *N-*methylimidazole (0.17 mL, 2.33 mmol) was added thereto, this solution was gradually added to the reaction solution, and the mixture was stirred at room temperature for 17 hours. The reaction solution was diluted with dichloromethane (20 mL), washed with distilled water (30 mL), and then dried over anhydrous sodium sulfate. Filtration, concentration under reduced pressure, and purification by column chromatography were performed to obtain Compound 98 (1.41 g, 58%).

EI-MS m/z: [M+H]⁺ 2270.2, 1/2[M+H]⁺ 1135.7.

### Preparation of Compound 99

Compound 98 (1.41 g, 0.62 mmol) was dissolved in tetrahydrofuran/distilled water (10 mL/10 mL), acetic acid (20 mL) was added thereto, and the mixture was stirred at room temperature in a nitrogen atmosphere for 16 hours. The reaction solution was concentrated under reduced pressure and then purified by column chromatography to obtain Compound 99 (800 mg, 63%).

EI-MS m/z : [M+H]⁺ 2042.8, [M+Na]⁺ 2064.6.

### Preparation of Compound 100

Compound 99 (740 mg, 0.36 mmol) was dissolved in dichloromethane (40 mL), then Dess-Martin periodinane (352 mg, 0.83 mmol) was added thereto, and the mixture was stirred at room temperature in a nitrogen atmosphere for 3 hours. The reaction solution was diluted with dichloromethane (20 mL), washed with saturated aqueous sodium hydrogen carbonate solution (30 mL), and then dried over anhydrous sodium sulfate. Filtration, concentration under reduced pressure, and purification by column chromatography were performed to obtain Compound 100 (626 mg, 84%).

EI-MS m/z: [M+H]⁺ 2038.3 1/2[M+H]⁺ 1019.6.

### <Example 30> Preparation of Compound 102

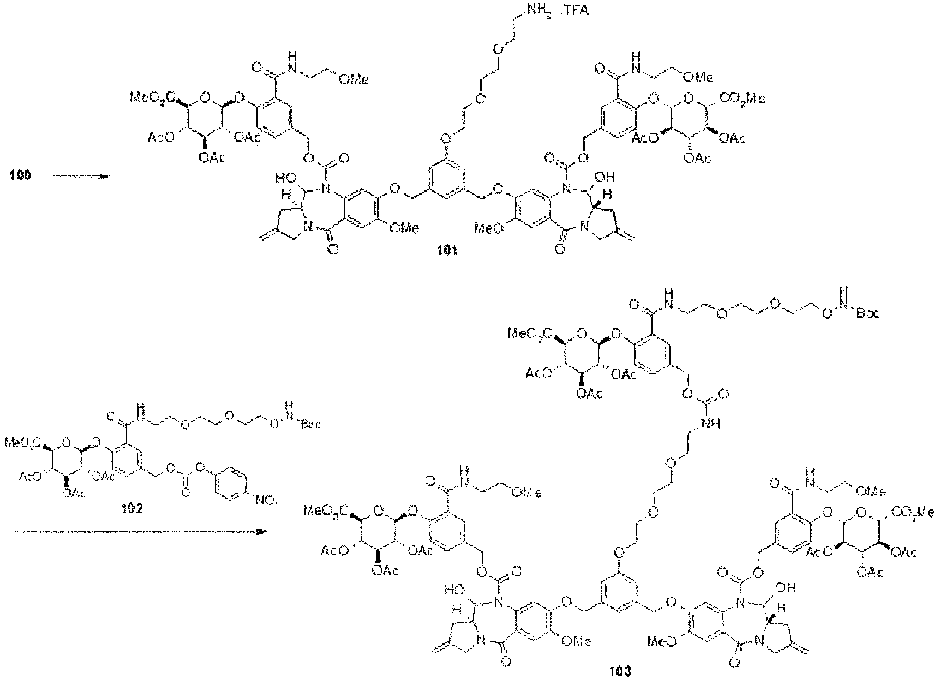

### Preparation of Compound 101

Compound 100 (440 mg, 0.216 mmol) was diluted with dichloromethane (4 mL), then trifluoroacetic acid (1 mL) was added thereto at 0°C, and the mixture was stirred for 1 hour. The reaction solution was concentrated under reduced pressure to obtain Compound 101 (crude 443 mg, 100%).

EI-MS m/z : [M+H]⁺ 1937.7, 1/2[M+H]⁺ 969.7.

### Preparation of Compound 103

Compound 101 (443 mg, 0.216 mmol) and Compound 102 (290 mg, 0.324 mmol, Compound 102 was prepared by the method described in Korean Patent Publication No. 10-2018-0078329) were dissolved in N,N-dimethylformamide (10 mL), *N,N*-diisopropylethylamine (0.11 mL, 0.648 mmol) was added thereto at 0°C, and the mixture was stirred at room temperature for 2 hours in a nitrogen atmosphere. N-methylimidazole (0.02 mL, 0.432 mmol) and Compound 102 (398 mg, 0.44 mmol) were further added to the reaction solution, and the mixture was stirred for 16 hours. The reaction solution was diluted with ethyl acetate (70 mL) and washed with distilled water (3 × 50 mL), and then the collected organic layer was washed with salt water (70 mL) and dried over anhydrous sodium sulfate. Filtration, concentration under reduced pressure, and purification by column chromatography were performed to obtain Compound 103 (340 mg, 56%).

EI-MS m/z: [M+H]⁺ 2694.3, 1/2[M+H]⁺ 1347.9.

### <Example 31> Preparation of Compound 105

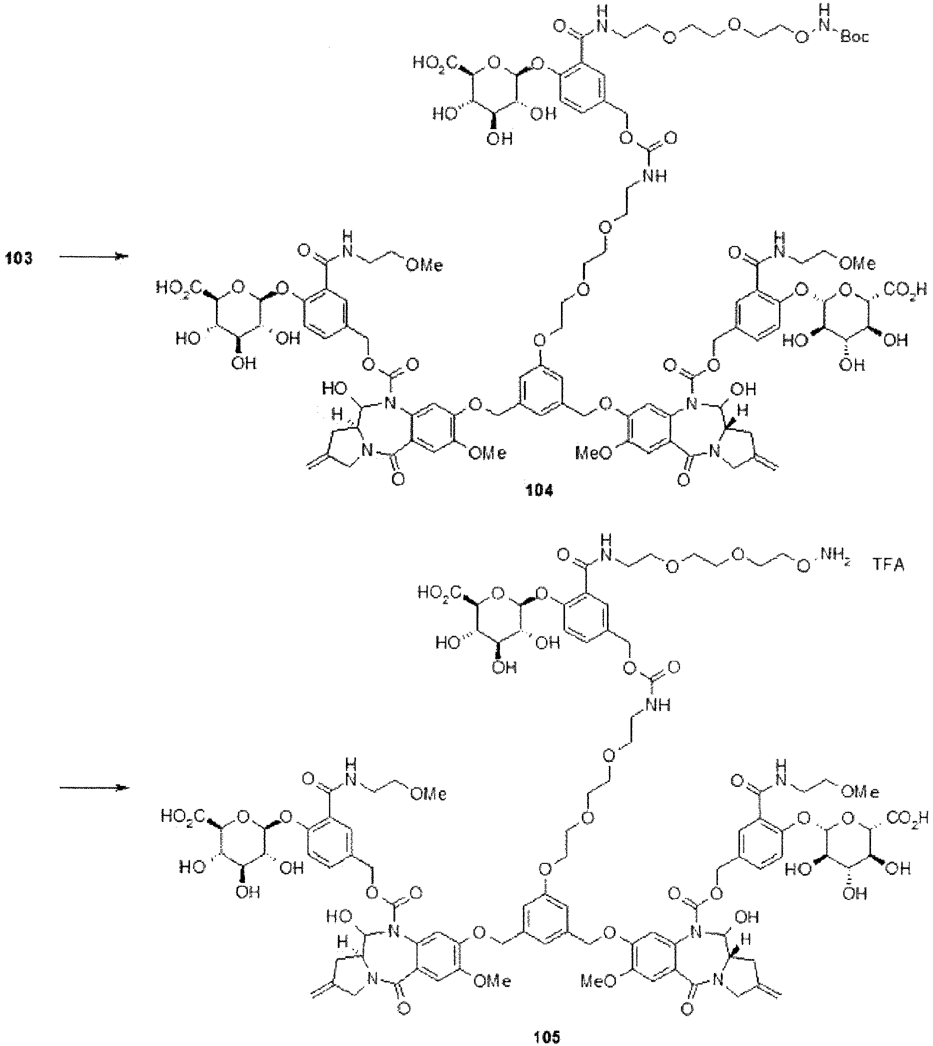

### Preparation of Compound 104

Compound 103 (340 mg, 0.126 mmol) was dissolved in methanol (9 mL), then a solution prepared by dissolving lithium hydroxide (53 mg, 1.26 mmol) in distilled water (9 mL) was gradually added thereto at -50°C, and the mixture was stirred at room temperature for 4 hours. The reaction solution was neutralized with acetic acid, and the reaction solution was concentrated under reduced pressure, then purified by HPLC, and freeze-dried to obtain Compound 104 (117 mg, 44%).

EI-MS m/z: [M+H]⁺ 2274.4, 1/2[M+H]⁺ 1137.4.

### Preparation of Compound 105

Compound 104 (117 mg, 0.05 mmol) was diluted with dichloromethane (8 mL), then trifluoroacetic acid (2 mL) was added thereto at 0°C, and the mixture was stirred for 1 hour. The reaction solution was concentrated under reduced pressure, then purified by HPLC, and freeze-dried to obtain Compound 105 as a white solid (49.7 mg, 43%).

EI-MS m/z: [M+H]⁺ 2173.9, 1/2[M+H]⁺ 1087.4.

### <Example 32> Preparation of Compound 110

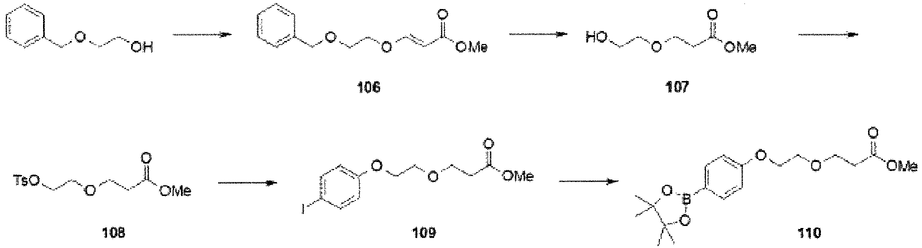

### Preparation of Compound 106

After 2-(benzyloxy)ethanol (5 g, 32.85 mmol) was dissolved in dichloromethane (100 mL), N-methylmorpholine (4 mL, 36.14 mmol) and methyl propiolate (3.2 mL, 36.14 mmol) were stirred at room temperature for 16 hours. The reaction solution was concentrated under reduced pressure, diluted with ethyl acetate (100 mL), and then washed with distilled water (70 mL). The organic layer was dried over anhydrous sodium sulfate, filtered, concentrated under reduced pressure, and then purified by column chromatography to obtain Compound 106 (6.90 g, 89%).

¹H-NMR (400 MHz, CDCl₃) δ 7.63 (d, *J* = 12.4 Hz, 1H), 7.38-7.29 (m, 5H), 5.23 (d, *J* = 12.8 Hz, 1H), 4.58 (s, 2H), 4.02 (t, *J* = 4.4 Hz, 2H), 3.73 (t, *J* = 3.2 Hz, 2H), 3.70 (s, 3H).

### Preparation of Compound 107

Compound 106 (6.9 g, 29.20 mmol) was dissolved in methanol (80 mL), and then palladium/charcoal (10% w/w, 3 g) was added thereto. The reaction solution was stirred at room temperature in a hydrogen atmosphere for 16 hours. The reaction solution was filtered through Celite and then concentrated to obtain Compound 107 (4.01 g, 92%).

¹H-NMR (400 MHz, CDCl₃) δ 3.78 (t, *J* = 6.0 Hz, 2H), 3.75-3.70 (m, 5H), 3.59 (t, *J* = 4.8 Hz, 2H), 2.61 (t, *J* = 6.0 Hz, 2H).

### Preparation of Compound 108

Compound 107 (2 g, 13.5 mmol) was dissolved in dichloromethane (100 mL), and then triethylamine (2.8 mL, 20.2 mmol) and 4-toluenesulfonyl chloride (3.09 g, 16.2 mmol) were added thereto at 0°C in a nitrogen atmosphere. The reaction solution was stirred at room temperature for 16 hours, diluted with dichloromethane (30 mL), and washed with distilled water (80 mL). The organic layer was dried over anhydrous sodium sulfate, filtered, concentrated under reduced pressure, and then purified by column chromatography to obtain Compound 108 (4.59 g, 56%).

¹H-NMR (400 MHz, CDCl₃) δ 7.80 (d, *J* = 8.8 Hz, 2H), 7.34 (d, *J* = 8.4 Hz, 2H), 4.14 (t, *J* = 4.4 Hz, 2H), 3.68 (t, *J* = 6.8 Hz, 2H), 3.68 (s, 3H), 3.65 (t, *J* = 4.4 Hz, 2H), 2.52 (t, *J* = 6.0 Hz, 2H), 2.45 (s, 3H).

### Preparation of Compound 109

Compound 108 (2.09 g, 6.91 mmol) and 4-iodophenol (1.82 g, 8.29 mmol) were dissolved in *N,N-*dimethylformamide (20 mL), then potassium carbonate (1.43 g, 10.36 mmol) and sodium iodide (207 mg, 1.38 mmol) were added thereto at 0°C, and the mixture was stirred at 70°C for 2 hours. The reaction solution was diluted with ethyl acetate (70 mL) and washed with distilled water (3 × 50 mL). The organic layer was dried over anhydrous sodium sulfate, filtered, concentrated under reduced pressure, and then purified by column chromatography to obtain Compound 109 (1.73 g, 71%).

¹H-NMR (400 MHz, CDCl₃) δ 7.54 (d, *J* = 8.8 Hz, 2H), 6.69 (d, *J* = 8.4 Hz, 2H), 4.07 (t, *J* = 4.8 Hz, 2H), 3.84-3.78 (m, 4H), 3.69 (s, 3H), 2.62 (t, *J* = 6.8 Hz, 2H).

### Preparation of Compound 110

Compound 109 (930 mg, 4.94 mmol), bis(pinacolato)diboron (1.01 g, 3.98 mmol), [1,1'-bis(diphenylphosphino)ferrocene]palladium(II) dichloride (0.43 g, 0.53 mmol), and potassium acetate (0.78 g, 7.95 mmol) were added to N,N-dimethylformamide (20 mL), and the mixture was stirred at 90°C in a nitrogen atmosphere for 16 hours. The reaction solution was diluted with ethyl acetate (70 mL) and washed with distilled water (3 × 50 mL). The organic layer was dried over anhydrous sodium sulfate, filtered, concentrated under reduced pressure, and then purified by column chromatography to obtain Compound 110 (685 mg, 74%).

¹H-NMR (400 MHz, CDC13) δ 7.73 (d, *J* = 8.4 Hz, 2H), 6.90 (d, *J* = 8.8 Hz, 2H), 4.13 (t, *J* = 4.8 Hz, 2H), 3.85-3.82 (m, 4H), 3.68 (s, 3H), 2.63 (t, *J* = 6.4 Hz, 2H), 1.33 (s, 12H).

### <Example 33> Preparation of Compound 117

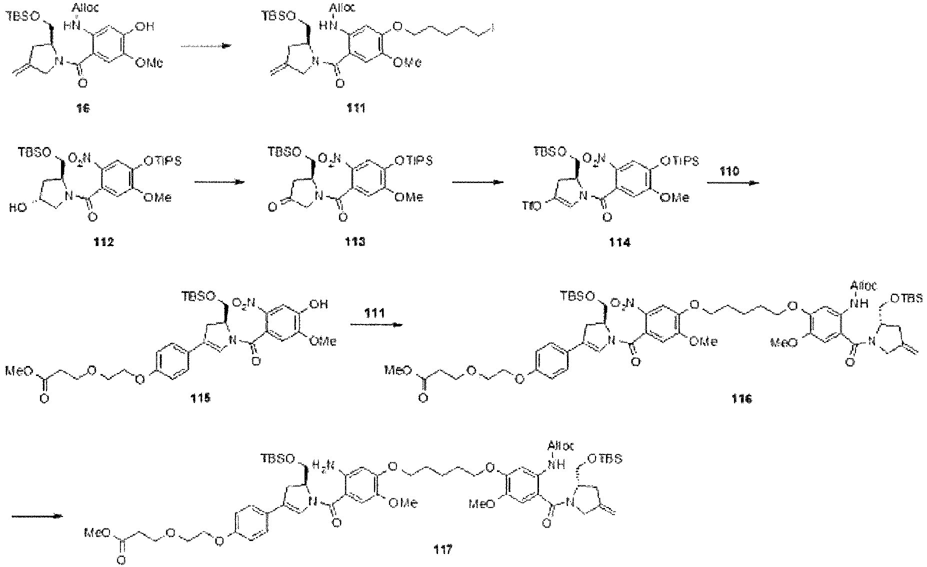

### Preparation of Compound 111

Compound 16 (7.9 g, 16.5 mmol) and diiodopentane (12.3 mL, 82.8 mmol) were dissolved in acetone (100 mL), then potassium carbonate (2.3 g, 16.5 mmol) was added thereto, and the mixture was stirred at 60°C for 10 hours. The reaction solution was concentrated under reduced pressure and then purified by column chromatography to obtain Compound 111 (9.7 g, 87%).

¹H-NMR (400 MHz, CDCl₃) δ 8.88 (brs 1H), 7.83 (s, 1H), 6.82 (s, 1H), 5.99-5.92 (m, 1H), 5.35 (d, *J* = 17.2 Hz, 1H), 5.24 (d, *J* = 10.4 Hz, 1H), 5.14-4.84 (m, 2H), 4.69-4.54 (m, 2H), 4.24-4.14 (m, 2H), 4.09 (t, *J* = 6.8 Hz, 2H), 3.91-3.42 (m, 5H), 3.22 (t, *J* = 7.3 Hz, 2H), 2.76-2.61 (m, 2H), 1.95-1.84 (m, 4H), 1.62-1.55 (m, 2H), 0.89 (s, 9H), 0.03 (s, 6H).

### Preparation of Compound 113

Compound 112 (5.3 g, 9.09 mmol, Compound 112 was prepared by the method described in *ACS. Med. Chem.* 2016, 7, 11, 983-987) was dissolved in dichloromethane (50 mL), then trichloroisocyanuric acid (1.48 g, 6.36 mmol) and TEMPO (142 mg, 0.91 mmol) were added thereto at -10°C, and the mixture was stirred for 1 hour. The reaction solution was diluted with dichloromethane (50 mL), washed with saturated aqueous sodium hydrogen carbonate (2 × 70 mL) and salt water (100 mL), and dried over anhydrous sodium sulfate. Filtration and concentration were performed to obtain Compound 113 (5.79 g, 100%).

¹H-NMR (400 MHz, CDCl₃) (rotamers) δ 7.71 (s, 1H), 6.73 (s, 1H), 5.01-4.99 (m, 1H), 4.32-4.29 (m, 1H), 3.91 (s, 3H), 3.73 (dd, 1H), 3.66 (d, *J* = 17.2 Hz, 1H), 3.46 (d, *J* = 16.8 Hz, 1H), 2.83-2.76 (m, 1H), 2.59-2.54 (m, 1H), 1.36-1.24 (m, 3H), 1.12 (d, *J* = 10.4 Hz, 18H), 0.89 (s, 9H), 0.11 (s, 3H), 0.09 (s, 3H).

### Preparation of Compound 114

Compound 113 (3.79 g, 6.51 mmol) was dissolved in dichloromethane (60 mL), and then 2,6-lutidine (3 mL, 26.0 mmol) and trifluoromethanesulfonic anhydride (3.3 mL, 19.5 mmol) were added thereto at -55°C in a nitrogen atmosphere. The reaction solution was stirred for 30 minutes, then diluted with dichloromethane (30 mL), and washed with saturated aqueous sodium hydrogen carbonate solution (100 mL). The organic layer was dried over anhydrous sodium sulfate, filtered, concentrated under reduced pressure, and then purified by column chromatography to obtain Compound 114 (2.95 g, 63%).

### Preparation of Compound 115

Compound 114 (2.95 g, 4.13 mmol) was dissolved in ethanol/toluene/distilled water (7 mL/14 mL/7 mL), and then Compound 110 (1.69 g, 4.82 mmol), sodium carbonate (1.42 g, 13.44 mmol), and tetrakis(triphenylphosphine)palladium(0) (0.52 g, 0.45 mmol) were added thereto at room temperature in a nitrogen atmosphere. The reaction solution was stirred at 85°C for 3 hours and then diluted with ethyl acetate (100 mL), and the organic layer was washed with salt water (100 mL) and distilled water (100 mL). The collected organic layer was dried over anhydrous sodium sulfate, filtered, and then concentrated under reduced pressure. The obtained compound was dissolved in *N,N-*dimethylformamide/distilled water (10 mL/0.2 mL), and sodium acetate (67 mg, 0.81 mmol) was added thereto, and the mixture was stirred at room temperature in a nitrogen atmosphere for 3 hours. The reaction solution was diluted with ethyl acetate (50 mL) and washed with distilled water (3 × 40 mL), and the organic layer was dried over anhydrous sodium sulfate. Filtration, concentration under reduced pressure, and purification by column chromatography were performed to obtain Compound 115 (1.10 g, 41%).

¹H-NMR (400 MHz, CDCl₃) δ 7.81 (s, 1H), 7.126 (d, *J* = 8.8 Hz, 2H), 6.91 (s, 1H), 6.81 (d, *J* = 8.8 Hz, 2H), 6.15 (s, 1H), 5.98 (s, 1H), 4.79 (m, 1H), 4.08 (t, *J* = 4.8 Hz, 2H), 4.01 (s, 3H), 3.88-3.78 (m, 4H), 3.68 (s, 3H), 3.24-3.12 (m 1H), 3.04-2.96 (m, 1H), 2.62 (t, *J* = 6.4 Hz, 2H), 0.88 (s, 9H), 0.15 (s, 6H).

### Preparation of Compound 116

Compound 115 (1.10 g, 1.74 mmol) and Compound 111 (1.28 g, 1.91 mmol) were dissolved in *N,N-*dimethylformamide (12 mL), then potassium carbonate (0.29 g, 2.08 mmol) was added thereto at 0°C, and the mixture was stirred at room temperature for 20 hours in a nitrogen atmosphere. The reaction solution was diluted with ethyl acetate (100 mL) and washed with distilled water (3 × 100 mL). The washed organic layer was washed with salt water (100 mL) and dried over anhydrous sodium sulfate. Filtration, concentration under reduced pressure, and purification by column chromatography were performed to obtain Compound 116 (1.34 g, 65%).

EI-MS m/z: [M+H]⁺ 1176.1.

### Preparation of Compound 117

Compound 116 (1.34 g, 1.14 mmol) was dissolved in ethyl acetate (6 mL) and ethanol (6 mL), then zinc dust (1.49 g, 22.8 mmol) was added thereto, and formic acid (0.86 mL, 22.8 mmol) diluted with ethanol (6 mL) was added thereto. The mixture was stirred at room temperature for 3 hours, then diluted with ethyl acetate (50 mL), and filtered through Celite, and ethyl acetate (100 mL) was added to the filtered solution. The organic layer was washed with distilled water (150 mL), saturated aqueous sodium hydrogen carbonate solution (150 mL), and salt water (100 mL) in this order, and then dried over anhydrous sodium sulfate. Filtration, concentration, and purification by column chromatography were performed to obtain Compound 117 (1.21 g, 93%).

EI-MS m/z: [M+H]⁺ 1146.5, 1/2[M+H]⁺ 573.4.

### <Example 34> Preparation of Compound 120

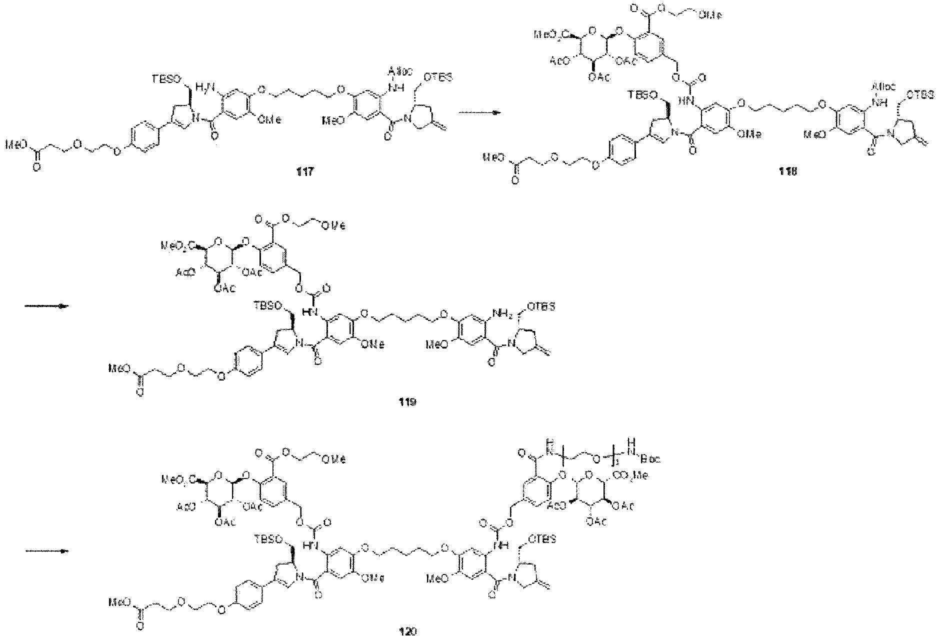

### Preparation of Compound 118

Compound 117 (700 mg, 0.61 mmol) and triphosgene (65 mg, 0.22 mmol) were dissolved in dry dichloromethane (30 mL), then N-methylimidazole (0.17 mL, 3.05 mmol) was added thereto at -10°C, and the mixture was stirred in a nitrogen atmosphere for 1 hour. Compound 20 (376 mg, 0.73 mmol) was dissolved in dry dichloromethane (10 mL), N-methylimidazole (0.04 mL, 0.73 mmol) was added thereto, then this solution was gradually added to the reaction solution, and the mixture was stirred at room temperature for 17 hours. The reaction solution was diluted with dichloromethane (20 mL), washed with distilled water (30 mL), and then dried over anhydrous sodium sulfate. Filtration, concentration under reduced pressure, and purification by column chromatography were performed to obtain Compound 118 (0.72 g, 69%).

EI-MS m/z: [M+H]⁺ 1713.1, 1/2[M+H]⁺ 857.0.

### Preparation of Compound 119

Compound 118 (720 mg, 0.42 mmol) was dissolved in dichloromethane (40 mL), then sodium 2-ethylhexanoate (105 mg, 0.63 mmol) and tetrakis(triphenylphosphine)palladium(0) (24 mg, 0.02 mmol) were added thereto, and the mixture was stirred at room temperature in a nitrogen atmosphere for 2 hours. The reaction solution was concentrated under reduced pressure and then purified by column chromatography to obtain Compound 119 (586 mg, 85%).

### Preparation of Compound 120

Compound 119 (586 mg, 0.36 mmol) and triphosgene (38 mg, 0.13 mmol) were dissolved in dry dichloromethane (15 mL), then N-methylimidazole (0.1 mL, 1.80 mmol) was added thereto at -10°C, and the mixture was stirred in a nitrogen atmosphere for 1 hour. Compound 23 (315 mg, 0.43 mmol) was dissolved in dry dichloromethane (5 mL), N-methylimidazole (0.02 mL, 0.43 mmol) was added thereto, then this solution was gradually added to the reaction solution, and the mixture was stirred at room temperature for 17 hours. The reaction solution was diluted with dichloromethane (15 mL), washed with distilled water (20 mL), and then dried over anhydrous sodium sulfate. Filtration, concentration under reduced pressure, and purification by column chromatography were performed to obtain Compound 120 (516 mg, 60%).

EI-MS m/z: [M+H]⁺ 2385.5, 1/2[M+H]⁺ 1193.2.

### <Example 35> Preparation of Compound 122

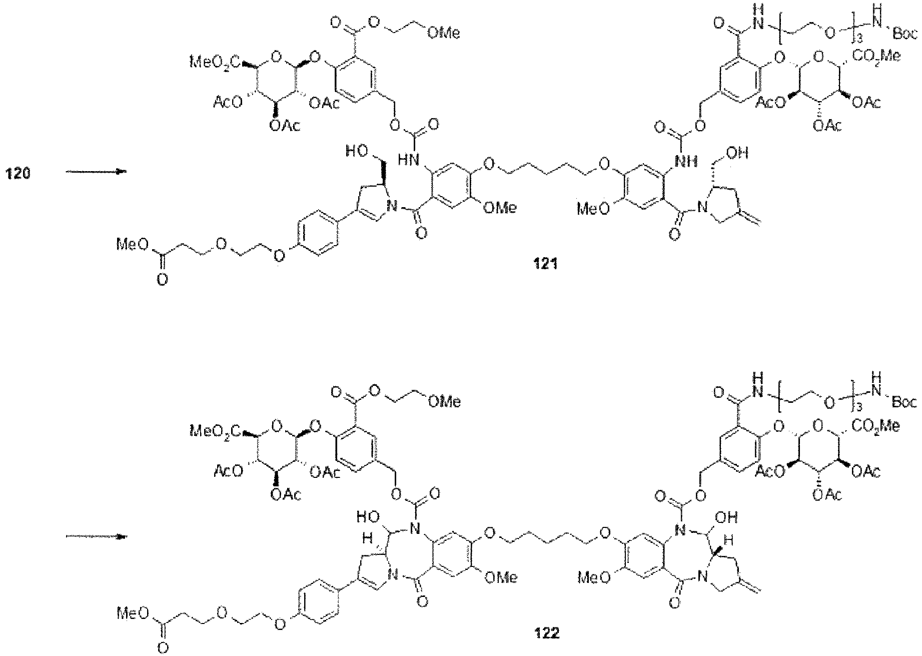

### Preparation of Compound 121

Compound 120 (516 mg, 0.216 mmol) was dissolved in tetrahydrofuran/distilled water (3 mL/3 mL), then acetic acid (7 mL) was added thereto, and the mixture was stirred at room temperature in a nitrogen atmosphere for 16 hours. The reaction solution was concentrated under reduced pressure and then purified by column chromatography to obtain Compound 121 (383 mg, 82%).

EI-MS m/z : [M+H]⁺ 2157.9, [M+Na]⁺ 2179.1.

### Preparation of Compound 122

Compound 121 (383 mg, 0.18 mmol) was dissolved in dichloromethane (10 mL), then Dess-Martin periodinane (173 mg, 0.41 mmol) was added thereto, and the mixture was stirred at room temperature in a nitrogen atmosphere for 4 hours. The reaction solution was diluted with dichloromethane (30 mL), washed with saturated aqueous sodium hydrogen carbonate solution (30 mL), and dried over anhydrous sodium sulfate. Filtration, concentration under reduced pressure, and purification by column chromatography were performed to obtain Compound 122 (200 mg, 52%).

### <Example 36> Preparation of Compound 124

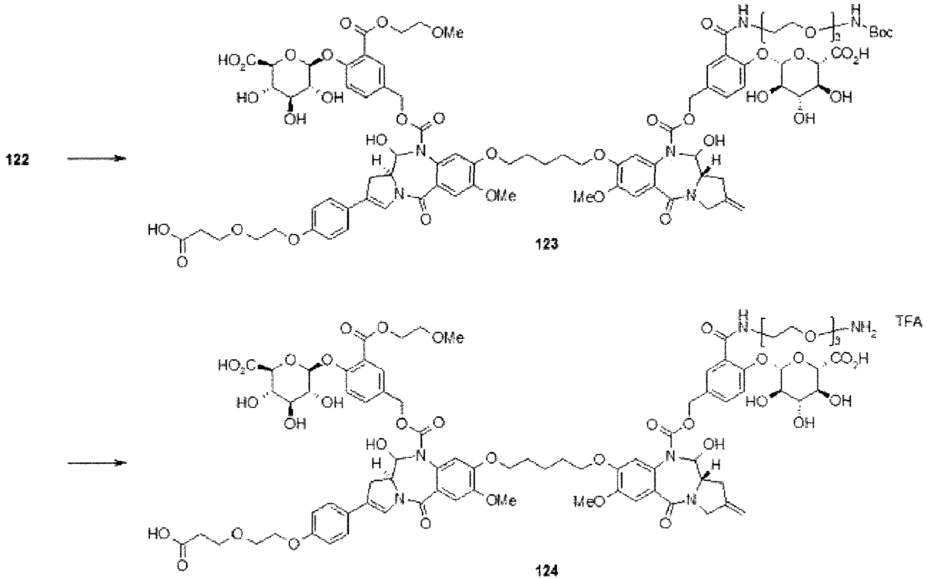

### Preparation of Compound 123

Compound 122 (200 mg, 0.093 mmol) was dissolved in methanol (6 mL), then a solution prepared by dissolving lithium hydroxide (35 mg, 0.835 mmol) in distilled water (6 mL) was gradually added thereto at -50°C, and the mixture was stirred at room temperature for 3 hours. The reaction solution was neutralized with formic acid, concentrated under reduced pressure, then purified by HPLC, and freeze-dried to obtain Compound 123 (95 mg, 55%) .

EI-MS m/z : [M+H]⁺ 1858.9, [M+Na]⁺ 1880.3.

### Preparation of Compound 124

Compound 123 (90 mg, 0.051 mmol) was diluted with dichloromethane (4 mL), then trifluoroacetic acid (1 mL) was added thereto at 0°C, and the mixture was stirred for 2 hours. The reaction solution was concentrated under reduced pressure, then purified by HPLC, and freeze-dried to obtain Compound 124 as a white solid (34 mg, 35.7%).

EI-MS m/z: [M+H]⁺ 1758.9, 1/2[M+H]⁺ 879.9

### <Example 37> Preparation of Compound 130

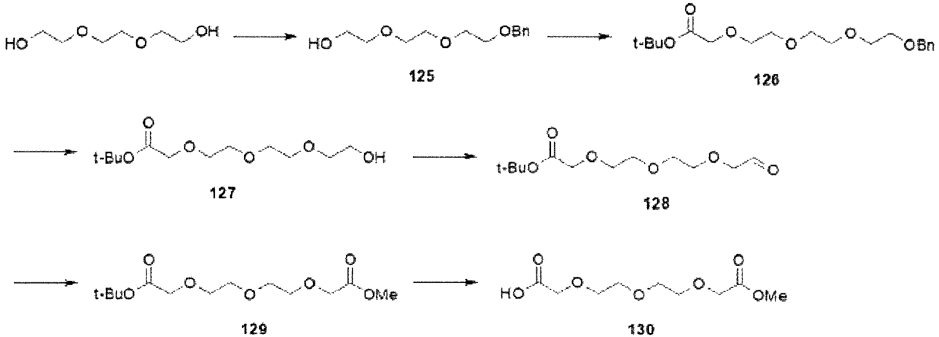

### Preparation of Compound 125

Triethylene glycol (10.0 g, 66.6 mmol) was dissolved in dichloromethane (60 mL), and then silver(I) oxide (23.1 g, 73.3 mmol) and benzyl bromide (8.7 mL, 99.9 mmol) were sequentially added thereto at 0°C in a nitrogen atmosphere. The reaction solution was heated to room temperature, stirred for 20 hours, and then filtered through Celite. The filtered solution was concentrated and then purified by column chromatography to obtain Compound 125 (11.2 g, 70%).

¹H-NMR (400 MHz, CDCl₃) δ 7.24-7.36 (m, 5H), 4.57 (s, 2H), 3.76-3.56 (m, 12H), 2.60 (br s, 1H).

### Preparation of Compound 126

Sodium hydride (1.3 g, 31.9 mmol) was added to tetrahydrofuran (250 mL) at 0°C, then Compound 125 (5.9 g, 24.6 mmol) diluted with tetrahydrofuran (50 mL) was gradually added to the reaction solution, and the mixture was stirred at 0°C in a nitrogen atmosphere for 1 hour. Thereafter, t-butyl bromoacetate (4.7 mL, 24.6 mmol) was added thereto, and the mixture was stirred at room temperature for 18 hours. The reaction solution was diluted with ethyl acetate (200 mL), washed with saturated aqueous ammonium chloride solution (100 mL), and then dried over anhydrous sodium sulfate. Filtration, concentration under reduced pressure, and purification by column chromatography were performed to obtain Compound 126 (5.9 g, 56%).

¹H-NMR (400 MHz, CDCl₃) δ 7.25-7.36 (m, 5H), 4.57 (s, 2H), 4.02 (s, 2H), 3.76-3.62 (m, 12H), 1.47 (s, 9H).

### Preparation of Compound 127

Compound 126 (9.2 g, 25.9 mmol) was dissolved in ethanol (200 mL), and then palladium/charcoal (10% w/w, 9.0 g) was added thereto. The reaction solution was stirred at room temperature in a hydrogen atmosphere for 2 hours. The reaction solution was filtered through Celite and then concentrated to obtain Compound 127 (6.47 g, 94%).

¹H-NMR (400 MHz, CDCl₃) δ 4.03 (s, 2H), 3.76-3.67 (m, 12H), 2.59 (m, 1H), 1.47 (s, 9H).

### Preparation of Compound 128

Oxalyl chloride (3.5 mL, 41.2 mmol) was diluted with dichloromethane (80 mL) at -78°C, and then a solution prepared by diluting dimethylsulfoxide (3.4 mL, 48.4 mmol) with dichloromethane (10 mL) was gradually added thereto. After 10 minutes, Compound 127 (6.4 g, 24.2 mmol) dissolved in dichloromethane (10 mL) was added to the reaction solution at -78°C in a nitrogen atmosphere. The reaction solution was stirred for 30 minutes, then triethylamine (16.8 mL, 121.0 mmol) was added thereto, and the mixture was stirred at room temperature for 18 hours. After distilled water (20 mL) was added thereto, the mixture was diluted with dichloromethane (100 mL), and washed with 0.5 N aqueous hydrochloric acid solution (50 mL) and saturated aqueous sodium hydrogen carbonate solution (50 mL). The collected organic layer was dried over anhydrous sodium sulfate, filtered, and then concentrated to obtain Compound 128 (6.2 g, 97%).

¹H-NMR (400 MHz, CDCl₃) δ 9.74 (s, 1H), 4.17 (s, 2H), 4.03 (s, 2H), 3.76-3.67 (m, 8H), 1.47 (s, 9H).

### Preparation of Compound 129

Compound 128 (3.0 g, 11.4 mmol) was dissolved in tetrahydrofuran (20 mL), then 2 M 2-methyl-2-butene tetrahydrofuran solution (28 mL, 57.2 mmol) was added thereto, and a solution prepared by dissolving sodium chlorite (3.2 g, 28.6 mmol) and disodium phosphate (2.2 g, 16.0 mmol) in distilled water (40 mL) and hydrogen peroxide (30 wt% aqueous solution, 18.0 g, 160.1 mmol) were sequentially added thereto at 0°C in a nitrogen atmosphere. The reaction solution was stirred at 50°C for 2 hours, acidified to pH 3 with 1 N aqueous hydrochloric acid solution, then diluted with ethyl acetate (200 mL), washed with salt water (100 mL), and dried over anhydrous sodium sulfate. The resultant was filtered, then basified with diisopropylethylamine, then concentrated, and dissolved in tetrahydrofuran (40 mL), then methyl iodide (2.1 mL, 34.2 mmol) and potassium carbonate (1.6 g, 11.4 mmol) were sequentially added thereto at 0°C in a nitrogen atmosphere, and the mixture was stirred at room temperature for 16 hours. The reaction solution was diluted with ethyl acetate (200 mL), washed with distilled water (100 mL), and dried over anhydrous sodium sulfate. Filtration, concentration, and purification by column chromatography were performed to obtain Compound 129 (1.1 g, 33%).

¹H-NMR (400 MHz, CDCl₃) δ 4.17 (s, 2H), 4.02 (s, 2H), 3.76-3.62 (m, 11H), 1.47 (s, 9H).

### Preparation of Compound 130

Compound 129 (1.1 g, 3.8 mmol) was dissolved in dichloromethane (5 mL), trifluoroacetic acid (5 mL, 65.3 mmol) and anisole (4.1 mL, 37.6 mmol) were sequentially added thereto at 0°C in a nitrogen atmosphere. The reaction solution was stirred at room temperature for 4 hours and concentrated to obtain Compound 130 (867 mg, 97%).

¹H-NMR (400 MHz, CDCl₃) δ 7.48 (m, 1H), 4.18 (s, 2H), 4.16 (s, 2H), 3.77-3.73 (m, 11H).

### <Example 38> Preparation of Compound 137

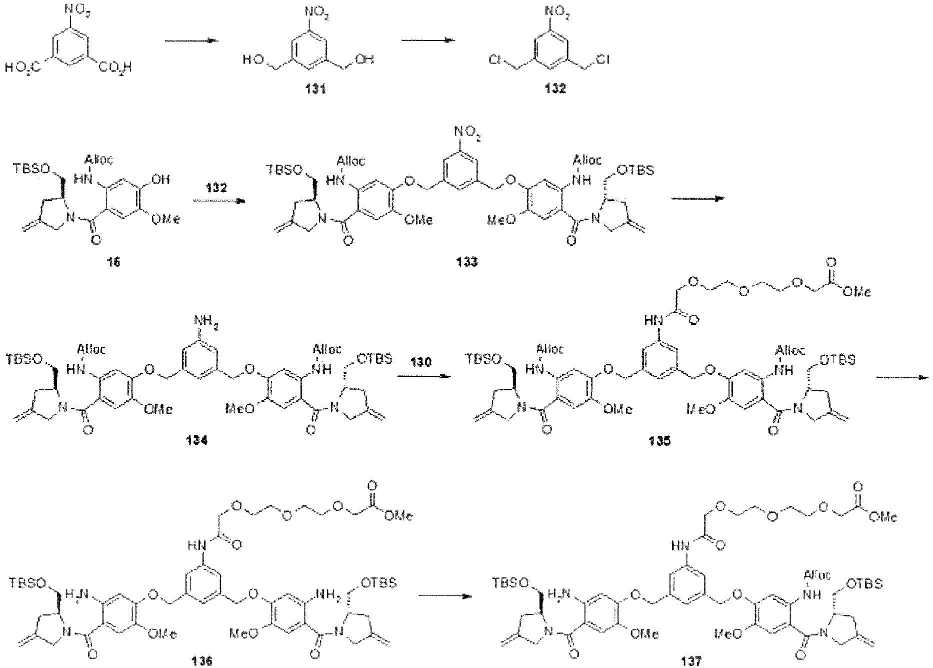

### Preparation of Compound 131

After 5-nitrobenzene-1,3-dicarboxylic acid (5 g, 23.7 mmol) was dissolved in tetrahydrofuran (30 mL), and then borane (1 M tetrahydrofuran solution, 94.8 mL, 94.8 mmol) was gradually added thereto at -20°C in a nitrogen atmosphere. The reaction solution was stirred for 48 hours while being gradually heated to room temperature, and then cooled to 0°C, and methanol (30 mL) was gradually added to the reaction solution. The reaction solution was concentrated, then diluted with ethyl acetate (100 mL), and washed with distilled water (100 mL). The collected organic layer was dried over anhydrous sodium sulfate, filtered and then concentrated to obtain Compound 131 (4.6 g).

¹H-NMR (400 MHz, CDCl₃) δ 8.16 (s, 2H), 7.73 (s, 1H), 4.84 (d, *J* = 4.0 Hz, 4H), 1.91 (t, *J* = 5.6Hz, 2H).

### Preparation of Compound 132

Compound 131 (4.6 g) was dissolved in dichloromethane (150 mL), and then triethylamine (18.2 mL, 130.4 mmol) and methanesulfonyl chloride (7.7 mL, 99.5 mmol) were added thereto at 0°C in a nitrogen atmosphere. The reaction solution was stirred at room temperature for 16 hours, and then dichloromethane (100 mL) was added thereto. The organic layer was washed with saturated aqueous sodium hydrogen carbonate solution (200 mL) and dried over anhydrous sodium sulfate. Filtration, concentration, and purification by column chromatography were performed to obtain Compound 132 (3.06 g, 2 steps 59%).

¹H-NMR (400 MHz, CDCl3) δ 8.23 (s, 2H), 7.77 (s, 1H), 4.66 (s, 4H).

### Preparation of Compound 133

Compound 132 (500 mg, 2.27 mmol) was dissolved in *N,N*-dimethylformamide (20 mL), then potassium iodide (188.4 mg, 1.14 mmol), Compound 16 (2.8 g, 5.9 mmol), and potassium carbonate (940 mg, 6.8 mmol) were added thereto in this order at 0°C, and the mixture was stirred at 40°C for 16 hours in a nitrogen atmosphere. Distilled water (30 mL) was added to the reaction solution, and then extraction with ethyl acetate (2 × 50 mL) was performed. The collected organic layer was washed with salt water (50 mL) and dried over anhydrous sodium sulfate. Filtration, concentration under reduced pressure, and purification by column chromatography were performed to obtain Compound 133 (1.72 g, 69%).

¹H-NMR (400 MHz, CDCl₃) δ 8.80 (br s, 1H), 8.34 (s, 2H), 7.93-7.90 (m, 3H), 6.85 (s, 2H), 5.94-5.93 (m, 2H), 5.34 (d, J = 17.6 Hz, 2H), 5.27-5.22 (m, 6H), 4.99-4.91 (m, 4H), 4.61 (s, 4H), 4.21-4.17 (m, 2H), 4.03-3.85 (m, 8H), 3.64 (m, 2H), 2.70 (s, 4H), 0.88 (s, 18H), 0.03 (s, 12H) .

### Preparation of Compound 134

Compound 133 (200 mg, 0.182 mmol) was dissolved in ethanol (5 mL), and then zinc dust (475 mg, 7.268 mmol) and formic acid (0.27 mL, 7.268 mmol) were added thereto. The reaction solution was stirred at room temperature for 1 hour and then filtered through Celite, and ethyl acetate (20 mL) was added thereto. The organic layer was washed with saturated aqueous sodium hydrogen carbonate solution (20 mL) and salt water (20 mL) in this order, and then dried over anhydrous sodium sulfate. Filtration and concentration were performed to obtain Compound 134 (200 mg, 99%).

EI-MS m/z: [M+H]⁺ 1070.8, [M+Na]⁺ 1092.9.

¹H-NMR (400 MHz, CDCl₃) δ 8.80 (br s, 1H), 7.89 (s, 2H), 6.93 (s, 1H), 6.82-6.78 (m, 4H), 5.95-5.93 (m, 2H), 5.36-5.09 (m, 8H), 4.98-4.90 (m, 4H), 4.62 (m, 4H), 4.20-4.03 (m, 5H), 3.83-3.64 (m, 11H), 2.69 (s, 4H), 0.87 (s, 18H), 0.23 (s, 9H).

### Preparation of Compound 135

Compound 134 (1.2 g, 1.1 mmol) and Compound 130 (386 mg, 1.6 mmol) were dissolved in dimethylformamide (15 mL), then *N,N,N',N'*-tetramethyl-*O*-(1H-benzotriazol-1-yl)uronium hexafluorophosphate (779 mg, 2.0 mmol) and diisopropylethylamine (0.56 mL, 3.3 mmol) were sequentially added thereto at 0°C in a nitrogen atmosphere, and the mixture was stirred at room temperature for 3 hours. The reaction solution was diluted with ethyl acetate (100 mL), washed with saturated aqueous ammonium chloride solution (50 mL), saturated aqueous sodium hydrogen carbonate solution (50 mL), and distilled water (50 mL) in this order, and dried over anhydrous sodium sulfate. Filtration, concentration, and purification by column chromatography were performed to obtain Compound 135 (1.1 g, 78%).

EI-MS m/z: [M+H]⁺ 1289.7, 1/2[M+H]⁺ 645.1.

### Preparation of Compound 136

Compound 135 (1.1 g, 0.85 mmol) was dissolved in dichloromethane (20 mL), then pyrrolidine (0.27 mL, 3.34 mmol) and tetrakis(triphenylphosphine)palladium(0) (49 mg, 0.043 mmol) were added thereto, and the mixture was stirred at room temperature in a nitrogen atmosphere for 30 minutes. Distilled water (30 mL) was added to the reaction solution, and then extraction with dichloromethane (2 × 50 mL) was performed. The collected organic layer was washed with salt water (50 mL) and dried over anhydrous sodium sulfate. Filtration, concentration under reduced pressure, and purification by column chromatography were performed to obtain Compound 136 (937 mg, 97%).

EI-MS m/z: [M+H]⁺ 1112.1, 1/2[M+H]⁺ 561.1.

### Preparation of Compound 137

Compound 136 (937 mg, 0.83 mmol) was dissolved in dichloromethane (40 mL), then pyridine (0.14 mL, 1.67 mmol) and allyl chloroformate (0.080 mL, 0.75 mmol) were added thereto at -78°C in a nitrogen atmosphere, and the reaction solution was stirred for 1 hour. Distilled water (50 mL) was added to the reaction solution, and then extraction with dichloromethane (2 × 50 mL) was performed. The collected organic layer was washed with salt water (50 mL) and dried over anhydrous sodium sulfate. Filtration, concentration under reduced pressure, and purification by column chromatography were performed to obtain Compound 137 (465 mg, 46%).

EI-MS m/z: [M+H]⁺ 1205.3, 1/2[M+H]⁺ 603.0.

### <Example 39> Preparation of Compound 140

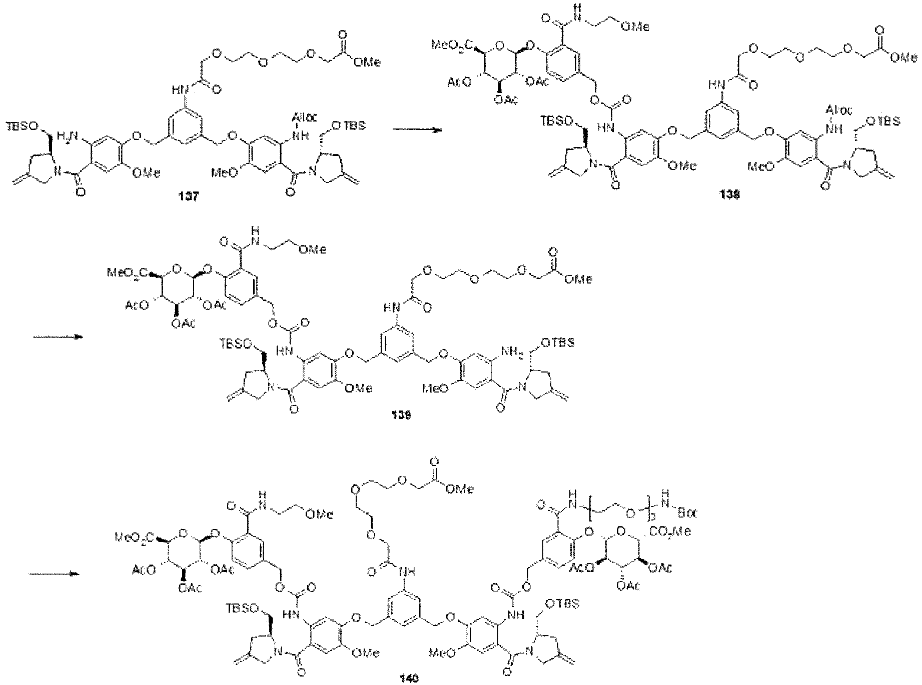

### Preparation of Compound 138

Compound 137 (465 mg, 0.38 mmol) was dissolved in dry tetrahydrofuran (20 mL), then triphosgene (40 mg, 0.14 mmol) and N-methylimidazole (0.12 mL, 1.54 mmol) were added thereto at 0°C, and the mixture was stirred in a nitrogen atmosphere for 1 hour. Compound 20 (251 mg, 0.46 mmol) was dissolved in dry tetrahydrofuran (5 mL), N-methylimidazole (0.03 mL, 0.38 mmol) was added thereto, and then this solution was gradually added to the reaction solution. The reaction solution was stirred for 17 hours, then diluted with ethyl acetate (30 mL), washed with salt water (20 mL), and dried over anhydrous sodium sulfate. Filtration, concentration under reduced pressure, and purification by column chromatography were performed to obtain Compound 138 (261 mg, 38%).

EI-MS m/z: [M+H]⁺ 1772.3, 1/2[M+H]⁺ 886.7.

### Preparation of Compound 139

Compound 138 (261 mg, 0.147 mmol) was dissolved in dichloromethane (3 mL), then pyrrolidine (0.018 mL, 0.221 mmol) and tetrakis(triphenylphosphine)palladium(0) (5 mg, 0.0044 mmol) were added thereto, and the mixture was stirred at room temperature in a nitrogen atmosphere for 30 minutes. Distilled water (20 mL) was added to the reaction solution, and then extraction with dichloromethane (2 × 20 mL) was performed. The collected organic layer was washed with salt water (20 mL) and dried over anhydrous sodium sulfate. Filtration, concentration under reduced pressure, and purification by column chromatography were performed to obtain Compound 139 (198 mg, 80%).

EI-MS m/z: [M+H]⁺ 1688.5, 1/2[M+H]⁺ 844.6.

### Preparation of Compound 140

Compound 139 (198 mg, 0.117 mmol) was dissolved in dry tetrahydrofuran (1.5 mL), then triphosgene (12.5 mg, 0.042 mmol) and N-methylimidazole (0.037 mL, 0.469 mmol) were added thereto at 0°C, and the mixture was stirred in a nitrogen atmosphere for 1 hour. Compound 23 (102.9 mg, 0.141 mmol) was dissolved in dry tetrahydrofuran (1.5 mL), N-methylimidazole (0.009 mL, 0.117 mmol) was added thereto, and then this solution was gradually added to the reaction solution. The reaction solution was stirred at room temperature for 6 hours, and then ethyl acetate (20 mL) was added thereto. The organic layer was washed with distilled water (20 mL) and dried over anhydrous sodium sulfate. Filtration, concentration, and purification by column chromatography were performed to obtain Compound 140 (227 mg).

EI-MS m/z: [M+H]⁺ 2444.6, 1/2[M+H]⁺ 1222.8.

### <Example 40> Preparation of Compound 144

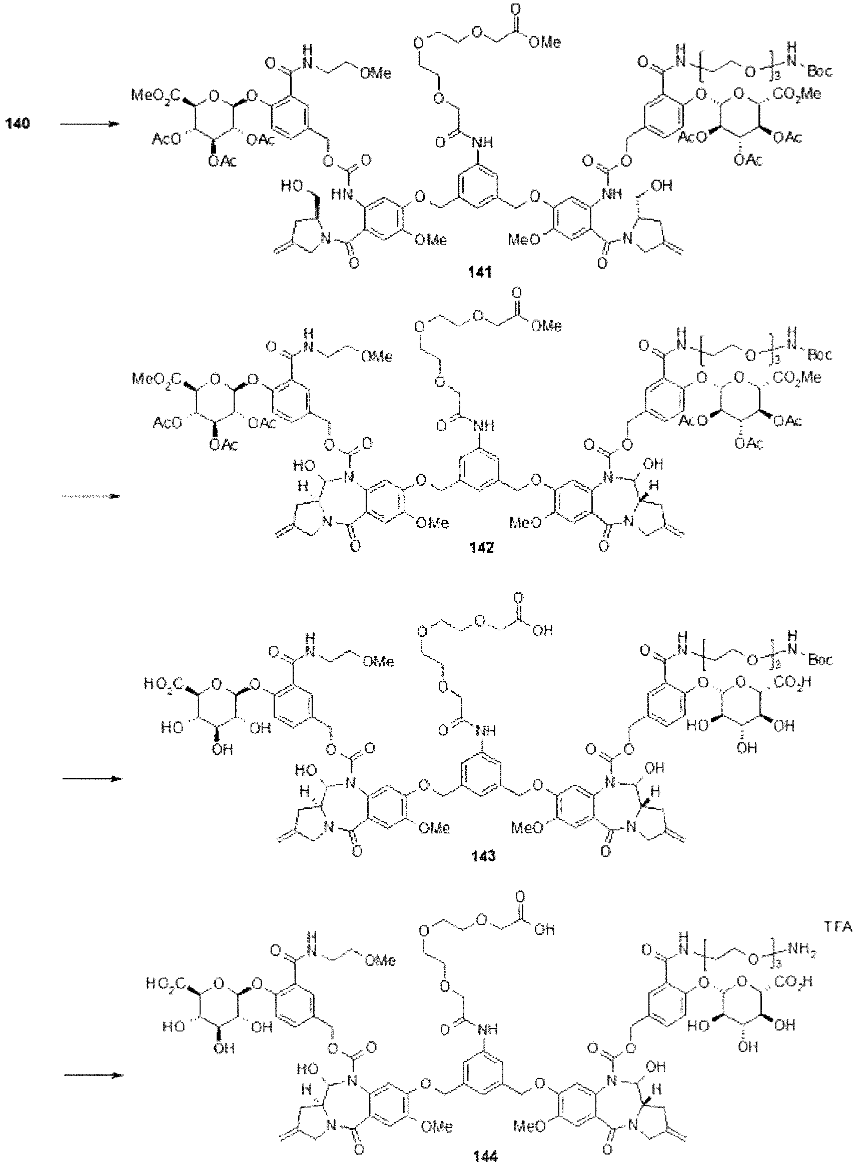

### Preparation of Compound 141

Compound 140 (227 mg) was dissolved in tetrahydrofuran/distilled water (1.0 mL/1.0 mL), acetic acid (2 mL) was added thereto, and then the mixture was stirred at room temperature in a nitrogen atmosphere for 16 hours. Distilled water (20 mL) was added to the reaction solution, and then extraction with ethyl acetate (2 × 20 mL) was performed. The collected organic layer was washed with salt water (30 mL) and dried over anhydrous sodium sulfate. Filtration, concentration under reduced pressure, and purification by column chromatography were performed to obtain Compound 141 (100 mg) .

EI-MS m/z: [M+H]⁺ 2216.2, 1/2[M+H]⁺ 1108.7.

### Preparation of Compound 142

Compound 141 (100 mg) was dissolved in dichloromethane (1.5 mL), then Dess-Martin periodinane (45.9 mg, 0.108 mmol) was added thereto, and the mixture was stirred at room temperature in a nitrogen atmosphere for 16 hours. Saturated aqueous sodium hydrogen carbonate solution (20 mL) was added to the reaction solution, and then extraction with ethyl acetate (2 × 20 mL) was performed. The collected organic layer was washed with salt water (30 mL) and dried over anhydrous sodium sulfate. Filtration, concentration under reduced pressure, and purification by column chromatography were performed to obtain Compound 142 (64 mg).

EI-MS m/z: [M+H]⁺ 2212.5, 1/2[M+H]⁺ 1106.7.

### Preparation of Compound 143

Compound 142 (64 mg, 0.029 mmol) was dissolved in methanol/tetrahydrofuran (1.4 mL/1.4 mL), and then a solution prepared by dissolving lithium hydroxide (7.3 mg, 0.173 mmol) in distilled water (1.4 mL) was gradually added thereto at -50°C. The reaction solution was stirred for 4 hours while gradually raising the reaction temperature to 0°C, then neutralized with acetic acid, concentrated under reduced pressure, then purified by HPLC, and freeze-dried to obtain Compound 143 (20 mg).

EI-MS m/z: [M+H]⁺ 1917.5, 1/2[M+H]⁺ 959.6.

### Preparation of Compound 144

Compound 143 (20 mg, 0.010 mmol) was dissolved in dichloromethane (0.45 mL), and then trifluoroacetic acid (0.05 mL) was gradually added thereto at 0°C. The reaction solution was stirred for 1 hour, concentrated under reduced pressure, then purified by HPLC, and freeze-dried to obtain Compound 144 (9.2 mg, 46%).

EI-MS m/z: [M+H]⁺ 1817.9, 1/2[M+H]⁺ 909.5.

### <Example 41> Preparation of Compound 148

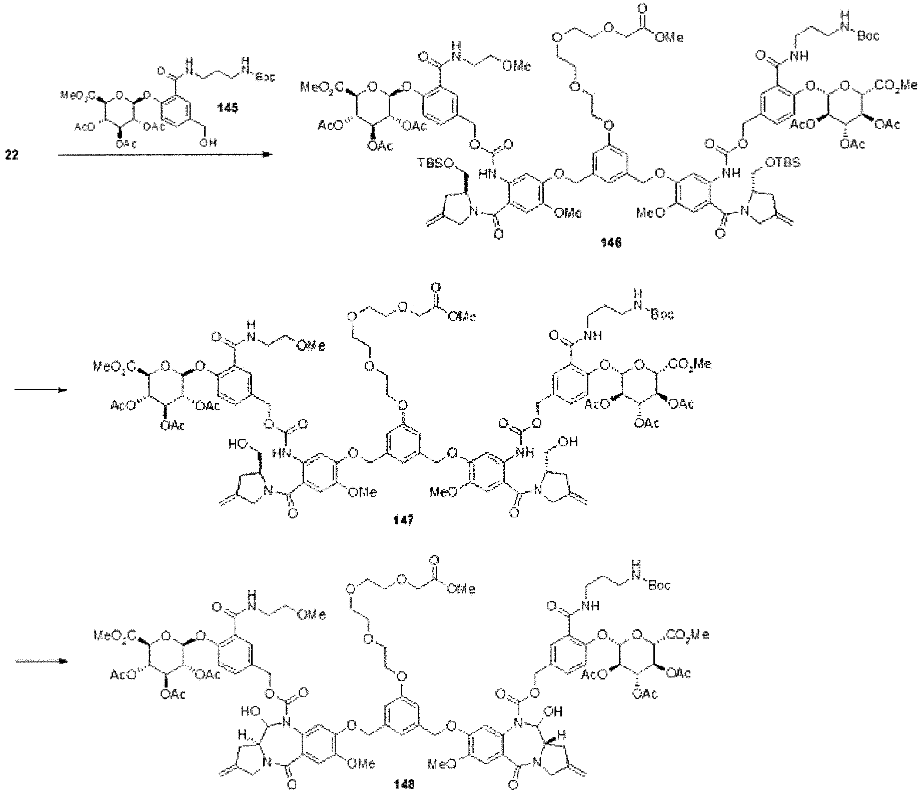

### Preparation of Compound 146

Compound 22 (677 mg, 0.398 mmol) was dissolved in dry tetrahydrofuran (4 mL), then triphosgene (42.5 mg, 0.143 mmol) and N-methylimidazole (0.13 mL, 1.59 mmol) were added thereto at 0°C, and the mixture was stirred in a nitrogen atmosphere for 1 hour. Compound 145 (305.9 mg, 0.478 mmol, Compound 145 was prepared by the method described in Korean Patent Publication No. 10-2018-0110645) was dissolved in dry tetrahydrofuran (4 mL), N-methylimidazole (0.03 mL, 0.398 mmol) was added thereto, and then this solution was gradually added to the reaction solution. The reaction solution was stirred at room temperature for 16 hours, and then ethyl acetate (30 mL) was added thereto. The organic layer was washed with distilled water (20 mL) and then dried over anhydrous sodium sulfate. Filtration, concentration under reduced pressure, and purification by column chromatography were performed to obtain Compound 146 (780 mg).

EI-MS m/z: [M+H]⁺ 2342.1, 1/2[M+H]⁺ 1171.4

### Preparation of Compound 147

Compound 146 (780 mg) was dissolved in tetrahydrofuran/distilled water (3.5 mL/3.5 mL), acetic acid (7 mL) was added thereto, and then the mixture was stirred at room temperature in a nitrogen atmosphere for 6 hours. Distilled water (20 mL) was added to the reaction solution, and then extraction with ethyl acetate (2 × 20 mL) was performed. The collected organic layer was washed with salt water (30 mL) and dried over anhydrous sodium sulfate. Filtration, concentration under reduced pressure, and purification by column chromatography were performed to obtain Compound 147 (393 mg, 3 steps 47%).

EI-MS m/z: [M+H]⁺ 2113.5, 1/2[M+H]⁺ 1057.2.

### Preparation of Compound 148

Compound 147 (393 mg) was dissolved in dichloromethane (6 mL), then Dess-Martin periodinane (174 mg, 0.409 mmol) was added thereto, and the mixture was stirred at room temperature in a nitrogen atmosphere for 6 hours. Saturated aqueous sodium hydrogen carbonate solution (20 mL) was added to the reaction solution, and then extraction with ethyl acetate (2 × 20 mL) was performed. The collected organic layer was washed with salt water (30 mL) and dried over anhydrous sodium sulfate. Filtration, concentration under reduced pressure, and purification by column chromatography were performed to obtain Compound 148 (310 mg).

EI-MS m/z: [M+H]⁺ 2109.7, 1/2[M+H]⁺ 1055.3.

### <Example 42> Preparation of Compound 151

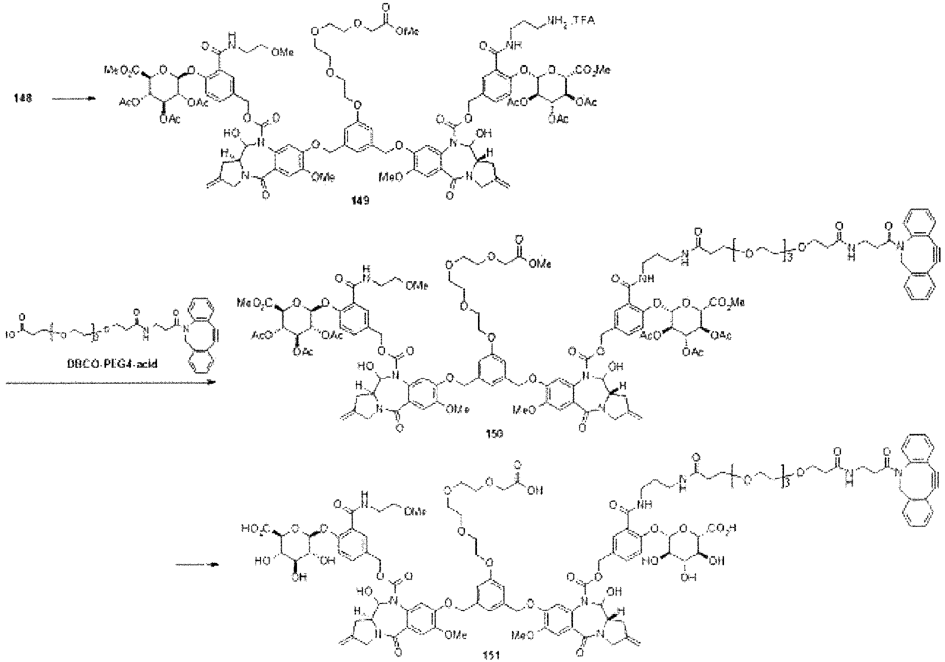

### Preparation of Compound 149

Compound 148 (310 mg) was dissolved in dichloromethane (4.75 mL), and then trifluoroacetic acid (0.5 mL) was gradually added thereto at 0°C. The reaction solution was stirred for 3 hours, then concentrated under reduced pressure, then purified by HPLC, and freeze-dried to obtain Compound 149 (380 mg).

EI-MS m/z: [M+H]⁺ 2009.3, 1/2[M+H]⁺ 1005.1.

### Preparation of Compound 150

Compound 149 (160 mg) and DBCO-PEG4-acid (40.6 mg, 0.074 mmol) were dissolved in N,N-dimethylformamide (3 mL), and then (1-[bis(dimethylamino)methylene]-1*H*-1,2,3-triazole[4,5-b]pyridinium hexafluorophosphate (36.4 mg, 0.096 mmol) and diisopropylethylamine (0.033 mL, 0.191 mmol) were sequentially added thereto at 0°C in a nitrogen atmosphere. The reaction solution at room temperature for 16 hours, then saturated aqueous ammonium chloride solution (20 mL) was added to the reaction solution, and then extraction with ethyl acetate (2 × 20 mL) was performed. The collected organic layer was washed with salt water (30 mL) and dried over anhydrous sodium sulfate. Filtration, concentration under reduced pressure, and purification by column chromatography were performed to obtain Compound 150 (98 mg, 2 steps 52%).

EI-MS m/z: [M+Na]⁺ 2565.0, 1/2[M+H]⁺ 1272.4.

### Preparation of Compound 151

Compound 150 (98 mg, 0.0385 mmol) was dissolved in methanol/tetrahydrofuran (0.7 mL/2.9 mL), and then a solution prepared by dissolving lithium hydroxide (16.15 mg, 0.385 mmol) in distilled water (1.8 mL) was gradually added thereto at -50°C. The reaction solution was stirred for 2 hours while gradually raising the reaction temperature to 0°C, then neutralized with acetic acid, concentrated under reduced pressure, then purified by HPLC, and freeze-dried to obtain Compound 151 (13.2 mg, 15%) .

EI-MS m/z: [M+H]⁺ 2249.1, 1/2[M+H]⁺ 1125.3.

### <Example 43> Preparation of Compound 156

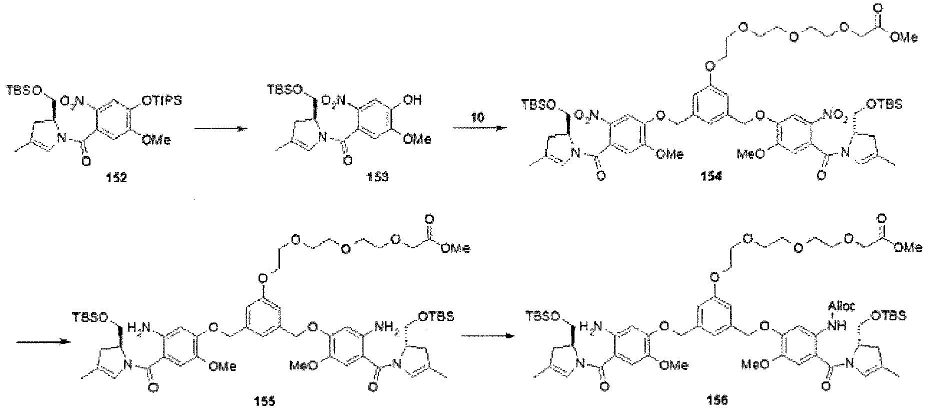

### Preparation of Compound 153

Compound 152 (4.9 g, 8.46 mmol, Compound 152 was prepared by the method described in *ACS. Med. Chem.* 2016, 7, 11, 983-987) was dissolved in *N,N-*dimethylformamide/distilled water (98 mL/6 mL), then sodium acetate (694 mg, 8.46 mmol) was added thereto, and the reaction solution was stirred at 70°C in a nitrogen atmosphere. After 3 hours, the reaction temperature was lowered to room temperature, and ethyl acetate (100 mL) was added to the reaction solution. The organic layer was washed with distilled water (70 mL) and salt water (70 mL) in this order, and then dried over anhydrous sodium sulfate. Filtration, concentration, and purification by column chromatography were performed to obtain Compound 153 (3.52 g, 98%).

¹H-NMR (400 MHz, CDCl₃) δ 7.74 (s, 1H), 6.79 (s, 1H), 6.16 (br s, 1H), 5.54 (s, 1H), 4.65 (m, 1H), 3.98 (s, 3H), 2.84-2.71 (m, 1H), 2.60-2.45 (m, 1H), 1.62 (s, 3H), 1.58 (s, 1H), 0.89 (s, 9H), 0.10 (s, 6H).

### Preparation of Compound 154

Compound 153 (3.5 g, 8.49 mmol) and Compound 10 (1.9 g, 3.69 mmol) were dissolved in N,N-dimethylformamide (20 mL), then potassium carbonate (2.6 g, 18.4 mmol) was added thereto at 0°C, and the mixture was stirred at room temperature in a nitrogen atmosphere for 20 hours. Distilled water (30 mL) was added to the reaction solution, and then extraction with ethyl acetate (2 × 50 mL) was performed. The collected organic layer was washed with salt water (50 mL) and dried over anhydrous sodium sulfate. Filtration, concentration under reduced pressure, and purification by column chromatography were performed to obtain Compound 154 (3.33 g, 78%).

¹H-NMR (400 MHz, CDCl₃) δ 7.75 (d, 2H), 7.13 (s, 1H), 7.01 (s, 2H), 6.80 (s, 2H), 5.55 (s, 2H), 5.18 (s, 4H), 4.65 (m, 2H), 4.16 (s, 4H), 3.96 (s, 7H), 3.87 (t, *J* = 4.8 Hz, 3H), 3.71-3.63 (m, 13H), 2.86-2.66 (m, 2H), 2.58-2.44 (m, 2H), 1.59 (s, 6H) 0.89 (s, 18H), 0.10 (s, 12H).

### Preparation of Compound 155

Compound 154 (3.3 g, 2.82 mmol) was dissolved in ethanol (47 mL), then zinc dust (7.2 g, 113.06 mmol) and formic acid (5% in EtOH, 3 mL) were added thereto, and the mixture was stirred at room temperature in a nitrogen atmosphere for 3 hours. Saturated aqueous sodium hydrogen carbonate solution (50 mL) was added to the reaction solution, and then extraction with ethyl acetate (2 × 50 mL) was performed. The collected organic layer was washed with salt water (50 mL) and dried over anhydrous sodium sulfate. Filtration, concentration under reduced pressure, and purification by column chromatography were performed to obtain Compound 155 (2.15 g, 70%).

¹H-NMR (400 MHz, CDCl₃) δ 7.02 (s, 1H), 6.92 (s, 2H), 6.75 (s, 2H), 6.25-6.15 (m, 4H), 5.08 (s, 4H), 4.71-4.55 (m, 2H), 4.29 (s, 4H), 4.18-3.62 (m, 23H), 2.78-2.64 (m, 2H), 2.58-2.46 (m, 2H), 1.62 (2, 6H) 0.87 (s, 18H), 0.11-0.01 (m, 12H).

### Preparation of Compound 156

Compound 155 (2.2 g, 1.99 mmol) was dissolved in dichloromethane (250 mL), then pyridine (0.4 mL, 4.97 mmol) and allyl chloroformate (0.19 mL, 1.79 mmol) were added thereto at -78°C in a nitrogen atmosphere, and the reaction solution was stirred for 3 hours. Distilled water (100 mL) was added to the reaction solution, and then extraction with dichloromethane (2 × 100 mL) was performed. The collected organic layer was washed with salt water (100 mL) and dried over anhydrous sodium sulfate. Filtration, concentration under reduced pressure, and purification by column chromatography were performed to obtain Compound 156 (730 mg, 35%).

¹H-NMR (400 MHz, CDCl₃) δ 8.76 (br s, 1H), 7.91 (br s, 1H), 7.09 (s, 1H), 6.97 (s, 1H), 6.94 (s, 1H), 6.80 (s, 1H), 6.75 (s, 1H), 6.26-6.10 (m, 3H), 5.99-5.88 (m, 1H), 5.33 (d, *J* = 17.2 Hz, 1H), 5.22 (d, *J* = 10.8 Hz, 1H), 5.13 (s, 2H), 5.10 (s, 2H), 4.72-4.56 (m, 4H), 4.29 (br s, 2H), 4.21-4.09 (m, 5H), 4.01-3.91 (m, 3H), 3.90-3.75 (m, 11H), 3.71-3.63 (m, 12H), 2.78-2.66 (m, 2H), 2.61-2.48 (m, 2H), 1.60 (s, 6H) 0.87 (s, 18H), 0.11-0.01 (m, 12H).

### <Example 44> Preparation of Compound 163

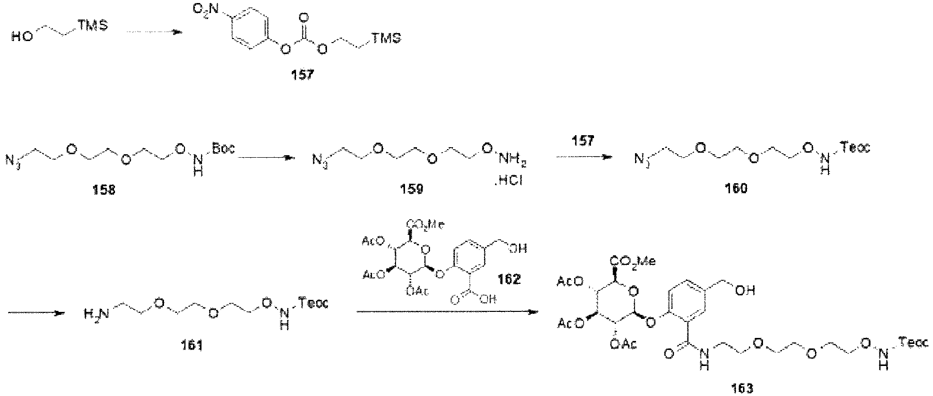

### Preparation of Compound 157

After 2-(trimethylsilyl)ethanol (1 g, 8.45 mmol) and bis(4-nitrophenyl)carbonate (3.1 g, 10.14 mmol) were dissolved in *N,N*-dimethylformamide (20 mL), then *N,N-*diisopropylethylamine (2.9 mL, 16.9 mmol) was added thereto at 0°C, and the mixture was stirred at room temperature in a nitrogen atmosphere for 20 hours. Distilled water (30 mL) was added to the reaction solution, and then extraction with ethyl acetate (2 × 50 mL) was performed. The collected organic layer was washed with salt water (50 mL) and dried over anhydrous sodium sulfate. Filtration, concentration under reduced pressure, and purification by column chromatography were performed to obtain Compound 157 (2.27g, 95%).

¹H-NMR (400 MHz, CDCl₃) δ 8.27 (d, 2H), 7.37 (d, 2H), 4.39 (t, 2H), 1.15 (t, 2H), 0.08 (s, 9H).

### Preparation of Compound 159

Compound 158 (5.0 g, 17.2 mmol, Compound 158 was prepared by the method described in Korean Patent Publication No. 10-2018-0078329) was dissolved in dichloromethane (20 mL), and then hydrochloric acid (4 M 1,4-dioxane solution, 8.61 mL, 34.4 mmol) was added thereto at 0°C in a nitrogen atmosphere. The mixture was stirred for 2 hours and then concentrated under reduced pressure to obtain Compound 159 (3.0 g, 76%).

¹H-NMR (400 MHz, CDCl₃) δ 5.48 (br s, 2H), 3.82 (s, 2H), 3.64 (s, 8H), 3.36 (s, 2H).

### Preparation of Compound 160

Compound 159 (1.1 g, 4.85 mmol) was dissolved in *N,N*-dimethylformamide (15 mL), then *N,N-*diisopropylethylamine (2.2 mL, 12.1 mmol) and Compound 157 (1.7 g, 5.82 mmol) were added thereto, then the reaction solution was stirred at room temperature for 6 hours, and then ethyl acetate (30 mL) was added thereto. The organic layer was washed with distilled water (15 mL) and salt water (15 mL) in this order, and then dried over anhydrous sodium sulfate. Filtration, concentration, and purification by column chromatography were performed to obtain Compound 160 (450 mg, 27%).

¹H-NMR (400 MHz, CDCl₃) δ 7.71 (s, 1H), 4.30-4.21 (m, 2H), 4.03 (s, 1H), 3.79-3.61 (m, 8H), 3.40 (s, 2H), 1.68 (s, 1H), 1.03-0.95 (m, 2H), 0.03 (s, 9H).

### Preparation of Compound 161

Compound 160 (450 mg, 1.34 mmol) was dissolved in methanol (20 mL), and then palladium/charcoal (10 % w/w, 100 mg) was added thereto. The reaction solution was stirred at room temperature in a hydrogen atmosphere for 18 hours. The reaction solution was filtered through Celite and then concentrated to obtain Compound 161 (371 mg, 89%).

¹H-NMR (400 MHz, CDCl₃) δ 4.30-4.18 (m, 2H), 4.06-3.98 (m, 2H), 3.78-3.59 (m, 8H), 3.58-3.52 (m, 2H), 2.87 (t, 2H), 1.02 (t, 2H), 0.03 (s, 9H).

### Preparation of Compound 163

Compound 162 (642 mg, 1.32 mmol, Compound 162 was prepared by the method described in Korean Patent Publication No. 10-2018-0078329) and Compound 161 (371 mg, 1.20 mmol) were dissolved in *N,N*-dimethylformamide (10 mL), and then *N,N,N'*,*N*'-tetramethyl-*O*-(1*H*-benzotriazol-1-yl)uronium hexafluorophosphate (1.2 g, 3.02 mmol) and *N,N*-diisopropylethylamine (0.8 mL, 4.8 mmol) were added thereto at 0°C in a nitrogen atmosphere. The reaction solution was stirred at room temperature for 14 hours, then saturated aqueous ammonium chloride solution (50 mL) was added to the reaction solution, and extraction with ethyl acetate (2 × 50 mL) was performed. The collected organic layer was washed with salt water (50 mL) and dried over anhydrous sodium sulfate. Filtration, concentration, and purification by column chromatography were performed to obtain Compound 163 (621 mg, 67%).

¹H-NMR (400 MHz, CDCl₃) δ 8.12 (s, 1H), 7.95 (s, 1H), 7.50 (t, 1H), 7.45 (d, *J* = 8.0 Hz, 1H), 6.99 (d, *J* = 8.8 Hz, 1H), 5.42-5.20 (m, 4H), 4.62 (s, 2H), 4.31-4.14 (m, 3H), 3.96 (s, 2H), 3.62-3.48 (m, 15H), 2.41 (br s, 1H), 2.03 (s, 9H), 0.98 (t, 2H), 0.01 (s, 9H).

### <Example 45> Preparation of Compound 166

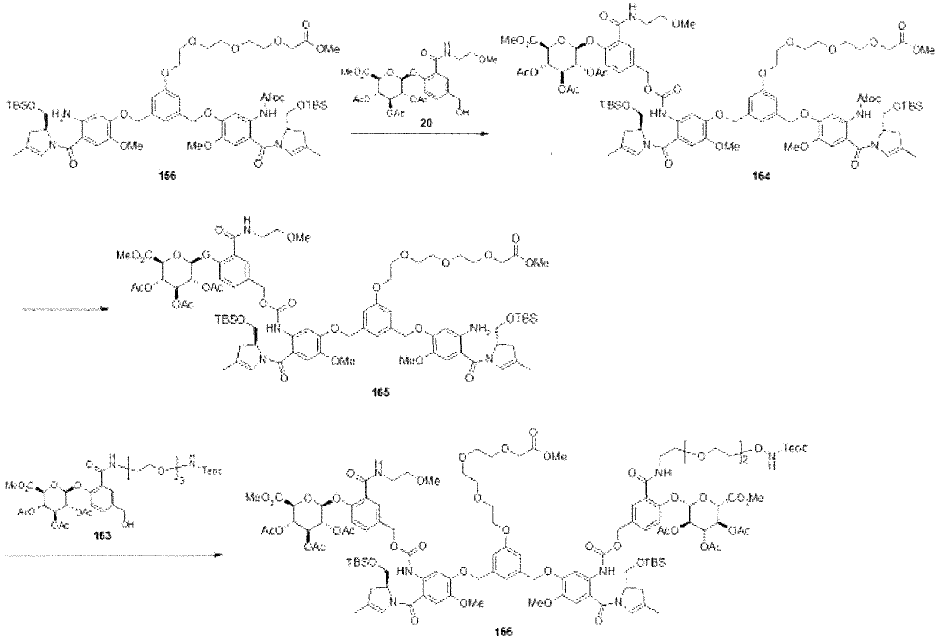

### Preparation of Compound 164

Compound 156 (720 mg, 0.61 mmol) was dissolved in dry tetrahydrofuran (15 mL), triphosgene (65 mg, 0.22 mmol) and *N*-methylimidazole (0.19 mL, 2.42 mmol) were added thereto at -10°C, and the mixture was stirred in a nitrogen atmosphere for 1 hour. Compound 20 (360 mg, 0.67 mmol) was dissolved in dry tetrahydrofuran (15 mL), N-methylimidazole (0.05 mL, 0.6 mmol) was added thereto, and then this solution was gradually added to the reaction solution. After 1 hour, the reaction solution was heated to reflux and stirred for 12 hours. The reaction solution was diluted with ethyl acetate (30 mL), then washed with salt water (20 mL), and dried over anhydrous sodium sulfate. Filtration, concentration under reduced pressure, and purification by column chromatography were performed to obtain Compound 164 (508 mg, 48%).

¹H-NMR (400 MHz, CDCl₃) δ 8.87-8.62 (m, 2H), 8.02 (m, 1H), 7.96-7.84 (m, 1H), 7.49-7.41 (m, 2H), 7.14 (s, 1H), 7.06-6.94 (m, 3H), 6.78 (d, *J* = 3.2 Hz, 2H), 6.19 (br s, 2H), 5.98-5.84 (m, 1H), 5.41-5.02 (m, 10H), 4.69-4.54 (m, 4H), 4.21-4.06 (m, 5H), 4.02-3.88 (m, 2H), 3.87-3.74 (m, 9H), 3.72-3.62 (m, 14H), 3.59-3.52 (m, 2H), 3.39 (s, 2H), 2.77-2.64 (m, 2H), 2.58-2.48 (m, 2H), 2.03 (s, 9H), 1.66 (s, 6H), 0.86 (s, 18H), 0.09-0.01 (m, 12H).

### Preparation of Compound 165

Compound 164 (495 mg, 0.28 mmol) was dissolved in dichloromethane (10 mL), then pyrrolidine (0.070 mL, 0.84 mmol) and tetrakis(triphenylphosphine)palladium(0) (8 mg, 0.007 mmol) were added thereto, and the mixture was stirred at room temperature in a nitrogen atmosphere for 2 hours. Distilled water (20 mL) was added to the reaction solution, and then extraction with dichloromethane (2 × 20 mL) was performed. The collected organic layer was washed with salt water (20 mL) and dried over anhydrous sodium sulfate. Filtration, concentration under reduced pressure, and purification by column chromatography were performed to obtain Compound 165 (372 mg, 79%).

¹H-NMR (400 MHz, CDCl₃) δ 8.91-8.79 (m, 1H), 7.93 (br s, 1H), 7.59-7.34 (m, 2H), 7.10 (s, 1H), 7.03 (s, 1H), 6.97 (d, *J* = 11.6 Hz, 2H), 6.78 (d, *J* = 12.8 Hz, 2H), 6.22 (d, *J* = 14.0 Hz, 2H), 5.46-5.26 (m, 3H), 5.12 (d, *J* = 12.8 Hz, 6H), 4.64 (br s, 2H), 4.32 (br, 2H), 4.24-4.20 (m, 1H), 4.16 (s, 5H), 4.04-3.89 (m, 3H), 3.87-3.64 (m, 26H), 3.57 (s, 3H), 3.31 (s, 2H), 3.23 (s, 1H), 2.78-2.64 (m, 2H), 2.62-2.48 (m, 2H), 2.05 (s, 9H), 1.68 (s, 6H), 0.88 (s, 18H), 0.04 (s, 12H).

### Preparation of Compound 166

Compound 165 (365 mg, 0.22 mmol) was dissolved in dry tetrahydrofuran (10 mL), then triphosgene (23 mg, 0.08 mmol) and *N*-methylimidazole (0.07 mL, 0.87 mmol) were added thereto at -10°C, and the mixture was stirred in a nitrogen atmosphere for 1 hour. Compound 163 (200 mg, 0.26 mmol) was dissolved in dry tetrahydrofuran (10 mL), N-methylimidazole (0.04 mL, 0.44 mmol) was added thereto, and then this solution was gradually added to the reaction solution. After 1 hour, the reaction solution was heated to reflux and stirred for 16 hours. The reaction solution was diluted with ethyl acetate (30 mL), then washed with salt water(20 mL), and dried over anhydrous sodium sulfate. Filtration, concentration under reduced pressure, and purification by column chromatography were performed to obtain Compound 166 (513 mg).

¹H-NMR (400 MHz, CDCl₃) δ 8.84 (br s, 1H), 8.08-7.99 (m, 2H), 7.98-7.88 (m, 2H), 7.71-7.62 (m, 1H), 7.58-7.51 (m, 1H), 7.49-7.41 (m, 6H), 7.09-6.98 (m, 4H), 6.79 (s, 2H), 6.20 (br s, 2H), 5.42-5.24 (m, 8H), 5.19-5.04 (m, 6H), 4.66 (s, 4H), 4.24-4.16 (m, 4H), 4.04-3.92 (m, 4H), 3.88-3.76 (m, 1H), 3.76-3.64 (m, 3H), 3.55 (s, 3H), 3.41 (s, 2H), 3.31 (s, 1H), 2.78-2.64 (m, 2H), 2.59-2.49 (m, 2H), 2.04 (s, 18H), 1.67 (s, 6H), 1.00 (t, *J* = 8.4 Hz, 2H), 0.87 (s, 18H), 0.02 (s, 21H).

### <Example 46> Preparation of Compound 170

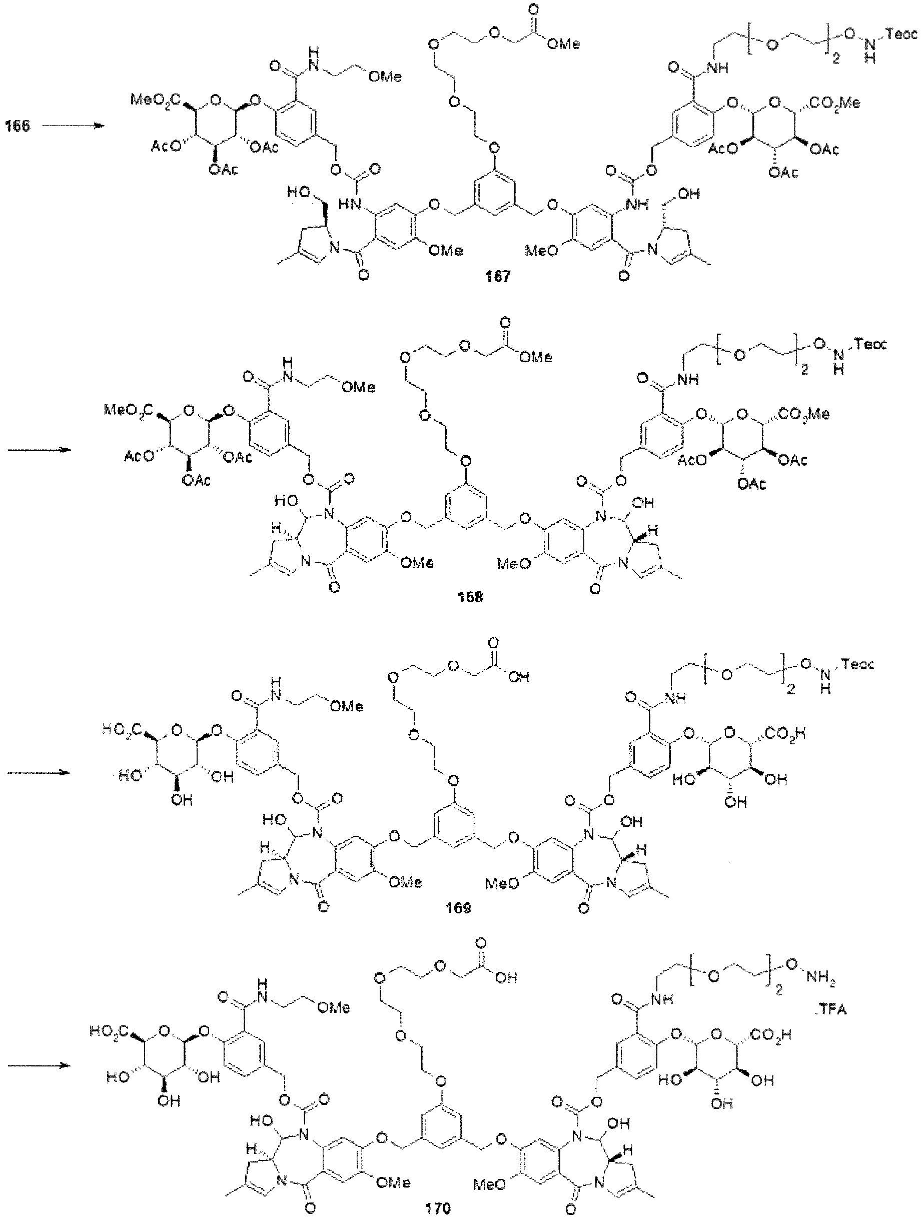

### Preparation of Compound 167

Compound 166 (513 mg, 0.21 mmol) was dissolved in tetrahydrofuran/distilled water (4 mL/4 mL), acetic acid (8 mL) was added thereto, and then the mixture was stirred at room temperature in a nitrogen atmosphere for 16 hours. The reaction solution was concentrated under reduced pressure and then purified by column chromatography to obtain Compound 167 (172 mg).

¹H-NMR (400 MHz, CDCl₃) δ 8.44 (br s, 1H), 8.14-7.96 (m, 2H), 7.72-7.49 (m, 2H), 7.48-7.32 (m, 2H), 7.09 (s, 1H), 7.08-6.84 (m, 4H), 6.80 (s, 2H), 6.04 (s, 2H), 5.44-5.24 (m, 5H), 5.23-4.98 (m, 6H), 4.70 (s, 2H), 4.28-4.06 (m, 7H), 4.01 (S, 1H), 3.90-3.76 (m, 9H), 3.76-3.61 (m, 18H), 3.56 (s, 3H), 3.41 (s, 2H), 3.31 (s, 1H), 2.90-2.74 (m, 2H), 2.36-2.22 (m, 2H), 2.14-2.08 (m, 3H), 2.04 (s, 18H), 1.64 (s, 6H). EI-MS m/z : [M+H]+ 2247.3, 1/2[M+H]+ 1124.2.

### Preparation of Compound 168

Compound 167 (165 mg, 0.056 mmol) was dissolved in dichloromethane (10 mL), then Dess-Martin periodinane (52 mg, 0.12 mmol) was added thereto, and the mixture was stirred at room temperature in a nitrogen atmosphere for 24 hours. The reaction solution was concentrated under reduced pressure and then purified by column chromatography to obtain Compound 168 (128 mg, 78%).

EI-MS m/z: [M+H]⁺ 2243.3, 1/2[M+H]⁺ 1121.5.

### Preparation of Compound 169

Compound 168 (120 mg, 0.053 mmol) was dissolved in methanol/tetrahydrofuran (1 mL/3 mL), and then a solution prepared by dissolving lithium hydroxide (22 mg, 0.54 mmol) in distilled water (1 mL) was gradually added thereto at -78°C. The mixture was stirred for 4 hours while gradually raising the reaction temperature to 0°C. The reaction solution was neutralized with acetic acid, then concentrated under reduced pressure, purified by HPLC, and freeze-dried to obtain Compound 169 (20 mg, 20%) as a white solid.

EI-MS m/z: [M+H]⁺ 1950.7, 1/2[M+H]⁺ 975.2.

### Preparation of Compound 170

Compound 169 (19 mg, 0.01 mmol) was diluted with tetrahydrofuran (2 mL), then tetrabutylammonium fluoride (0.28 mL, 0.29 mmol) was added thereto at room temperature, and the mixture was stirred for 2 hours. The reaction solution was concentrated under reduced pressure, then purified with resin, purified by HPLC, and freeze-dried to obtain Compound 170 as a white solid (2.3 mg).

EI-MS m/z: [M+H]⁺ 1804.9, 1/2[M+H]⁺ 903.1.

### <Example 47> Preparation of Compound 174

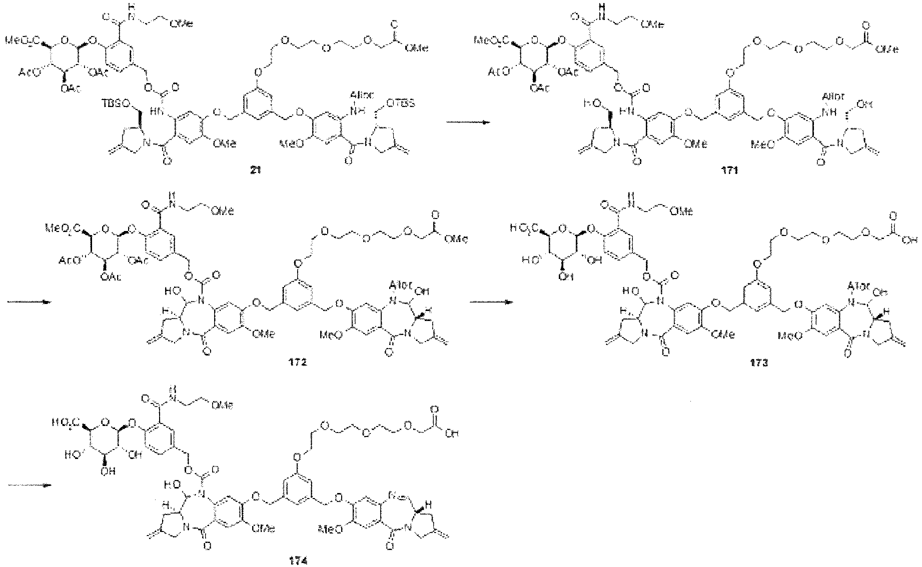

### Preparation of Compound 171

Compound 21 (500 mg, 0.28 mmol) was dissolved in tetrahydrofuran/distilled water (3 mL/3 mL), acetic acid (6 mL) was added thereto, and then the mixture was stirred at room temperature in a nitrogen atmosphere for 6 hours. The reaction solution was concentrated under reduced pressure and then purified by column chromatography to obtain Compound 171 (284 mg, 65%).

¹H-NMR (400 MHz, CDCl₃) δ 8.43 (br s, 2H), 8.05 (s, 1H), 7.52 (s, 1H), 7.40 (s, 2H), 7.06-6.98 (m, 1H), 6.95 (s, 1H), 6.89 (s, 2H), 6.94-6.74 (m, 2H), 5.48-5.25 (m, 5H), 5.24-5.05 (m, 7H), 5.01-4.75 (m, 4H), 4.78-4.46 (m, 4H), 4.17 (s, 7H), 3.90-3.86 (m, 3H), 3.84-3.76 (m, 6H), 3.73-3.65 (m, 16H), 3.62-3.54 (m, 3H), 3.41 (s, 3H), 2.90-2.74 (m, 2H), 2.36-2.22 (m, 2H), 2.04 (s, 3H), 2.04 (s, 18H), 1.64 (s, 6H). EI-MS m/z : [M+H]+ 2247.3, 1/2[M+H]+ 1124.2.

### Preparation of Compound 172

Compound 171 (280 mg, 0.056 mmol) was dissolved in dichloromethane (10 mL), then Dess-Martin periodinane (52 mg, 0.12 mmol) was added thereto, and the mixture was stirred at room temperature in a nitrogen atmosphere for 24 hours. The reaction solution was concentrated under reduced pressure and then purified by column chromatography to obtain Compound 172 (266 mg, 78%).

EI-MS m/z: [M+H]⁺ 1527.53, 1/2[M+H]⁺ 1549.36.

### Preparation of Compound 173

Compound 172 (226 mg, 0.17 mmol) was dissolved in methanol/tetrahydrofuran (2 mL/6 mL), and then a solution prepared by dissolving lithium hydroxide (43 mg, 1.04 mmol) in distilled water (6 mL) was gradually added thereto at -78°C. The mixture was stirred for 4 hours while gradually raising the reaction temperature to 0°C. The reaction solution was neutralized the with acetic acid, then concentrated under reduced pressure, purified by HPLC, and freeze-dried to obtain Compound 173 (151 mg, 63%) as a white solid.

EI-MS m/z: [M+H]⁺ 1372.84, 1/2[M+H]⁺ 686.83.

### Preparation of Compound 174

Compound 173 (100 mg, 0.07 mmol) was dissolved in dichloromethane (4 mL), then pyrrolidine (0.010 mL, 0.09 mmol) and tetrakis(triphenylphosphine)palladium(0) (2 mg, 0.004 mmol) were added thereto, and the mixture was stirred at room temperature in a nitrogen atmosphere for 2 hours. Distilled water (20 mL) was added to the reaction solution, and then extraction with dichloromethane (2 × 20 mL) was performed. The collected organic layer was washed with salt water (20 mL) and dried over anhydrous sodium sulfate. Filtration, concentration under reduced pressure, and purification by column chromatography were performed to obtain Compound 174 (46.9 mg, 53%).

EI-MS m/z: [M+H]⁺ 1270.5, 1/2[M+H]⁺ 635.8.

### <Example 48> Preparation of Compound 176

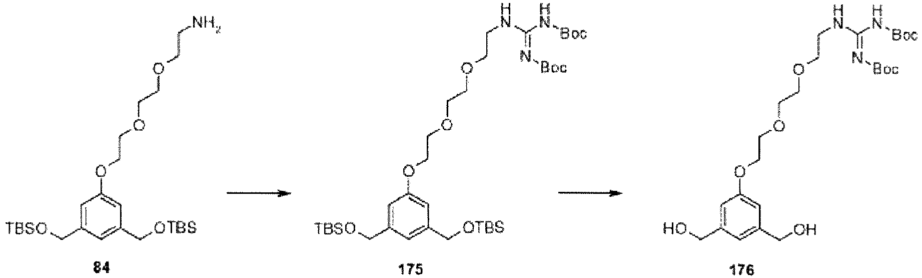

### Preparation of Compound 175

Compound 84 (2.0 g, 3.9 mmol) was dissolved in dichloromethane (30 mL), and then N,N'-bis-tert-butoxycarbonyl-1*H*-pyrazole-1-carboxyamidine (1.3 g, 4.2 mmol) and triethylamine (0.7 mL, 4.6 mmol) were added thereto at 0°C in a nitrogen atmosphere. The reaction solution was stirred at 0°C for 3 hours, then concentrated under reduced pressure, and purified by column chromatography to obtain Compound 175 (2.5 g, 88%).

¹H-NMR (400 MHz, CDCl₃) δ 0.09 (s, 12H), 0.94 (s, 18H), 1.47 (s, 9H), 1.50 (s, 9H), 3.62 (m, 4H), 3.67 (m, 2H), 3.74 (m, 2H), 3.88 (t, 2H), 4.13 (t, 2H), 4.69 (s, 4H), 6.77 (s, 2H), 6.86 (s, 1H), 8.59 (s, 1H), 11.48 (s, 1H).

### Preparation of Compound 176

Compound 175 (2.2 g, 2.9 mmol) was dissolved in tetrahydrofuran (30 mL), then tetrabutylammonium fluoride (1 M tetrahydrofuran solution, 7.4 mL, 7.4 mmol) was added thereto at 0°C in a nitrogen atmosphere, and the mixture was stirred at 0°C for 2 hours. The reaction solution was diluted with ethyl acetate (100 mL), washed with saturated aqueous ammonium chloride solution (50 mL) and distilled water (50 mL) in this order, and dried over anhydrous sodium sulfate. Filtration, concentration under reduced pressure, and purification by column chromatography were performed to obtain Compound 176 (1.5 g, 93%).

¹H-NMR (400 MHz, CDCl₃) δ 1.48 (s, 9H), 1.50 (s, 9H), 3.60 (m, 4H), 3.69 (m, 2H), 3.75 (m, 2H), 3.87 (t, 2H), 4.16 (t, 2H), 4.65 (sd, 4H), 6.87 (s, 2H), 6.92 (s, 1H), 8.59 (s, 1H), 11.41 (s, 1H).

### <Example 49> Preparation of Compound 179

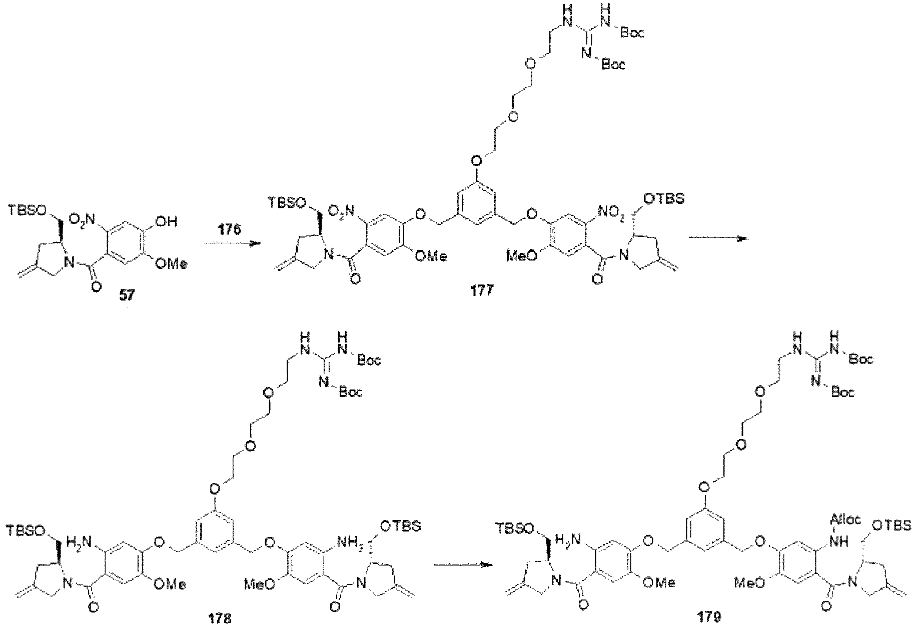

### Preparation of Compound 177

Compound 176 (800 mg, 1.51 mmol) was dissolved in dichloromethane (20 mL) and N,N-dimethylformamide (7 mL), then Compound 57 (1.4 g, 3.33 mmol), triphenylphosphine (994 mg, 3.79 mmol), and diisopropyl azodicarboxylate (0.74 mL, 3.79 mmol) were added thereto in this order at 0°C, and the mixture was stirred at room temperature in a nitrogen atmosphere for 3 hours. Distilled water (30 mL) was added to the reaction solution, and then extraction with ethyl acetate (2 × 50 mL) was performed. The collected organic layer was washed with salt water (50 mL) and dried over anhydrous sodium sulfate. Filtration, concentration under reduced pressure, and purification by column chromatography were performed to obtain Compound 177 (1.7 g, 84%).

EI-MS m/z: [M+H]⁺ 1336.4.

### Preparation of Compound 178

Compound 177 (1.7 g, 1.27 mmol) was dissolved in ethyl acetate (12 mL) and ethanol (12 mL), then zinc dust (5.03 g, 76.9 mmol) was added thereto, and formic acid (2.9 mL, 76.9 mmol) diluted with ethanol (12 mL) was added thereto. The mixture was stirred at room temperature for 4 hours, then diluted with ethyl acetate (30 mL), and filtered through Celite, and ethyl acetate (100 mL) was added thereto. The organic layer was washed with distilled water (150 mL), saturated aqueous sodium hydrogen carbonate solution (150 mL), and salt water (100 mL) in this order, and then dried over anhydrous sodium sulfate. Filtration, concentration, and purification by column chromatography were performed to obtain Compound 178 (1.21 g, 74%).

EI-MS m/z: [M+H]⁺ 1276.4.

### Preparation of Compound 179

Compound 178 (1.2 g, 0.94 mmol) was dissolved in dichloromethane (50 mL), then pyridine (0.22 mL, 2.82 mmol) and allyl chloroformate (0.1 mL, 0.94 mmol) were added thereto at -78°C in a nitrogen atmosphere, and the reaction solution was stirred for 3 hours. The reaction solution was concentrated under reduced pressure and then purified by column chromatography to obtain Compound 179 (312 mg, 23%).

EI-MS m/z: [M+H]⁺ 1360.5.

### <Example 50> Preparation of Compound 182

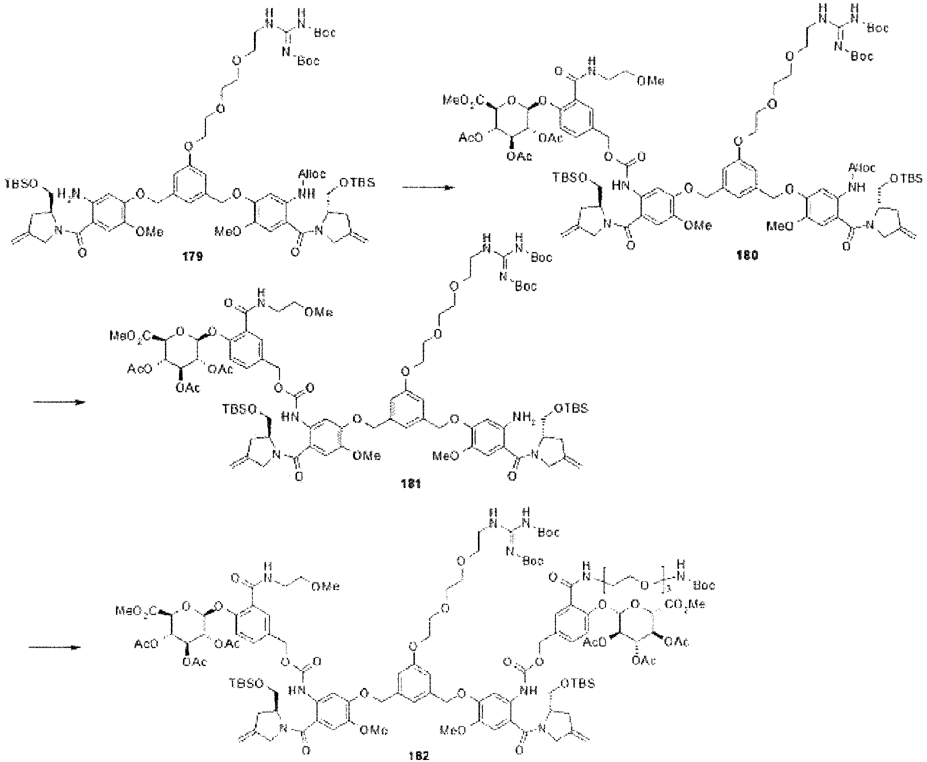

### Preparation of Compound 180

Compound 179 (312 mg, 0.23 mmol) and triphosgene (24 mg, 0.082 mmol) were dissolved in dry dichloromethane (15 mL), then N-methylimidazole (91 µL, 1.15 mmol) was added thereto at -10°C, and the mixture was stirred in a nitrogen atmosphere for 1 hour. Compound 20 (148 mg, 0.27 mmol) was dissolved in dry dichloromethane (3 mL), N-methylimidazole (18 µL, 0.23 mmol) was added thereto, then this solution was gradually added to the reaction solution, and the mixture was stirred at room temperature for 17 hours. The reaction solution was diluted with dichloromethane (20 mL), washed with distilled water (30 mL), and then dried over anhydrous sodium sulfate. Filtration, concentration under reduced pressure, and purification by column chromatography were performed to obtain Compound 180 (315 mg, 69%).

EI-MS m/z: [M+H]⁺ 1927.8, 1/2[M+H]⁺ 964.5.

### Preparation of Compound 181

Compound 180 (315 mg, 0.16 mmol) was dissolved in dichloromethane (3 mL), then pyrrolidine (40 µL, 0.49 mmol) and tetrakis(triphenylphosphine)palladium(0) (9 mg, 0.008 mmol) were added thereto, and the mixture was stirred at room temperature in a nitrogen atmosphere for 30 minutes. Distilled water (10 mL) was added to the reaction solution, and then extraction with dichloromethane (2 × 10 mL) was performed. The collected organic layer was washed with salt water (10 mL) and dried over anhydrous sodium sulfate. Filtration, concentration under reduced pressure, and purification by column chromatography were performed to obtain Compound 181 (221 mg, 80%).

EI-MS m/z: [M+H]⁺ 1843.8, 1/2[M+H]⁺ 922.4.

### Preparation of Compound 182

Compound 181 (221 mg, 0.12 mmol) was dissolved in dry dichloromethane (12 mL), then triphosgene (12 mg, 0.043 mmol) and N-methylimidazole (38 µL, 0.48 mmol) were added thereto at 0°C, and the mixture was stirred in a nitrogen atmosphere for 1 hour. Compound 23 (105 mg, 0.14 mmol) was dissolved in dry dichloromethane (5 mL), N-methylimidazole (9.5 µL, 0.12 mmol) was added thereto, and then this solution was gradually added to the reaction solution. The reaction solution was stirred for 17 hours, then diluted with dichloromethane (15 mL), then washed with salt water (20 mL), and dried over anhydrous sodium sulfate. Filtration, concentration under reduced pressure, and purification by column chromatography were performed to obtain Compound 182 (131 mg, 42%).

EI-MS m/z: 1/2[M+H]⁺ 1301.1.

### <Example 51> Preparation of Compound 183

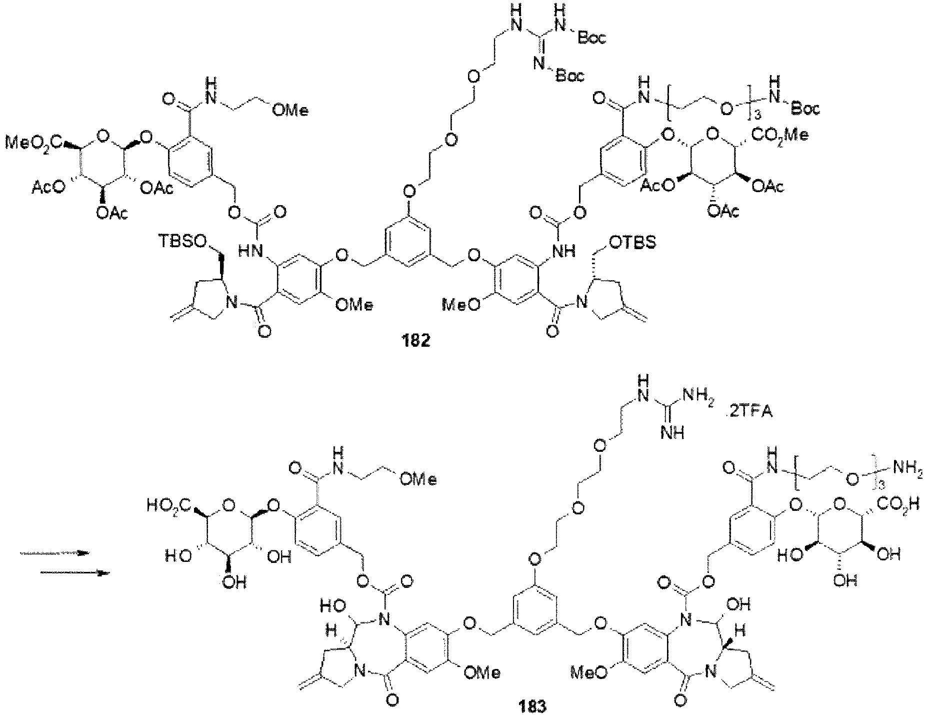

Compound 183 was prepared from Compound 182 by a method similar to that for the synthesis of Compound 97.

EI-MS m/z: [M+H]⁺ 1788.5, 1/2[M+H]⁺ 895.3.

### <Example 52> Preparation of Compound 188

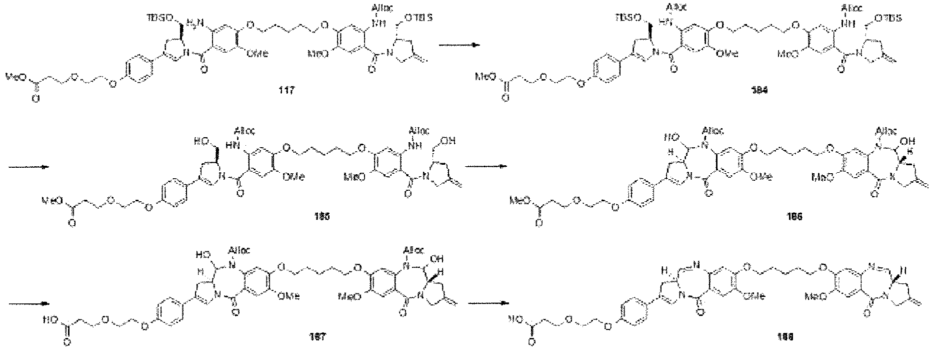

### Preparation of Compound 184

Compound 117 (250 mg, 0.218 mmol) was dissolved in dichloromethane (20 mL), then pyridine (0.035 mL, 0.436 mmol) and allyl chloroformate (0.026 mL, 0.24 mmol) were added thereto at 0°C in a nitrogen atmosphere, and the reaction solution was stirred for 1 hour. The reaction solution was diluted with dichloromethane (30 mL) and washed with distilled water (40 mL), and then the organic layer was dried over anhydrous sodium sulfate. Filtration and concentration under reduced pressure were performed to obtain Compound 184 (crude 273 mg, quant.).

### Preparation of Compound 185

Compound 184 (crude 273 mg, 0.218 mmol) was dissolved in tetrahydrofuran/distilled water (3 mL/3 mL), then acetic acid (7 mL) was added thereto, and the mixture was stirred at room temperature in a nitrogen atmosphere for 16 hours. The reaction solution was concentrated under reduced pressure and then purified by column chromatography to obtain Compound 185 (210 mg, 96%).

### Preparation of Compound 186

Compound 185 (210 mg, 0.209 mmol) was dissolved in dichloromethane (10 mL), then Dess-Martin periodinane (205 mg, 0.48 mmol) was added thereto, and the mixture was stirred at room temperature in a nitrogen atmosphere for 4 hours. The reaction solution was diluted with dichloromethane (30 mL), washed with saturated aqueous sodium hydrogen carbonate solution (30 mL), and dried over anhydrous sodium sulfate. Filtration, concentration under reduced pressure, and purification by column chromatography were performed to obtain Compound 186 (146 mg, 70%).

EI-MS m/z: [M+H]⁺ 997.6, 1/2[M+H]⁺ 499.4.

### Preparation of Compound 187

Compound 186 (146 mg, 0.146 mmol) was dissolved in tetrahydrofuran (1.6 mL), then a solution prepared by dissolving lithium hydroxide (9.2 mg, 0.219 mmol) in distilled water (1.6 mL) was gradually added thereto at 0°C, and the mixture was stirred at room temperature for 6 hours. The reaction solution was neutralized with acetic acid, concentrated under reduced pressure, then purified by HPLC, and freeze-dried to obtain Compound 187 (102 mg, 71%).

EI-MS m/z : [M+H]⁺ 983.7, 1/2[M+H]⁺ 492.5.

### Preparation of Compound 188

Compound 187 (102 mg, 0.103 mmol) was dissolved in dichloromethane (10 mL), then pyrrolidine (0.04 mL, 0.515 mmol) and tetrakis(triphenylphosphine)palladium(0) (2.4 mg, 0.002 mmol) were added thereto, and the mixture was stirred at room temperature in a nitrogen atmosphere for 1 hour. The reaction solution was concentrated under reduced pressure, then purified by HPLC, and freeze-dried to obtain Compound 188 (33 mg, 41%).

EI-MS m/z : [M+H]⁺ 779.5, 1/2[M+H]⁺ 390.4.

### <Example 53>

### Preparation of ADC

ADCs were prepared through the following two steps, and the commonly used LCB14-0512 and LCB14-0606 were prepared by the method described in Korean Patent Publication No. 10-2014-0035393. The structural formulas of LCB14-0512 and LCB14-0606 are as follows: 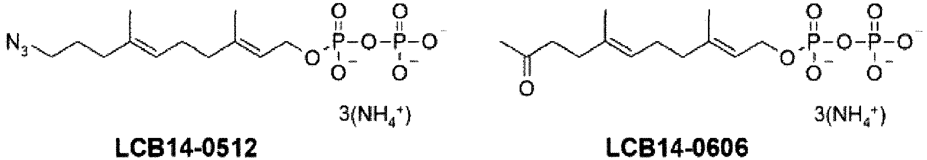
Step 1: Preparation of prenylated antibody
   A mixture for antibody prenylation reaction was prepared and reacted at 30°C for 16 hours. As the antibody, Herceptin-G7-CVIM (LC) used in Korean Patent Publication No. 10-2014-0035393 was used. The reaction mixture was composed of 24 µM antibody, 200 nM FTase (Calbiochem #344145), and a buffer (50 mM Tris-HCl (pH 7.4), 5 mM MgCl₂, 10 µM ZnCl₂, 0.25 mM DTT) containing 0.144 mM LCB14-0606 (in house, Korean Patent Publication No. 10-2014-0035393). After completion of the reaction, the prenylated antibody was desalted using G25 Sepharose column (AKTA purifier, GE healthcare) equilibrated with PBS buffer.
Step 2: Drug-conjugation method
   A mixture for oxime bond generation reaction between the prenylated antibody and the linker-drug was prepared by mixing 100 mM Na-acetate buffer pH 5.2, 10% DMSO, 24 µM antibody, and 240 µM linker-drug (in house, compound in Table 1), and gently stirred at 30°C. After 24 hours of reaction, excess small molecules were removed through FPLC (AKTA purifier, GE healthcare), and protein fractions were collected and concentrated.

**[Table 1]**

| | List of Anti-HER2 ADC prepared | | |
|---|---|---|---|
| Examples | | Linker-drug | ADC# |
| 6 | | Compound 28 | ADC1 |
| 13 | | Compound 48 | ADC2 |
| 19 | | Compound 64 | ADC3 |
| 24 | | Compound 81 | ADC4 |
| 28 | | Compound 97 | ADC5 |
| 31 | | Compound 105 | ADC6 |
| 36 | | Compound 124 | ADC7 |
| 40 | | Compound 144 | ADC8 |
| 46 | | Compound 170 | ADC9 |
| Control material | | | LCB14-0235 |

### <Experimental Example 1> In vitro cytotoxicity evaluation

The cell proliferation inhibitory activity of the drugs and ADCs presented in Tables 2 and 3 below on cancer cell lines was measured. As the cancer cell lines, commercially available human breast cancer cell lines MCF-7 (HER2 negative to normal), SK-BR3 (HER2 positive), and JIMT-1 (HER2 positive) were used. As control materials, SG2057 and LCB14-0235 in Table 1 using SG2057 as a toxin were used. In a 96-well plate, 5,000 SK-BR3, 5,000 MCF-7, and 2,500 JIMT-1 per well were seeded, cultured for 24 hours, and then treated with ADCs at a concentration of 25 fM to 10 nM (5-fold serial dilutions) and drugs at a concentration of 25 fM to 10 nM (5-fold serial dilutions) or 1 fM to 100 nM (10-fold serial dilutions). After 144 hours, the number of viable cells was quantified using SRB (Sulforhodamine B) dye.

**[Table 2] Comparison of cytotoxicity of pyrrolobenzodiazepine derivatives**

| Test samples | IC₅₀ (nM) | | |
|---|---|---|---|
| | SK-BR3 | JIMT-1 | MCF7 |
| SG2057 | 0.01 | 0.04 | 0.05 |
| Compound 32 | 18.2 | >100 | >100 |
| Compound 33 | >100 | >100 | >100 |
| Compound 52 | >100 | >100 | >100 |
| Compound 53 | 2.52 | 8.93 | 11. 8 |
| Compound 188 | 0.96 | 1.92 | - |

The structure of control material SG2057 is as follows.

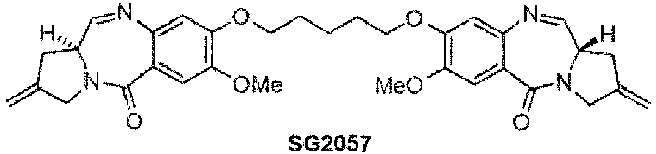

It was confirmed that the cytotoxicity of newly prepared pyrrolobenzodiazepines 32, 33, 52, 53, and 188 in SK-BR3, JIMT-1, and MCF7 breast cancer cell lines was significantly weaker than that of the control material, pyrrolobenzodiazepine (SG2057).

**[Table 3] Comparison of cytotoxicity of anti-HER2 ADCs**

| Test samples | IC₅₀ (nM) | | |
|---|---|---|---|
| | SK-BR3 | JIMT-1 | MCF7 |
| SG2057 | 0.02 | 0.05 | 0.06 |
| ADC1 | 0.01 | 0.24 | >10 |
| ADC2 | 0.03 | 2.53 | >10 |
| ADC3 | 0.01 | 3. 67 | >10 |
| ADC4 | 0.01 | 0.19 | 0.56 |
| ADC5 | 0.01 | 0.21 | >10 |
| ADC6 | 0.01 | 0.13 | >10 |
| ADC7 | 0.02 | 0.21 | 0.67 |
| ADC8 | 0.01 | 0.28 | >10 |
| ADC9 | 0.05 | >10 | >10 |
| LCB14-0235 | 0.01 | 0.17 | >10 |

The structure of linker-drug LCB20-0174 used in the control material ADC, LCB14-0235, is as follows. (LCB20-0174 was prepared by the method described in Korean Patent Publication No. 10-2018-0110645.)

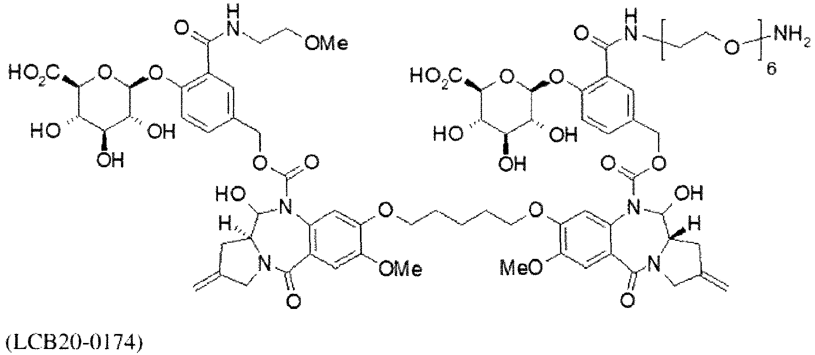

In SK-BR3 and JIMT-1 breast cancer cell lines, the cytotoxicity of ADC1 and 2, into which linker-drugs 28 and 48 among antibody-drug conjugates were introduced, exhibited remarkably excellent efficacy in an antigen-dependent manner than that of the newly prepared pyrrolobenzodiazepine derivatives 32 and 52 themselves. Other novel ADCs 1 to 9 including ADC1 and 2 had an IC50 of 0.01 to 0.05 nM in the SK-BR3 cell line and an IC50 of 0.13 nM to 10 nM or more in the JIMT-1 cell line in an antigen-dependent manner. It was confirmed that particularly ADC1, 5, 6, and 8 exhibited drug efficacy approximately equivalent to that of the control material, LCB14-0235.

### <Experimental Example 2> In vivo xenograft experiment

The efficacy of ADCs was evaluated using a JIMT-1 cell line (procured from Adexbio) which was resistant to Herceptin and T-DM1 and was a human breast cancer cell line that overexpressed HER2. The JIMT-1 cell line was mixed with Matrigel at a ratio of 1:1, and 2 × 10⁶ cells per mouse were subcutaneously administered to the right flank of 6-week-old female mice by 0.2 mL each. When the tumor size reached an average of 1 mm³, the mice were grouped, the test material was administered thereto through the tail vein at 10 mL/kg one time, and the tumor size and weight of the mice were measured two times a week.

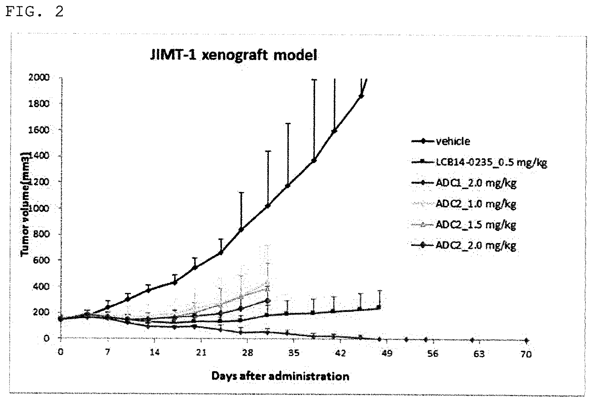

In the group in which ADC1 was administered at 2 mg/kg one time, tumors disappeared from all subjects after day 42, and excellent tumor inhibiting ability of ADC1 was confirmed (see FIG. 2).

### <Experimental Example 3> Rat pre-tox experiment

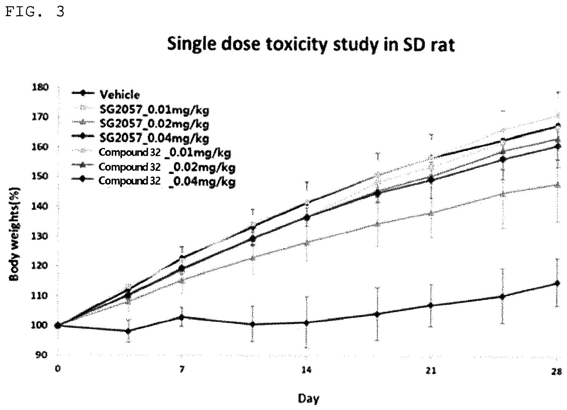

Twenty one 8-week-old male SD rats were divided into 7 groups of 3 rats each, the test material was administered thereto through the tail vein at 2 mL/kg one time, and the weight/death/clinical symptoms and the like thereof were observed. The body weight was measured two times a week, and death and clinical symptoms were observed daily. Four weeks after administration, planned slaughter was performed to examine the gross findings of organs. The results are as illustrated in FIG. 3.

When SG2057 and Compound 32 were administered at the same dosage, it was confirmed that SG2057 significantly inhibited weight gain in the 0.02 mg/kg and 0.04 mg/kg groups. At the end of the 4th week experiment, the body weight in the vehicle group increased to 167% as compared to the body weight at the time of administration, and the body weights of the SG2057-administered group increased to 167%, 148%, and 115%, respectively. On the other hand, the weight gain pattern of the Compound 32-administered group was similar to that of the control group, the body weights thereof increased to 171%, 164%, and 161%, respectively, and the inhibition of weight gain due to toxicity was confirmed to be very insignificant. This predicts that Compound 32 exhibits lower toxicity in SD rats than SG2057.

### <Experimental Example 4> Bystander effect experiment

The cell proliferation inhibitory activity of the drugs and ADC presented in Table 4 below on cancer cell lines was measured. The SK-BR3 human breast cancer cell line (procured from Korea Cell Line Bank (KCLB)) was used as a HER2-positive cell line, and the MDA-MB-468 human triple-negative breast cancer cell line (procured from ATCC) was used as a HER2-negative cell line. As control materials, MMAE was used as a free toxin already known to exhibit a bystander effect and MMAF known to exhibit no bystander effect was used. It was intended to examine whether toxin and ADC have a bystander effect by comparing SG2057 with a derivative of SG2057 and performing the treatment with each ADC containing the free toxin described above. All ADCs used in the experiment were anti-HER2 ADCs. In a 96-well plate, 6,000 SK-BR3, 6,000 MDA-MB-468, or 3,000 SK-BR3 and 3,000 MDA-MB-468 per well were seeded together, cultured for 24 hours, and then treated with each drug and ADC at a single concentration of 1 nM. After 144 hours, the number of viable cells was quantified using SRB (Sulforhodamine B) dye.

**[Table 4]**

| Test samples | Cell viability (%) | | |
|---|---|---|---|
| | SK-BR3 | MDA-MB-468 | Co-culture (SK-BR3:MDA-MB-468 = 1:1 |
| MMAF | 101 | 105 | 99 |
| MMAE | 12 | 14 | 12 |
| SG2057 | 7 | 4 | 6 |
| LCB14-0235 | 11 | 102 | 32 |
| Compound 32 | 98 | 101 | 98 |
| ADC1 | 11 | 102 | 71 |

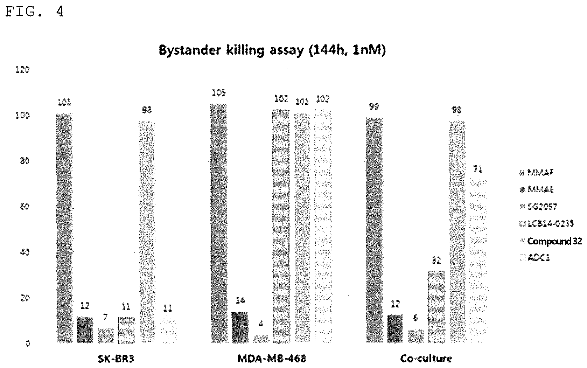

Unlike MMAE and SG2057, the newly synthesized Compound 32 did not exhibit cytotoxicity in both the SK-BR3 cell line and the MDA-MB-468 cell line and under a co-culture condition. This trend was similar to that of MMAF known to exhibit no bystander effect. ADC1 into which Compound 32 was introduced also exhibited cytotoxicity only in SK-BR3 depending on the level of antigen expression and exhibited only weak cytotoxicity under a co-culture condition, and the cell viability was confirmed to be 71%. On the other hand, LCB14-0235 into which SG2057 was introduced exhibited excellent cytotoxicity in SK-BR3, and the cell viability was 32% under a co-culture condition as well because of a bystander effect (see FIG. 4). The results indicate that Compound 32 and ADC1 do not exhibit a bystander effect.

### [Industrial Applicability]

The pyrrolobenzodiazepine dimer compound according to the present invention exhibits anticancer activity equivalent to or superior to that of existing anticancer agents when being applied to a ligand-drug conjugate as a drug and administered as well as exhibits low activity and greatly diminished toxicity in the free toxin form. The pyrrolobenzodiazepine dimer compound has a significantly improved therapeutic index and thus can be used in the field of treatment of proliferative diseases since targeting of proliferative diseases such as cancer is possible, specific treatment of the proliferative diseases is possible, the drug efficacy can be maximized, and the expression of side effects can be minimized.

## Claims

1. A pyrrolobenzodiazepine dimer compound having a structure represented by the following Chemical Formula I or a pharmaceutically acceptable salt or solvate thereof: 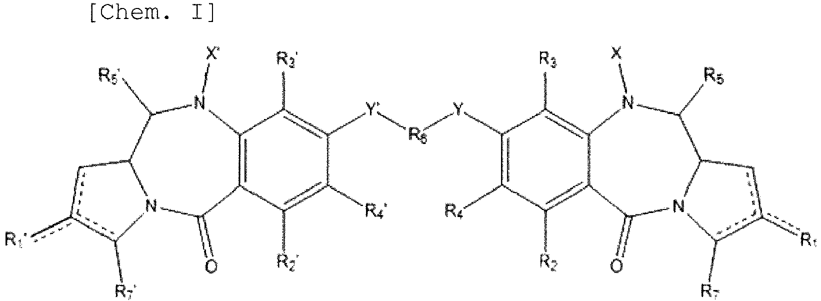 wherein
a dotted line indicates arbitrary presence of a double bond between C1 and C2 or between C2 and C3,
R₁ is selected from the group consisting of hydrogen, OH, =O, =CH₂, CN, R^{m}, OR^{m}, =CH-R^{m'} =C(R^{m'})₂, O-SO₂-R^{m}, CO₂R^{m}, COR^{m}, halo, and dihalo or may be one of those described in definition of R₆ below,
wherein R^{m} is selected from the group consisting of substituted or unsubstituted C₁₋₁₂ alkyl, substituted or unsubstituted C₂₋₁₂ alkenyl, substituted or unsubstituted C₂₋₁₂ alkynyl, substituted or unsubstituted C₅₋₂₀ aryl, substituted or unsubstituted C₅₋₂₀ heteroaryl, substituted or unsubstituted C₃₋₆ cycloalkyl, substituted or unsubstituted 3- to 7-membered heterocyclyl, substituted or unsubstituted 3- to 7-membered heterocycloalkyl, and substituted or unsubstituted 5- to 7-membered heteroaryl,
wherein when C₁₋₁₂ alkyl, C₂₋₁₂ alkenyl, C₂₋₁₂ alkynyl, C₅₋₂₀ aryl, C₅₋₂₀ heteroaryl, C₃₋₆ cycloalkyl, 3- to 7-membered heterocyclyl, 3- to 7-membered heterocycloalkyl, or 5- to 7-membered heteroaryl is substituted, hydrogen atoms of C₁₋₁₂ alkyl, C₁₋₁₂ alkoxy, C₂₋₁₂ alkenyl, C₂₋₁₂ alkynyl, C₅₋₂₀ aryl, C₅₋₂₀ heteroaryl, C₃₋₆ cycloalkyl, 3- to 7-membered heterocyclyl, 3- to 7-membered heterocycloalkyl, or 5- to 7-membered heteroaryl are each independently substituted with any one or more selected from the group consisting of C₁₋₁₂ alkyl, C₂₋₁₂ alkenyl, C₂₋₁₂ alkynyl, C₅₋₂₀ aryl, C₅₋₂₀ heteroaryl, C₃₋₆ cycloalkyl, 3-to 7-membered heterocyclyl, 3- to 7-membered heterocycloalkyl, and 5- to 7-membered heteroaryl, and
R^{m'} is selected from the group consisting of R^{m}, CO₂R^{m}, COR^{m}, CHO, CO₂H, and halo;
R₂, R₃, and R₅ are each independently selected from the group consisting of H, R^{m}, OH, OR^{m}, SH, SR^{m}, NH₂, NHR^{m}, NR^{m}R^{m'}, NO₂, Me₃Sn, and halo,
wherein R^{m} and R^{m'} are as defined above;
R₄ is selected from the group consisting of hydrogen, R^{m}, OH, OR^{m}, SH, SR^{m}, NH₂, NHR^{m}, NR^{m}R^{m'}, NO₂, Me₃Sn, halo, substituted or unsubstituted C₁₋₆ alkyl, substituted or unsubstituted C₁₋₆ alkoxy, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 7-membered heterocycloalkyl, substituted or unsubstituted C₅₋₁₂ aryl, substituted or unsubstituted 5- to 7-membered heteroaryl, -CN, -NCO, -ORⁿ, -OC(O)Rⁿ, -OC(O)NRⁿR^{n'}, -OS(O)R^{n,} -OS(O)₂Rⁿ, -SRⁿ, -S(O)Rⁿ, - S(O)₂Rⁿ, -S(O)NRⁿR^{n'}, -S(O)₂NRⁿR^{n'}, -OS(O)NRⁿR^{n'}, -OS(O)₂NRⁿR^{n'}, -NRⁿR^{n'}, -NRⁿC(O)R^{o}, -NRⁿC(O)OR^{o}, -NRⁿC(O)NR^{o}R^{o'}, -NRⁿS(O)R^{o}, -NRⁿS(O)₂R^{o}, -NRⁿS(O)NR^{o}R^{o'}, -NRⁿS(O)₂NR^{o}R^{o'}, -C(O)Rⁿ, - C(O)ORⁿ, and -C(O)NRⁿR^{n'},
wherein when C₁₋₆ alkyl, C₁₋₆ alkoxy, C₂₋₆ alkenyl, C₂₋₆ alkynyl, C₃₋₆ cycloalkyl, 3- to 7-membered heterocycloalkyl, C₅₋₁₂ aryl, or 5- to 7-membered heteroaryl is substituted, hydrogen atoms of C₁₋₆ alkyl, C₁₋₆ alkoxy, C₂₋₆ alkenyl, C₂₋₆ alkynyl, C₃₋₆ cycloalkyl, 3-to 7-membered heterocycloalkyl, C₅₋₁₂ aryl, or 5- to 7-membered heteroaryl may each be independently substituted with C₁₋₆ alkyl, C₁₋₆ alkoxy, C₂₋₆ alkenyl, C₂₋₆ alkynyl, C₃₋₆ cycloalkyl, 3- to 7-membered heterocycloalkyl, C₅₋₁₂ aryl, 5- to 7-membered heteroaryl, -ORP, -OC(O)R^{p}, -OC(O)NR^{p}R^{p'}, -OS(O)R^{p}, -OS(O)₂R^{p}, -SR^{p}, -S(O)R^{p}, -S(O)₂R^{p}, -S(O)NR^{p}R^{p'}, - S(O)₂NR^{p}R^{p'}, -OS(O)NR^{p}R^{p'}, -OS(O)₂NR^{p}R^{p'}, -NR^{p}R^{p'}, -NR^{p}C(O)R^{q}, -NR^{p}C(O)OR^{x}, -NR^{p}C(O)NR^{x}R^{x'}, -NR^{p}S(O)R^{x}, -NR^{p}S(O)₂R^{x}, - NR^{p}S(O)NR^{x}R^{x'}, -NR^{p}S(O)₂NR^{x}R^{x'}, -C(O)R^{p}, -C(O)OR^{p}, or - C(O)NR^{p}R^{p},
wherein Rⁿ, R^{o}, R^{p}, R^{x}, R^{n'}, R^{o'}, R^{p'}, and R^{x'} are each independently selected from the group consisting of H, C₁₋₇ alkyl, C₂₋₇ alkenyl, C₂₋₇ alkynyl, C₃₋₁₃ cycloalkyl, 3- to 7-membered heterocycloalkyl, C₆₋₁₀ aryl, and 5- to 7-membered heteroaryl, or
R₄ may be one of those described in definition of R₆ below;
any one selected from the group consisting of - C(O)O*, -S(O)O*, -C(O)*, -C(O)NR*, -S(O)₂NR*, -P(O)R'NR*, S(O)NR*, and -PO₂NR* groups is independently attached to each of X and X',
wherein * is a portion to which a linker is attached, and
R and R' are each independently H, OH, N₃, CN, NO₂, SH, NH₂, ONH₂, NHNH₂, halo, substituted or unsubstituted C₁₋₈ alkyl, substituted or unsubstituted C₃₋₈ cycloalkyl, substituted or unsubstituted C₁₋₈ alkoxy, substituted or unsubstituted C₁₋₈ alkylthio, substituted or unsubstituted C₃₋₂₀ heteroaryl, substituted or unsubstituted C₅₋₂₀ aryl, or mono- or di-C₁₋₈ alkylamino;
wherein C₁₋₈ alkyl, C₃₋₈ cycloalkyl, C₁₋₈ alkoxy, C₁₋₈ alkylthio, C₃₋₂₀ heteroaryl, or C₅₋₂₀ aryl is substituted with a substituent selected from the group consisting of H, OH, N₃, CN, NO₂, SH, NH₂, ONH₂, NNH₂, halo, C₁₋₆ alkyl, C₁₋₆ alkoxy, and C₅₋₁₂ aryl when substituted;
Y and Y' are each independently selected from the group consisting of O, S, and N(H);
R₆ is a polar functional group having a structure represented by the following Chemical Formula II,
[Chem. II] -A-B₁-B₂-C
wherein
A is C₁₋₆ alkyl, C₂₋₆ alkenyl, C₂₋₆ alkynyl, C₃₋₂₀ aryl, C₃₋₈ cycloalkyl, 3- to 7-membered-heterocycloalkyl, or 5-to 7-membered-heteroaryl that is unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl or siloxy,
B₁ is a bond, -O-, -NH-, -S-, -CO₂, -CONR^{m}-, -CONR^{m}R^{m'}, -CH=CH-, -C≡C-, -N-, or -P- or may be a branching unit,
wherein the branching unit is C₂₋₁₀₀ alkenyl (wherein carbon atoms of alkenyl may be substituted with one or more heteroatoms selected from the group consisting of N, O, and S, and alkenyl may be further substituted with one or more C₁₋₂₀ alkyl), a hydrophilic amino acid, -C(O)-, - C(O)NR"-, -C(O)O-, -(CH₂)ₛ-NHC(O)-(CH₂)ₜ-, -(CH₂)ᵤ-C(O)NH-(CH₂)ᵥ-, -(CH₂)ₛ-NHC(O)-(CH₂)ₜ-C(O)-, -(CH₂)ᵤ-C(O)NH-(CH₂)ᵥ-C(O)-, -S(O)₂NR"-, -P(O)R‴NR-,-S(O)NR""-, or -PO₂NR- (wherein R"" and R""' are each independently H, C₁₋₈ alkyl, C₃₋₈ cycloalkyl, C₁₋₈ alkoxy, C₁₋₈ alkylthio, mono- or di-C₁₋₈ alkylamino, C₃₋₂₀ heteroaryl, or C₅₋₂₀ aryl; and s, t, u, and v are each independently an integer from 0 to 10),
B₂ is a bond or has a structure of -(CH₂)ᵣ(V(CH₂)ₚ)_{q}-, -((CH₂)ₚV)_{q}-, -V(Y(CH₂)ₚ)_{q}-, -(CH₂)ᵣ(V(CH₂)ₚ)_{q}Y-, - ((CH₂)ₚV)_{q}(CH₂)ᵣ-, -Y((CH₂)ₚV)_{q}-, or -(CH₂)ᵣ(V(CH₂)ₚ)_{q}YCH₂-, wherein r is an integer from 0 to 10, p is an integer from 1 to 10, q is an integer from 1 to 20, V and Y are each independently a single bond, -O-, -S-, -NR₂₁-, - C(O)NR₂₂-, -NR₂₃C(O)-, -NR₂₃C(O)R₂₄-, -NR₂₄SO₂-, or -SO₂NR₂₅-, and R₂₁ to R₂₅ are each independently hydrogen, (C₁₋₆) alkylene, (C₁₋₆) alkyl, (C₁₋₆) alkyl (C₆₋₂₀) aryl, or (C₁₋₆) alkyl (C₃₋₂₀) heteroaryl, and
C is selected from the group consisting of -R^{q}OR^{r}, - OR^{r}, -OC(O)OR^{r}, -R^{q}OC(O)OR^{r}, -C(O)OR^{r}, -R^{q}C(O)OR^{r}, -C(O)R^{r}, -R^{q}C(O)R^{r}, -OC(O)R^{r}, -R^{q}OC(O)R^{r}, -(R^{q}O)ₙ-OR^{r}, -(OR^{q})ₙ-OR^{r}, - C(O)-O-C(O)R^{r}, -R^{q}C(O)-O-C(O)R^{r}, -SR^{r}, -R^{q}SR^{r}, -SSR^{r}, - R^{q}SSR^{r}, -S(=O)R^{r}, -R^{q}S(=O)R^{r}, -R^{q}C(=S)R^{r}-, -R^{q}C(=S)SR^{r}, - R^{q}SO₃R^{r}, -SO₃R^{r}, -CN, -R^{q}CN, 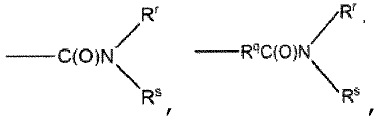 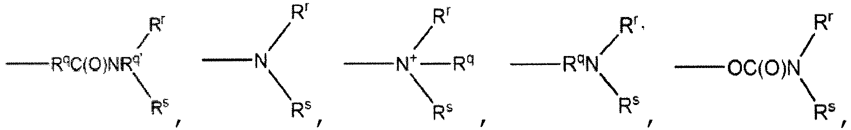 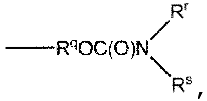 -SO₂R^{q}, -R^{q}SO₂R^{r}, -OSO₂R^{r}, -R^{q}OSO₂R^{r}, -OSO₂OR^{r}, -R^{q}OSO₂OR^{r}, 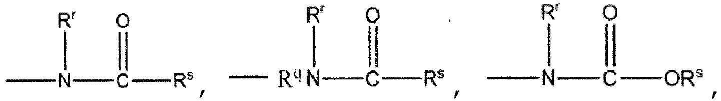 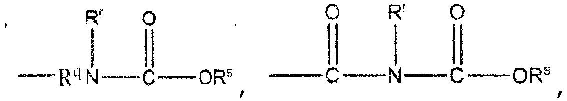 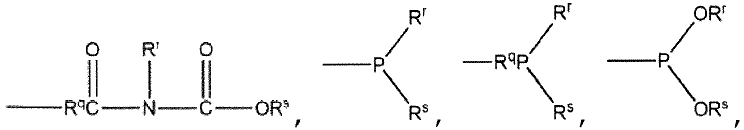 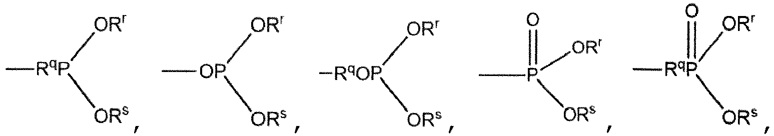 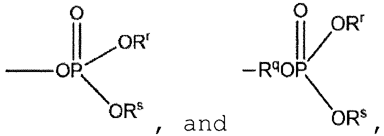
wherein R^{q} and R^{q'} are the same as or different from each other and are each independently C₁₋₂₀ linear or branched alkylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy;
C₂₋₂₀ linear or branched alkenylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy;
C₂₋₂₀ linear or branched alkynylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy;
C₃₋₁₂ cycloalkylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy;
C₆₋₄₀ arylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy;
C₁₋₂₀ alkoxylylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy; or
C₁₋₂₀ carbonyloxylene unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carbonyloxy, halocarbonyloxy, aryloxy, haloaryloxy, silyl, or siloxy;
R^{r} and R^{s} are the same as or different from each other and are each independently hydrogen; halogen;
C₁₋₂₀ linear or branched alkyl unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy;
C₂₋₂₀ linear or branched alkenyl unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy;
C₂₋₂₀ linear or branched alkynyl unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy;
C₃₋₁₂ cycloalkyl unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy;
C₆₋₄₀ aryl unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy;
C₁₋₂₀ alkoxy unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy; or
C₁₋₂₀ carbonyloxy unsubstituted or substituted with one or more substituents selected from halogen, alkyl, alkenyl, alkynyl, haloalkyl, haloalkenyl, haloalkynyl, aryl, haloaryl, aralkyl, haloaralkyl, alkoxy, haloalkoxy, carboxyl, carbonyloxy, halocarbonyloxy, aryloxy, haloaryloxy, silyl, or siloxy, and
n is each independently an integer from 1 to 10; and
R₇ is H, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ cycloalkyl, substituted or unsubstituted 3- to 7-membered heterocycloalkyl, substituted or unsubstituted C₆₋₁₀ aryl, substituted or unsubstituted 5- to 7-membered heteroaryl, -OR^{t}, -OC(O)R^{t}, -OC(O)NR^{t}R^{t'}, -OS(O)R^{t}, - OS(O)₂R^{t}, -SR^{t}, -S(O)R^{t}, -S(O)₂R^{t}, -S(O)NR^{t}R^{t'}, -S(O)₂NR^{r}R^{r'}, -OS(O)NR^{t}R^{t'}, -OS(O)₂NR^{t}R^{t'}, -NR^{t}R^{t'}, -NR^{t}C(O)R^{u}, -NR^{t}C(O)OR^{u}, -NR^{t}C(O)NR^{u}R^{u'}, -NR^{t}S(O)R^{u}, -NR^{t}S(O)₂R^{u}, -NR^{t}S(O)NR^{u}R^{u'}, - NR^{t}S(O)₂NR^{u}R^{u}, -C(O)R^{t}, -C(O)OR^{u}, or -C(O)NR^{t}R^{t'},
wherein when C₁₋₆ alkyl, C₂₋₆ alkenyl, C₂₋₆ alkynyl, C₃₋₆ cycloalkyl, 3- to 7-membered heterocycloalkyl, C₆₋₁₀ aryl, or 5- to 7-membered heteroaryl is substituted, hydrogen atoms of C₁₋₆ alkyl, C₂₋₆ alkenyl, C₂₋₆ alkynyl, C₃₋₆ cycloalkyl, 3- to 7-membered heterocycloalkyl, C₆₋₁₀ aryl, or 5- to 7-membered heteroaryl are each independently substituted with C₁₋₆ alkyl, C₂₋₆ alkenyl, C₂₋₆ alkynyl, C₃₋₆ cycloalkyl, 3- to 7-membered heterocycloalkyl, C₆₋₁₀ aryl, 5- to 7-membered heteroaryl, -OR^{v}, -OC(O)R^{v}, -OC(O)NR^{v}R^{v'}, -OS(O)R^{v}, -OS(O)₂R^{v}, -SR^{v}, -S(O)R^{v}, -S(O)₂R^{v}, -S(O)NR^{v}R^{v'}, - S(O)₂NR^{v}R^{v'}, -OS(O)NR^{v}R^{v'}, -OS(O)₂NR^{v}R^{v'}, -NR^{v}R^{v'}, -NR^{v}C(O)R^{w}, -NR^{v}C(O)OR^{w}, -NR^{v}, C(O)NR^{w}R^{w'}, -NR^{v}S(O)R^{w}, -NR^{v}S(O)₂R^{w},-NR^{v}S(O)NR^{w}R^{w'}, -NR^{v}S(O)₂NR^{w}R^{w'}, -C(O)R^{v}, -C(O)OR^{v}, or-C(O)NR^{v}R^{v'},
wherein R^{t}, R^{t'}, R^{u}, R^{u'}, R^{v}, R^{v'}, R^{w}, and R^{w'} are each independently selected from the group consisting of H, C₁₋₇ alkyl, C₂₋₇ alkenyl, C₂₋₇ alkynyl, C₃₋₁₃ cycloalkyl, 3- to 7-membered heterocycloalkyl, C₅₋₁₀ aryl, and 5- to 7-membered heteroaryl.

2. The pyrrolobenzodiazepine dimer compound or a pharmaceutically acceptable salt or solvate thereof according to claim 1, wherein R₁ is selected from the group consisting of H, OH, =O, =CH₂, CN, and C₁₋₆ alkyl.

3. The pyrrolobenzodiazepine dimer compound or a pharmaceutically acceptable salt or solvate thereof according to claim 1, wherein R₂, R₃, and R₅ are each independently selected from the group consisting of H, OH, and OR^{m}, wherein R^{m} is as defined in claim 1.

4. The pyrrolobenzodiazepine dimer compound or a pharmaceutically acceptable salt or solvate thereof according to claim 1, wherein R₄ is selected from the group consisting of H, R^{m}, OH, and OR^{m}, wherein R^{m} is C₁₋₆ alkyl.

5. The pyrrolobenzodiazepine dimer compound or a pharmaceutically acceptable salt or solvate thereof according to claim 1, wherein Y and Y' are O.

6. The pyrrolobenzodiazepine dimer compound or a pharmaceutically acceptable salt or solvate thereof according to claim 1, wherein R₆ has a structure represented by the following Chemical Formula II:
[Chem. II] -A-B₁-B₂-C
wherein
A is C₁₋₆ alkyl or C₃₋₂₀ aryl;
B₁ is selected from the group consisting of -O-, - NH-, -C≡C-, and -CONR^{m}R^{m'}, wherein R^{m} and R^{m'} are each independently hydrogen or C₁₋₆ alkyl;
B₂ is -((CH₂)ₚV)_{q}- or -V(Y(CH₂)ₚ)_{q}-,
wherein V is O or -NR₂₃C(O)R₂₄- (wherein R₂₃ and R₂₄ are each independently hydrogen or C₁₋₆ alkylene),
p is 2, and
q is an integer from 1 to 10; and
C is R^{q}C(O)OR^{r} or 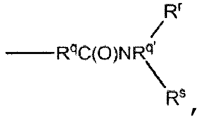
wherein R^{q} and R^{q'} are each independently C₁₋₆ alkylene, C₂₋₆ alkenylene, or C₂₋₆ alkynylene, and
R^{r} and R^{s} are each independently selected from the group consisting of hydrogen, carboxyl, C₁₋₆ alkyl substituted with carboxyl, and C₁₋₆ alkyl.

7. The pyrrolobenzodiazepine dimer compound or a pharmaceutically acceptable salt or solvate thereof according to claim 1, wherein R₇ is hydrogen, C₁₋₆ alkyl, or OR^{t} (wherein R^{t} is H or C₁₋₇ alkyl).

8. The pyrrolobenzodiazepine dimer compound or a pharmaceutically acceptable salt or solvate thereof according to claim 1, wherein X and X' are each independently selected from the group consisting of - C(O)O*, -C(O)*, and -C(O)NR*, wherein each R is independently H, OH, N₃, CN, NO₂, SH, NH₂, ONH₂, NNH₂, halo, C₁₋₃ alkyl, or C₁₋₃ alkoxy.

9. The pyrrolobenzodiazepine dimer compound or a pharmaceutically acceptable salt or solvate thereof according to claim 1, wherein the pyrrolobenzodiazepine dimer compound is one selected from the group consisting 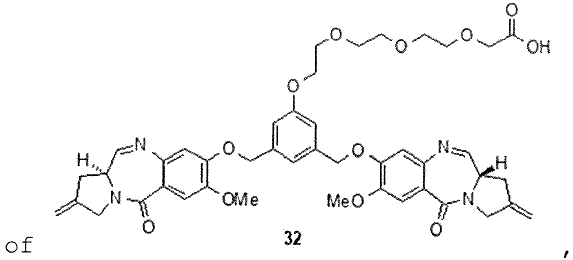 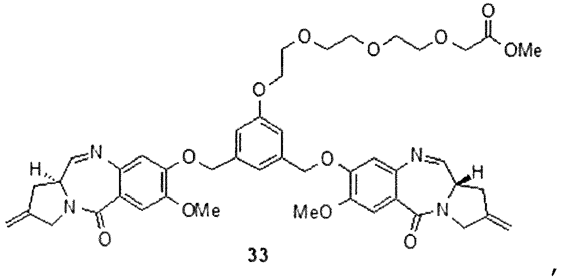 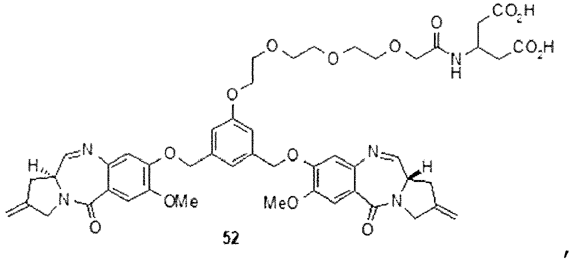 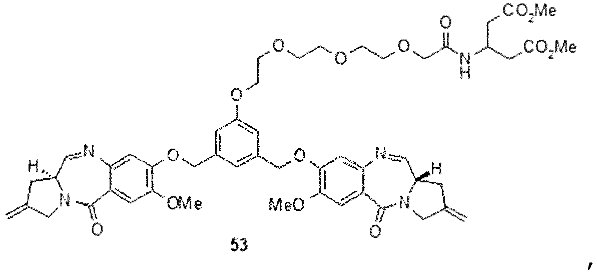 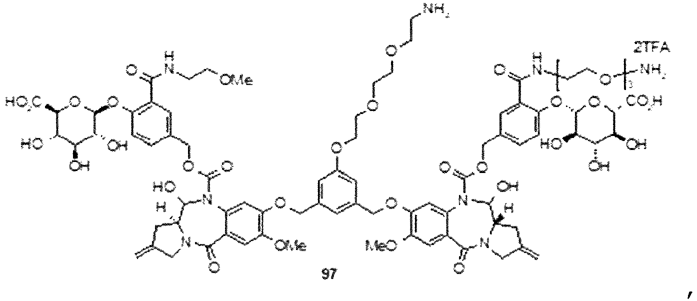 and

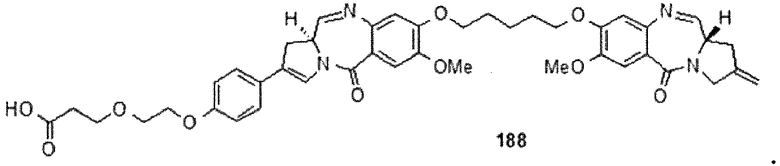

10. A conjugate having a structure represented by the following Chemical Formula III or a pharmaceutically acceptable salt or solvate thereof:
[Chem. III] **Ligand** - **(L** - **D)ₙ**
wherein
**Ligand** is a ligand;
L is a linker;
D is the pyrrolobenzodiazepine dimer compound according to any one of claims 1 to 9,
wherein the linker is bound to D through N10 position, N10' position, or N10 and N10' positions of D; and
n is an integer from 1 to 20.

11. The conjugate or a pharmaceutically acceptable salt or solvate thereof according to claim 10, wherein the linker is bound to the compound according to any one of claims 1 to 9 through X and X' of the compound.

12. The conjugate or a pharmaceutically acceptable salt or solvate thereof according to claim 10, wherein n is an integer from 1 to 10.

13. A pyrrolobenzodiazepine dimer-linker compound having a structure represented by the following Chemical Formula IV or a pharmaceutically acceptable salt or solvate thereof: 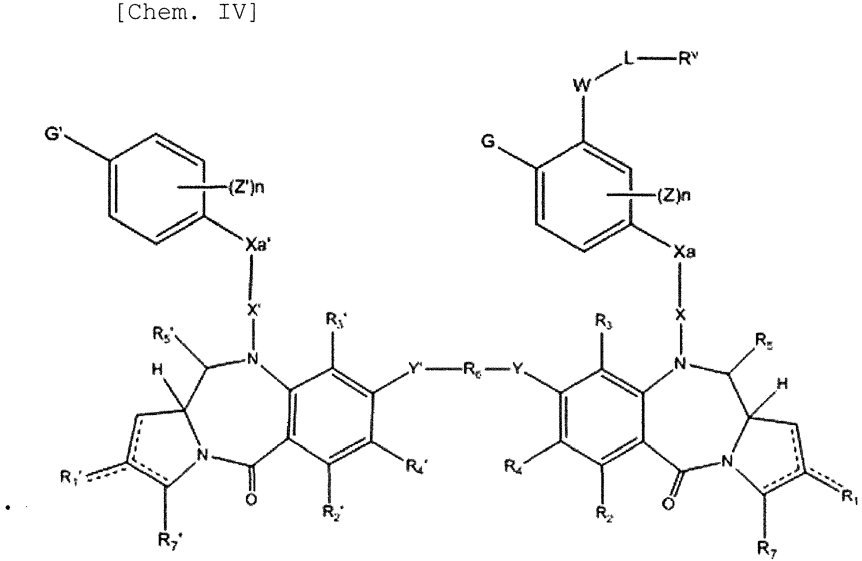 wherein
a dotted line, R₁, R₂, R₃, R₄, R₅, R₆, R₇, X, Y, R₁' , R₂' , R₃', R₄', R₅', R₇', X', and Y' are each as defined for those of the compound represented by Chemical Formula I in claim 1;
Xa and Xa' are each independently a bond or substituted or unsubstituted C₁₋₆ alkylene, wherein C₁₋₆ alkylene is substituted with hydrogen, C₁₋₈ alkyl, or C₃₋₈ cycloalkyl when substituted;
G and G' is a glucuronide group, a galactoside group, or a derivative thereof;
Z is selected from the group consisting of H, C₁₋₈ alkyl, halo, NO₂, CN, 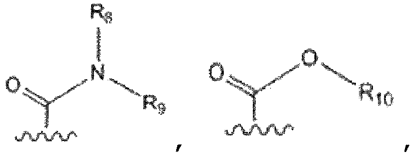 and - (CH₂)ₘ-OCH₃;
n is an integer from 1 to 3, and each Z may be the same as or different from each other when n is an integer 2 or more;
W is -C(O)-, -C(O)NR"-, -C(O)O-, -S(O)₂NR"-,-P(O)R′NR-, -S(O)NR"-, or -PO₂NR"-, wherein R" and R"′ are each independently H, C₁₋₈ alkyl, C₃₋₈ cycloalkyl, C₁₋₈ alkoxy, C₁₋₈ alkylthio, mono- or di-C₁₋₈ alkylamino, C₃₋₂₀ heteroaryl, or C₆₋₂₀ aryl;
L is one or more units selected from the group consisting of a branching unit, a connection unit, and a binding unit, or a combination of these units,
wherein the connection unit connects W with a binding unit, W with a branching unit, a branching unit with another branching unit, or a branching unit with a binding unit, the branching unit connects a connection unit with W or a connection unit with another connection unit,
the branching unit is C₂₋₁₀₀ alkenyl (wherein carbon atoms of alkenyl may be substituted with one or more heteroatoms selected from the group consisting of N, O, and S, and alkenyl may be further substituted with one or more C₁₋₂₀ alkyl), a hydrophilic amino acid, -C(O)-, - C(O)NR"-, -C(O)O-, -(CH₂)ₛ-NHC(O)-(CH₂)ₜ-, -(CH₂₎ᵤ-C(O)NH-(CH₂)ᵥ-, -(CH₂)ₛ-NHC(O)-(CH₂)ₜ-C(O)-, -(CH₂)ᵤ-C(O)NH-(CH₂)ᵥ-C(O)-, -S(O)₂NR"-, -P(O)R"'NR""-,-S(O)NR""-, or -PO₂NR""- (wherein R"" and R""' are each independently H, C₁₋₈ alkyl, C₃₋₈ cycloalkyl, C₁₋₈ alkoxy, C₁₋₈ alkylthio, mono- or di-C₁₋₈ alkylamino, C₃₋₂₀ heteroaryl, or C₅₋₂₀ aryl; and s, t, u, and v are each independently an integer from 0 to 10),
the connection unit is -(CH₂)ᵣ(V(CH₂)ₚ)_{q}-, wherein r is an integer from 0 to 10, p is an integer from 0 to 12, q is an integer from 1 to 20, V is a single bond, -O-, or -S-, and
the binding unit is 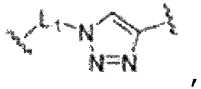 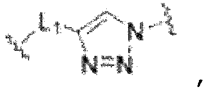 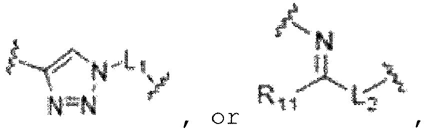 wherein L₁ is a single bond or C₂₋₃₀ alkenyl, R₁₁ is H or C₁₋₁₀ alkyl, and L₂ is C₂₋₃₀ alkenyl; and
R^{v} is -NH₂, N₃, substituted or unsubstituted C₁₋₁₂ alkyl, C₁₋₁₂ alkynyl, C₁₋₃ alkoxy, substituted or unsubstituted C₃₋₂₀ heteroaryl, C₃₋₂₀ heterocyclyl, or substituted or unsubstituted C₅₋₂₀ aryl,
wherein when C₁₋₁₂ alkyl, C₃₋₂₀ heteroaryl, C₃₋₂₀ heterocyclyl, or C₅₋₂₀ aryl is substituted, one or more hydrogen atoms present in C₃₋₂₀ heteroaryl, C₃₋₂₀ heterocyclyl, or C₅₋₂₀ aryl are each independently substituted with OH, =O, halo, C₁₋₆ alkyl, C₁₋₆ alkoxy, C₂₋₆ alkenyl oxy, carboxy, C₁₋₆ alkoxycarbonyl, C₁₋₆ alkylcarbonyl, formyl, C₃₋₈ aryl, C₅₋₁₂ aryloxy, C₅₋₁₂ arylcarbonyl, or C₃₋₆ heteroaryl.

14. The pyrrolobenzodiazepine dimer-linker compound or a pharmaceutically acceptable salt or solvate thereof according to claim 13, wherein Xa and Xa' are each independently a bond or C₁₋₃ alkyl.

15. The pyrrolobenzodiazepine dimer-linker compound or a pharmaceutically acceptable salt or solvate thereof according to claim 13,
wherein Z is selected from the group consisting of H, 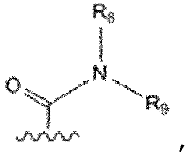 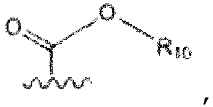 and -(CH₂)ₘ-OCH₃,
wherein R₈, R₉, and R₁₀ are each independently selected from the group consisting of H, C₁₋₃ alkyl, and C₁₋₃ alkoxy and m is 1 to 6.

16. The pyrrolobenzodiazepine dimer-linker compound or a pharmaceutically acceptable salt or solvate thereof according to claim 13, wherein W is -C(O)-, -C(O)NR‴-, or -C(O)O-, wherein R‴ is H or C₁₋₈ alkyl.

17. The pyrrolobenzodiazepine dimer-linker compound or a pharmaceutically acceptable salt or solvate thereof according to claim 13,
wherein L is one or more units selected from the group consisting of a branching unit, a connection unit, and a binding unit, or a combination of these units,
wherein the connection unit connects W with a binding unit, W with a branching unit, a branching unit with another branching unit, or a branching unit with a binding unit, the branching unit connects a connection unit with W or a connection unit with another connection unit,
the branching unit is C₂₋₁₀₀ alkenyl (wherein carbon atoms of alkenyl may be substituted with one or more heteroatoms selected from the group consisting of N, O, and S, and alkenyl may be further substituted with one or more C₁₋₆ alkyl), a hydrophilic amino acid, -C(O)-, - C(O)NR""-, -C(O)O-, -(CH₂)ₛ-NHC(O)-(CH₂)ₜ-, -(CH₂)ᵤ-C(O)NH-(CH₂)ᵥ-, -(CH₂)ₛ-NHC(O)-(CH₂)ₜ-C(O)-, or -(CH₂)ᵤ-C(O)NH-(CH₂)ᵥ-C(O)- (wherein R"" is H, C₁₋₈ alkyl, C₃₋₈ cycloalkyl, C₁₋₈ alkoxy, C₁₋₈ alkylthio, mono- or di-C₁₋₈ alkylamino, C₃₋₂₀ heteroaryl, or C₅₋₂₀ aryl; and s, t, u, and v are each independently an integer from 0 to 5),
the connection unit is -(CH₂)ᵣ(V(CH₂)ₚ)_{q}-, wherein r is an integer from 0 to 10, p is an integer from 0 to 12, q is an integer from 1 to 20, V is a single bond or -O-,
the binding unit is 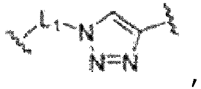 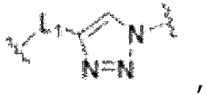 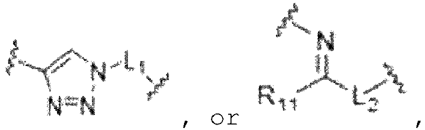 wherein L₁ is a single bond or C₂₋₈ alkenyl, R₁₁ is H or C₁₋₆ alkyl, and L₂ is C₂₋₈ alkenyl, and
the connection unit is -(CH₂)ᵣ (V(CH₂)ₚ)_{q}-, wherein r is an integer from 0 to 8, p is an integer from 1 to 12, q is an integer from 1 to 10, and V is a single bond or - O-.

18. The pyrrolobenzodiazepine dimer-linker compound or a pharmaceutically acceptable salt or solvate thereof according to claim 13, wherein the pyrrolobenzodiazepine dimer-linker compound is one selected from the group consisting of 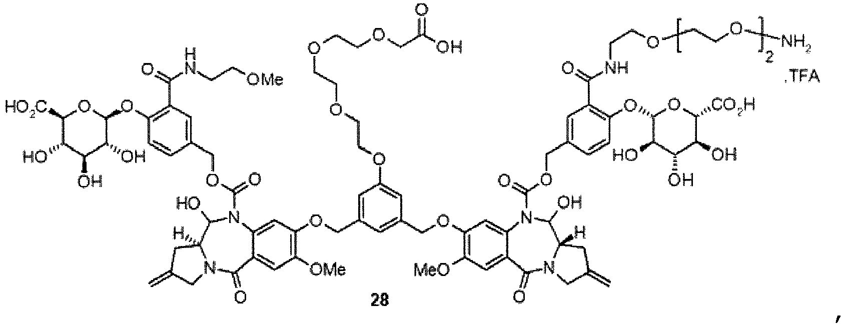 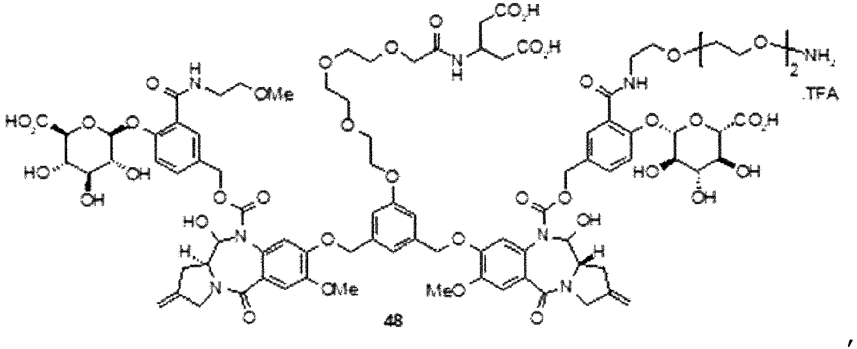 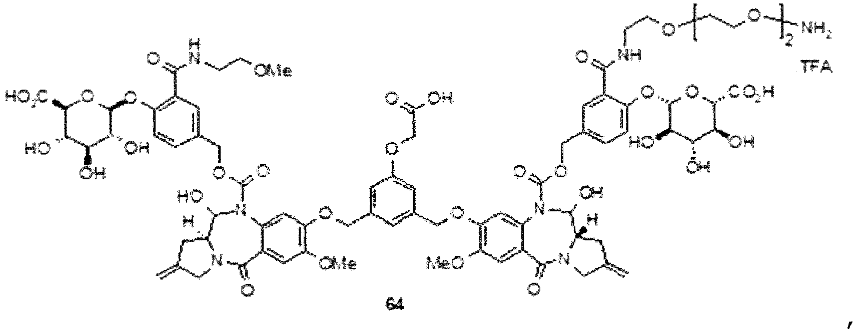 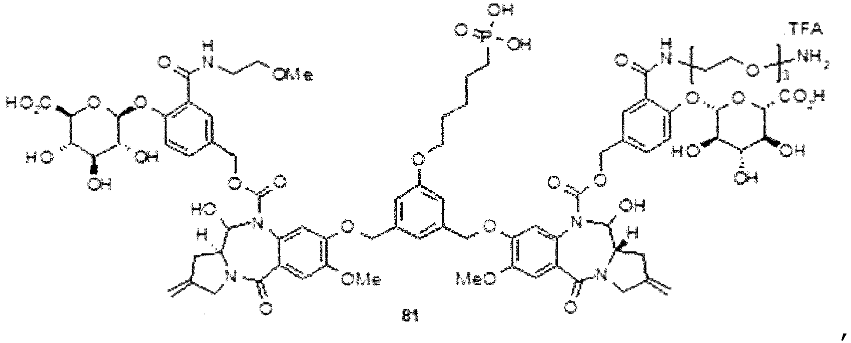 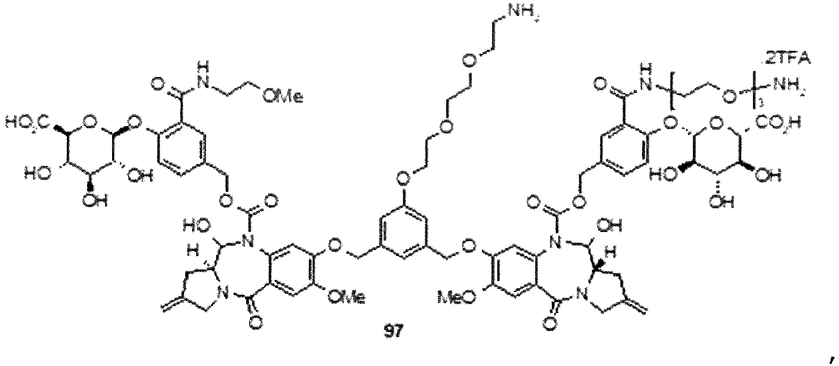 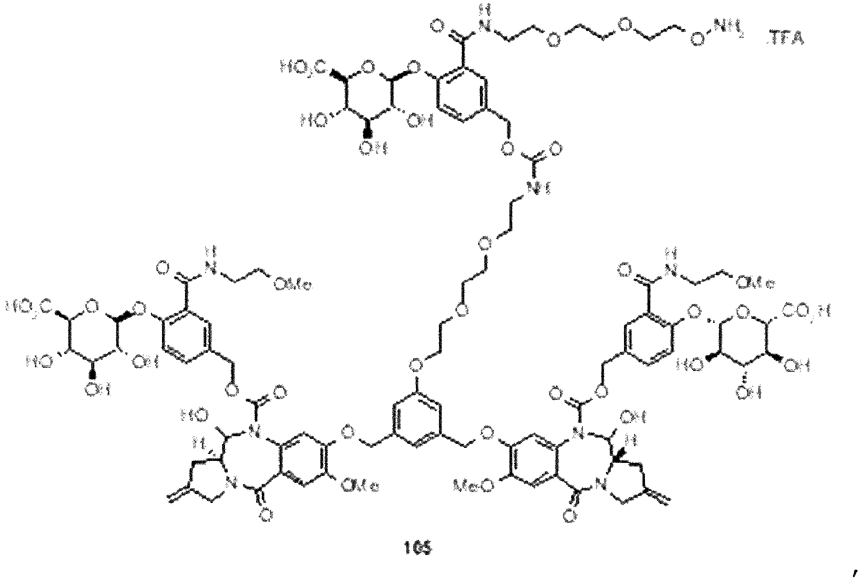 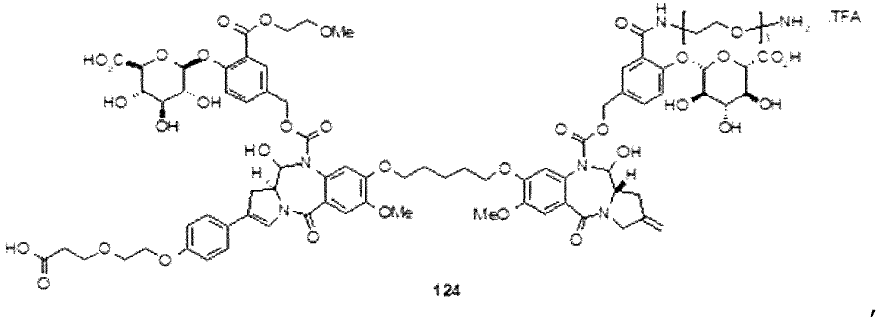 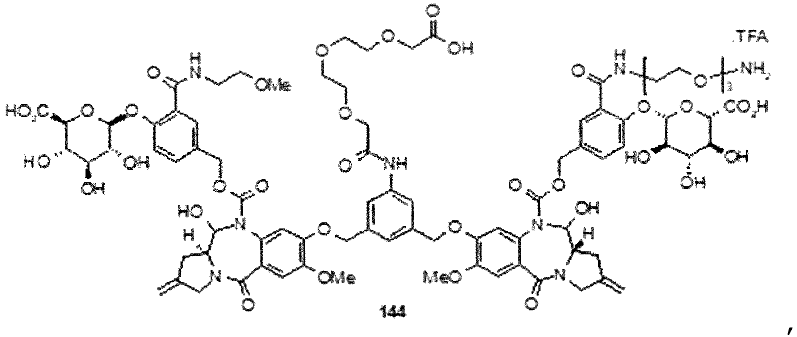 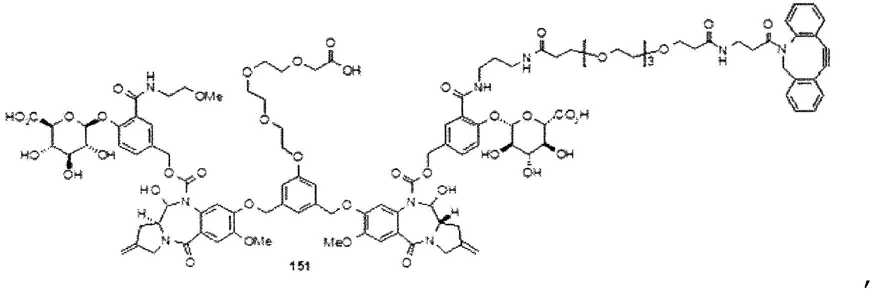 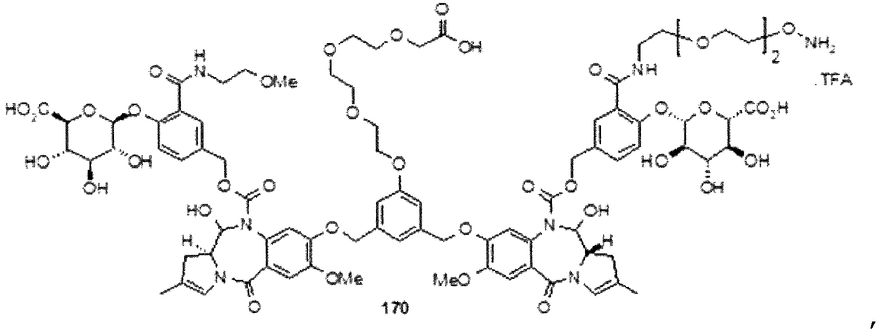 and

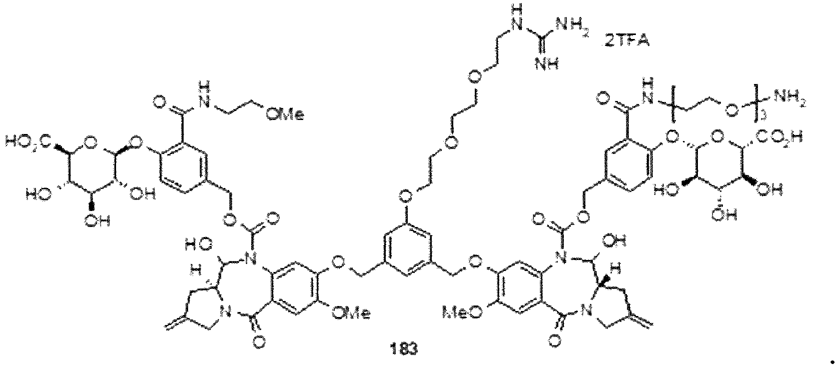

19. A pyrrolobenzodiazepine dimer-linker-ligand conjugate having a structure represented by the following Chemical Formula V or a pharmaceutically acceptable salt or solvate thereof: 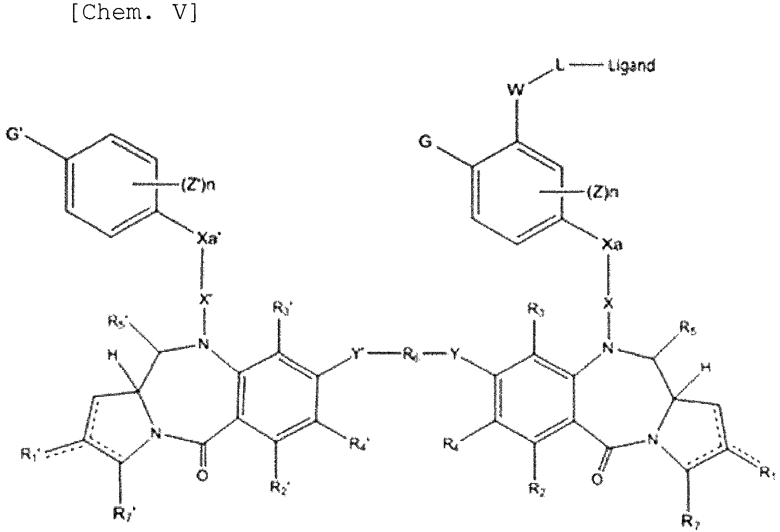 wherein
a dotted line, R₁, R₂, R₃, R₄, R₅, R₆, R₇, X, Y, R₁' , R₂', R₃', R₄', R₅', R₇', X', and Y' are each as defined for those of the compound represented by Chemical Formula I in claim 1;
Xa, G, Z, W, L, Xa', G', and Z' are each as defined for those of the compound represented by Chemical Formula IV in claim 13, and
Ligand is an antigen binding moiety.

20. The pyrrolobenzodiazepine dimer-linker-ligand conjugate or a pharmaceutically acceptable salt or solvate thereof according to claim 19, wherein Ligand is a protein.

21. The pyrrolobenzodiazepine dimer-linker-ligand conjugate or a pharmaceutically acceptable salt or solvate thereof according to claim 19, wherein Ligand is an antibody.

22. The pyrrolobenzodiazepine dimer-linker-ligand conjugate or a pharmaceutically acceptable salt or solvate thereof according to claim 21, wherein the antibody is selected from the group consisting of an anti-HER2 antibody, an anti-DLK1 antibody, an anti-ROR1 antibody, an anti-MUC1 antibody, an antibody CD19 antibody, and an anti-CD276 antibody.

23. The pyrrolobenzodiazepine dimer-linker-ligand conjugate or a pharmaceutically acceptable salt or solvate thereof according to claim 20, wherein the protein has one or more amino acid motifs recognizable by isoprenoid transferase.

24. The pyrrolobenzodiazepine dimer-linker-ligand conjugate or a pharmaceutically acceptable salt or solvate thereof according to claim 23, wherein the isoprenoid transferase is FTase (farnesyl protein transferase) or GGTase (geranylgeranyl transferase).

25. The pyrrolobenzodiazepine dimer-linker-ligand conjugate or a pharmaceutically acceptable salt or solvate thereof according to claim 23, wherein the amino acid motif is CYYX, XXCC, XCXC, or CXX,
wherein C is cysteine, Y is an aliphatic amino acid, and X is an amino acid that determines substrate specificity of isoprenoid transferase.

26. A pharmaceutical composition for prevention or treatment of a proliferative disease, which comprises the pyrrolobenzodiazepine dimer-linker-ligand conjugate according to any one of claims 19 to 25 or a pharmaceutically acceptable salt or solvate thereof.

27. The pharmaceutical composition for prevention or treatment of a proliferative disease according to claim 26, wherein the proliferative disease is selected from the group consisting of neoplasm, tumor, cancer, leukemia, psoriasis, bone disease, fibroproliferative disorder, and atherosclerosis.

28. The pharmaceutical composition for prevention or treatment of a proliferative disease according to claim 27, wherein the cancer is selected from the group consisting of lung cancer, small cell lung cancer, gastrointestinal cancer, colorectal cancer, bowel cancer, breast cancer, ovarian cancer, prostate cancer, testicular cancer, liver cancer, kidney cancer, bladder cancer, pancreatic cancer, brain cancer, sarcoma, osteosarcoma, Kaposi's sarcoma, and melanoma.

29. A pharmaceutical composition for prevention or treatment of a proliferative disease, which comprises:
the pyrrolobenzodiazepine dimer-linker-ligand conjugate according to any one of claims 19 to 25 or a pharmaceutically acceptable salt or solvate thereof; and
a pharmaceutically acceptable excipient.

30. A pharmaceutical composition for prevention or treatment of a proliferative disease, which comprises:
the pyrrolobenzodiazepine dimer-linker-ligand conjugate according to any one of claims 19 to 25 or a pharmaceutically acceptable salt or solvate thereof;
one or more therapeutic co-agents; and
a pharmaceutically acceptable excipient.

31. Use of the pyrrolobenzodiazepine dimer-linker-ligand conjugate according to any one of claims 19 to 25 or a pharmaceutically acceptable salt or solvate thereof for treatment of a proliferative disease.

32. The use according to claim 31, wherein the proliferative disease is selected from the group consisting of neoplasm, tumor, cancer, leukemia, psoriasis, bone disease, fibroproliferative disorder, and atherosclerosis.

33. The use according to claim 32, wherein the cancer is selected from the group consisting of lung cancer, small cell lung cancer, gastrointestinal cancer, colorectal cancer, bowel cancer, breast cancer, ovarian cancer, prostate cancer, testicular cancer, liver cancer, kidney cancer, bladder cancer, pancreatic cancer, brain cancer, sarcoma, osteosarcoma, Kaposi's sarcoma, and melanoma.

34. A method of treating a proliferative disease, which comprises administering the pyrrolobenzodiazepine dimer-linker-ligand conjugate according to any one of claims 19 to 25 or a pharmaceutically acceptable salt or solvate thereof to a subject.

35. The method of treating a proliferative disease according to claim 34, wherein the proliferative disease is selected from the group consisting of neoplasm, tumor, cancer, leukemia, psoriasis, bone disease, fibroproliferative disorder, and atherosclerosis.

36. The method of treating a proliferative disease according to claim 35, wherein the cancer is selected from the group consisting of lung cancer, small cell lung cancer, gastrointestinal cancer, colorectal cancer, bowel cancer, breast cancer, ovarian cancer, prostate cancer, testicular cancer, liver cancer, kidney cancer, bladder cancer, pancreatic cancer, brain cancer, sarcoma, osteosarcoma, Kaposi's sarcoma, and melanoma.
